# EUROPEAN PATENT APPLICATION

(11) **EP 4 678 663 A1**
(43) Date of publication of application: **14.01.2026**
(21) Application number: 24766419.6
(22) Date of filing: 04.03.2024
(51) Int. Cl.: C07K 19/00, C12N 9/48, C12N 15/62, A61K 38/48, A61K 47/68, A61P 29/00

(54) **NOVEL CORONAVIRUS PROTECTIVE NASAL SPRAY, AND PREPARATION AND USE THEREOF**

(30) Priority: 04.03.2023 CN 202310217327
(71) Applicant: SICHUAN CLOVER BIOPHARMACEUTICALS, INC., Chengdu, Sichuan 610041 (CN)
(72) Inventor: LIANG, Peng, Chengdu, Sichuan 610041 (CN)
(74) Representative: Kuttenkeuler, David
(86) International application number: PCT/CN2024/079998
(87) International publication number: WO 2024/183704

(57) **Abstract**

A fully human ACE2-Fc fusion protein, a pharmaceutical composition, preparation and kit containing same, and a nasal spray containing the fusion protein. Also provided is a use of the fusion protein for broad-spectrum prevention of coronavirus infections, such as infections with coronavirus SARS-CoV-2 and known and unknown variants thereof, including but not limited to original Hu-1, alpha, beta, gamma, delta, mu, omicron, JN.1, and/or other future strains. Also provided are a method for producing the fusion protein, and a method for using the fusion protein to prevent and/or treat infections with coronavirus SARS-CoV-2 and known and unknown variant strains thereof. Further provided is a use of the fusion protein and the pharmaceutical composition and preparation containing same for preventing the spread of coronavirus SARS-CoV-2 and known and unknown variant strains in infected subjects.

## Description

This application claims the priority of Chinese Patent Application No. 202310217327.3, filed on March 4, 2023, the entire contents of which are incorporated herein by reference.

### Technical Field

The present disclosure belongs to the field of biopharmaceuticals, and specifically relates to fully human ACE2-Fc fusion proteins for preventing and treating coronavirus infections as well as inhibiting coronavirus transmission, pharmaceutical compositions and formulations comprising the same, and kits, such as nasal sprays containing the fusion proteins. The disclosure further relates to methods for producing the fusion proteins, and the use thereof for preventing and/or treating infections caused by SARS-CoV-2 and its known variants or unknown variants of coronavirus SARS-CoV-2, as well as blocking transmission of SARS-CoV-2 and its known variants or unknown variants of coronavirus SARS-CoV-2 from infected subjects.

### Background Art

Coronavirus infects a wide range of birds and mammals, including humans. Coronaviruses may circulate annually within the human population, typically causing mild respiratory illnesses, though with higher severity in infants, the elderly, and immunocompromised individuals. However, certain coronaviruses-including Middle East respiratory syndrome coronavirus (MERS-CoV), severe acute respiratory syndrome coronavirus (SARS-CoV-1), and severe acute respiratory syndrome coronavirus 2 (SARS-CoV-2)-are highly pathogenic. Moreover, SARS-CoV-2 mutates rapidly. Starting from late 2020, a plurality of SARS-CoV- 2 variants of concern (VOCs) carrying mutations that may confer immune evasion have emerged. However, the development and deployment cycle for conventional vaccines is relatively long, far from sufficient to counter the threats posed by the novel coronavirus to work, life, health, and global epidemic prevention efforts. Therefore, broad-spectrum protective emergency technologies and equipment are crucial for reducing infection risks in the general population, alleviating the burden on epidemic prevention efforts, and revitalizing social and economic activities. The present disclosure provides methods, uses and articles of manufacture that satisfy the above and other needs.

### Summary of the Invention

In parallel with the surge in COVID-19 cases worldwide, a variety of new SARS-CoV-2 variants (VOCs) have emerged since the end of 2020. These VOCs appear to be associated with mutations in the spike (S) protein, which may increase viral transmissibility and/or confer immune escape from the immunity induced by first-wave COVID-19 vaccines based on the SARS-CoV-2 Hu-1 strain (also known as the SARS-CoV-2 prototype strain or SARS-CoV-2 wild-type strain). The spread of the B.1.1.7, B.1.351, and P.1 variants, identified in the UK, South Africa, and Brazil respectively, has led to their classification as VOCs. All these VOCs carry the N501Y mutation within the receptor-binding domain (RBD) of the S protein, which has been reported to increase transmission by 40% to 70%. Additionally, the B.1.351 and P.1 variants have two additional RBD mutations-E484K and K417-that may enable immune escape from antibodies elicited by both Hu-1-based vaccines and natural infection.

Randomized controlled clinical trials of the COVID-19 vaccines have shown that the vaccines are less effective against VOCs compared to the SARS-CoV-2 Hu-1 strain. The adjuvant-based COVID-19 vaccine NVX-CoV2373 demonstrated 89% efficacy in the UK (where B.1.1.7 was dominant), but only 49% efficacy in South Africa (where B.1.351 was dominant). The adenoviral vector COVID-19 vaccine ChAdOx1 showed only 10% efficacy against the B.1.351 variant. The Pfizer vaccine exhibited 75% efficacy against the B.1.351 variant, compared to 95% efficacy against Hu-1. Coronavac is an inactivated vaccine based on the Hu-1 strain and no neutralizing antibody titers against P.1 were detected in subjects who had been vaccinated.

Although there is some encouraging evidence that Hu-1-based COVID-19 vaccines may protect against severe disease and death caused by VOCs, lower vaccine efficacy against any COVID-19 illness with increased transmissibility may make achieving herd immunity particularly challenging. If not effectively controlled, the rapid global spread of SARS-CoV-2 VOCs could lead to the persistent emergence of new target variants or VOCs incorporating novel escape mutations. An example is the "India variant" (B.1.617), which emerged concurrently with a massive surge in COVID-19 cases in the spring of 2021 and has now been designated by WHO as a new VOC. The B.1.617.2 sublineage of this lineage has been named Delta. Additionally, the B.1.1.529 variant, first identified in South Africa, has been renamed Omicron.

In these cases, other epidemic prevention methods that can broadly respond to VOCs must be evaluated expeditiously.

In one aspect, the present disclosure provides a fusion protein containing a plurality of recombinant polypeptides, the fusion protein comprises human ACE2 protein or a fragment thereof and human IgG Fc protein or a functional variant thereof, and the fusion protein is constructed to specifically bind, via one or more binding sites, to wild-type, variant, or mutant forms of the human ACE2 receptor.

In some embodiments, the fusion protein is water-soluble.

Utilizing fully human antibody Fc fusion protein technology can significantly enhance the serum-free expression level of soluble proteins in vitro, facilitate affinity purification, and improve the in vitro/in vivo stability of the ACE2-Fc protein. As ACE2-Fc is a fully human fusion protein and the preferred administration route is nasal spray passive immunity, it is expected to have good safety.

In some embodiments, the human ACE2 protein or a fragment thereof comprises the human ACE2 ectodomain or a fragment thereof.

In some embodiments, the human ACE2 protein or a fragment thereof comprises an amino acid sequence selected from any one as set forth in SEQ ID NOs: 5, 7, 11, 12, 13, and 14; or an amino acid sequence having at least about 75%, 80%, 85%, 90%, 92%, 95%, 96%, 97%, 98%, or 99% sequence identity thereto, or a fragment of either thereof.

In some embodiments, the human ACE2 protein or a fragment thereof comprises an amino acid sequence as set forth in SEQ ID NO: 5, or an amino acid sequence having at least about 75%, 80%, 85%, 90%, 92%, 95%, 96%, 97%, 98%, or 99% sequence identity thereto, or a fragment of either thereof.

In some embodiments, the human IgG Fc protein or a functional variant thereof comprises an Fc region sequence selected from any one or more of human IgG1 Fc, IgG2 Fc, IgG3 Fc, and IgG4 Fc, or a functional variant or a fragment thereof.

In some embodiments, the human IgG Fc protein or a functional variant thereof comprises a human IgG1 Fc region sequence or a functional variant or a fragment thereof.

In some embodiments, the human IgG Fc protein or a functional variant thereof comprises an amino acid sequence as set forth in SEQ ID NO: 6 or SEQ ID NO: 8, or an amino acid sequence having at least about 75%, 80%, 85%, 90%, 92%, 95%, 96%, 97%, 98%, or 99% sequence identity thereto, or a fragment of either thereof.

In some embodiments, the human IgG Fc protein or a functional variant thereof comprises an amino acid sequence as set forth in SEQ ID NO: 6, or an amino acid sequence having at least about 75%, 80%, 85%, 90%, 92%, 95%, 96%, 97%, 98%, or 99% sequence identity thereto, or a fragment of either thereof.

In some embodiments, the fusion protein optionally comprises a signal peptide.

In some embodiments, the fusion protein optionally comprises a peptide linker, wherein the human ACE2 protein or a fragment thereof is directly linked to or via a peptide linker to the human IgG Fc protein or a functional variant thereof. For example, the peptide linker is selected from the group consisting of arginine-serine peptide linker (-RS-), valine-serine linker (-VS-), and glycine-serine linker (-GS-). The peptide linker can be a common peptide linker known in the art to link different functional polypeptide moieties in fusion proteins.

In some embodiments, the fusion protein optionally comprises a mutated sequence. The mutant sequence is used for C-terminal modification to facilitate tracking and detection.

In some embodiments, the fusion protein comprises an amino acid sequence selected from any one as set forth in SEQ ID NO: 1 through SEQ ID NO: 18, or an amino acid sequence having at least about 75%, 80%, 85%, 90%, 92%, 95%, 96%, 97%, 98%, or 99% sequence identity thereto, or a fragment thereof, or a combination thereof, e.g., an amino acid sequence selected from any one as set forth in SEQ ID NOs: 1-4 or 15-18, or an amino acid sequence having at least about 75%, 80%, 85%, 90%, 92%, 95%, 96%, 97%, 98%, or 99% sequence identity thereto, or a fragment thereof.

In some embodiments, the fusion protein also comprises a detectable label.

In one aspect, the present disclosure provides a pharmaceutical composition comprising the fusion protein of the present disclosure and optionally a pharmaceutically acceptable carrier.

In some embodiments, the pharmaceutical composition comprises from about 0.1 mg/ml to about 100 mg/ml of the fusion protein according to any one of claims 1 to 15, e.g., about 0.50 mg/ml to about 20.00 mg/ml, such as, about 1.25 mg/ml, about 2.50 mg/ml, or about 5.00 mg/ml.

In some embodiments, the pharmaceutical composition is formulated for administration via intramuscular, intradermal, subcutaneous, intravenous, intraarterial, intraarticular, intraperitoneal, intranasal, sublingual, tonsillar, oropharyngeal or other parenteral and mucosal routes, preferably formulated for intranasal administration, such as a nasal spray.

In one aspect, the present disclosure provides the fusion protein or pharmaceutical composition of the present disclosure for use in preventing or treating an infection of coronavirus SARS-CoV-2 and a variant thereof and/or preventing transmission of coronavirus SARS-CoV-2 and a variant thereof from an infected subject.

In one aspect, the present disclosure provides the use of the fusion protein or pharmaceutical composition of the present disclosure in the manufacture of a medicament for preventing or treating an infection of coronavirus SARS-CoV-2 and a variant thereof and/or for preventing transmission of coronavirus SARS-CoV-2 and a variant thereof from an infected subject.

In one aspect, the present disclosure provides a method for preventing or treating an infection of coronavirus SARS-CoV-2 and a variant thereof, and/or for preventing transmission of coronavirus SARS-CoV-2 and a variant thereof from an infected subject, wherein the method comprises administering to a subject a therapeutically effective amount of the fusion protein or pharmaceutical composition of the present disclosure.

In some embodiments, the administration is intranasal administration.

In one aspect, the present disclosure provides a kit for preventing or treating an infection of coronavirus SARS-CoV-2 and a variant thereof ; and/or for preventing transmission of coronavirus SARS-CoV-2 and a variant thereof from an infected subject, the kit comprising: the fusion protein or pharmaceutical composition of the present disclosure;
a container; and
an optional package insert or label indicating prophylaxis and/or therapy.

In the present disclosure, the coronavirus SARS-CoV-2 and a variant thereof include, but are not limited to, SARS-CoV-2 Hu-1, Alpha, Beta, Gamma, Delta, Mu, Omicron, JN.1; and unknown variants of coronavirus SARS-CoV-2.

In one aspect, the present disclosure provides a nucleic acid encoding the fusion protein or a fragment thereof of the present disclosure, a vector comprising the nucleic acid; and a host cell comprising the nucleic acid or the vector.

### Brief Description of the Drawings

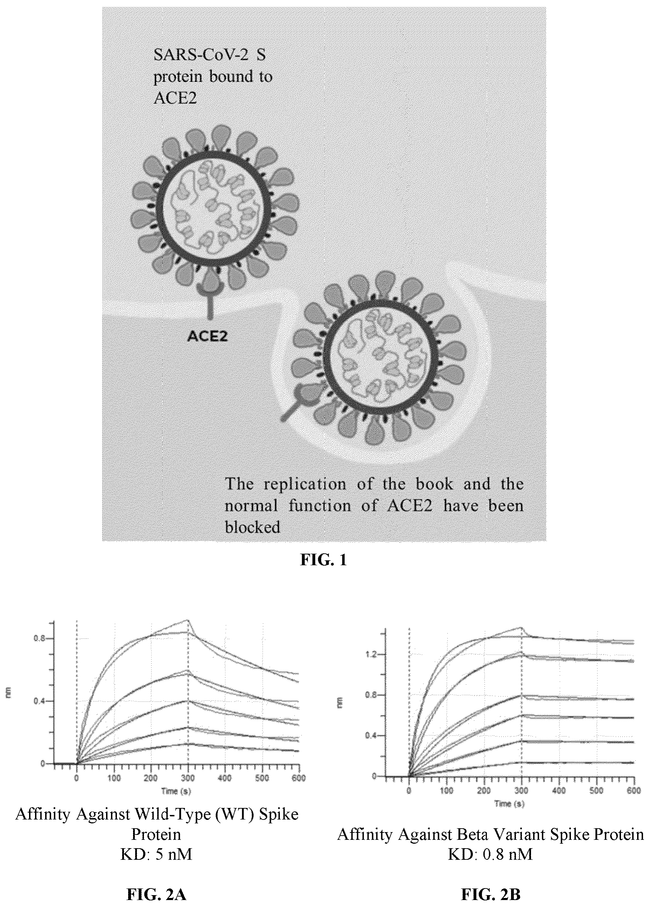

FIG. 1 shows the mechanism by which SARS-CoV-2 infects the host via the ACE2 receptor.

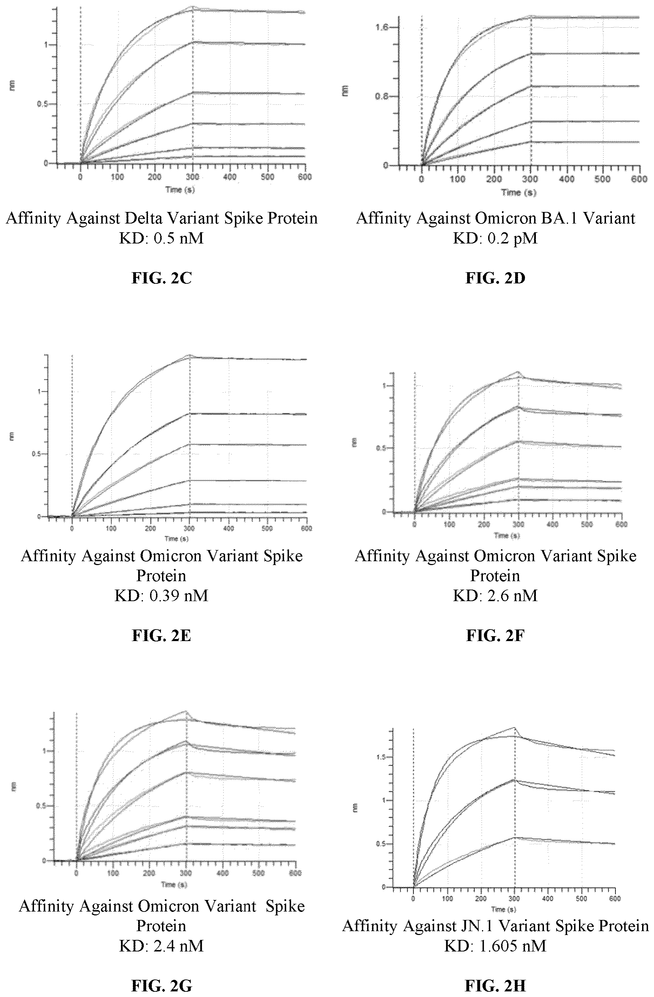

FIGs. 2A-2H show the affinity test results of ACE2-Fc fusion protein (SCB-719) with the S proteins of different SARS-CoV-2 variants. FIG. 2A shows the affinity test results with the S protein of Wild-type strain (Hu-1). FIG. 2B shows the affinity test results with the S protein of Beta variant. FIG. 2C shows the affinity test results with the S protein of Delta variant. FIG. 2D shows the affinity test results with the S protein of Omicron variant (BA.1). FIG. 2E shows the affinity test results with the S protein of Omicron variant (BA 4/5). FIG. 2F shows the affinity test results with the S protein of Omicron variant (XBB1.5). FIG. 2G shows the affinity test results with the S protein of Omicron variant (EG5.1). FIG. 2H shows the affinity test results with the S protein of JN.1 variant.

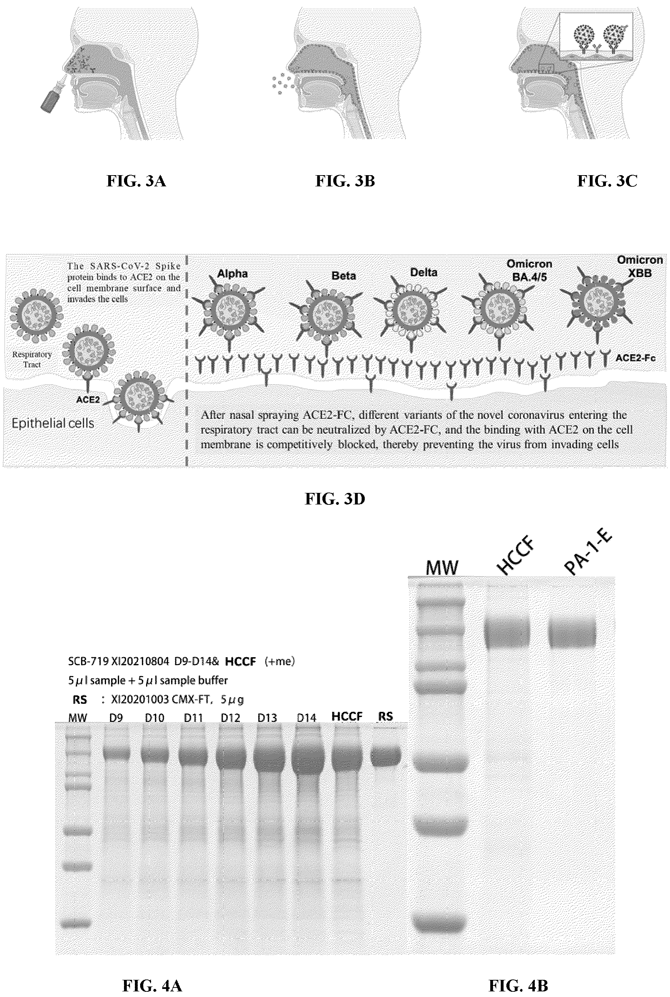

FIGs. 3A-3D schematically illustrate the mechanism by which the ACE2-Fc fusion protein nasal spray prevents SARS-CoV-2 infection. FIG. 3A shows the administration of ACE2-Fc fusion protein nasal spray into the nasal cavity. FIG. 3B shows airborne viruses or virus-laden droplets entering the nasal cavity. FIG. 3C shows the soluble ACE2-Fc fusion protein nasal spray, developed using Fc fusion technology, coats the upper respiratory tract (primarily the nasal cavity) following intranasal delivery. This forms the first line of defense against viral invasion, functioning as a biologically active "invisible mask." FIG. 3D shows the process of SARS-CoV-2 cell entry and its inhibition by the ACE2-Fc fusion protein. When transmissible viruses or virus-laden droplets are present in the air, the initial gateway to human infection occurs through upper respiratory tract cells. At this critical point, ACE2-Fc fusion proteins coating the nasal cavity and upper airways competitively bind to SARS-CoV-2 viruses of any variant. This interaction blocks the virus's spike (S) protein from accessing host cell binding sites, thereby preventing viral entry into host cells and subsequent human infection.

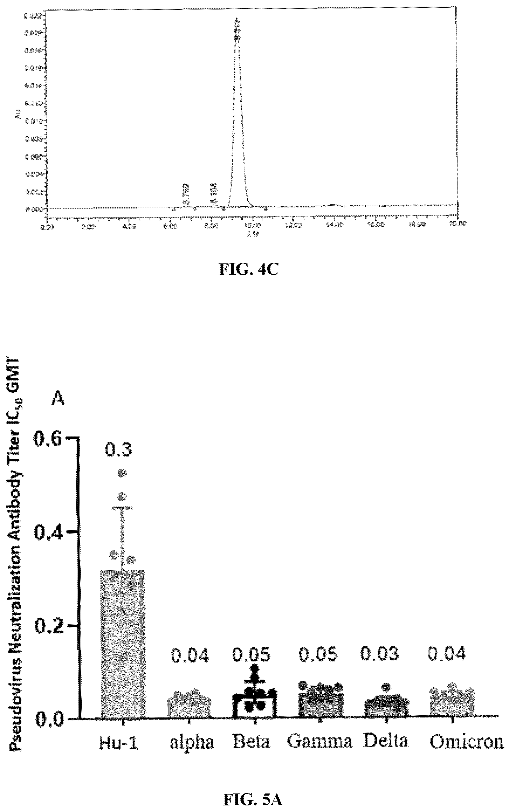

FIGs. 4A-4C schematically illustrate the purification and characterization of the ACE2-Fc fusion protein of an embodiment of the present disclosure. FIG. 4A shows the expression level of ACE2-Fc fusion protein in CHO. FIG. 4B shows the affinity purification profile of the ACE2-Fc fusion protein. FIG. 4C shows the purity profile of the ACE2-Fc fusion protein.

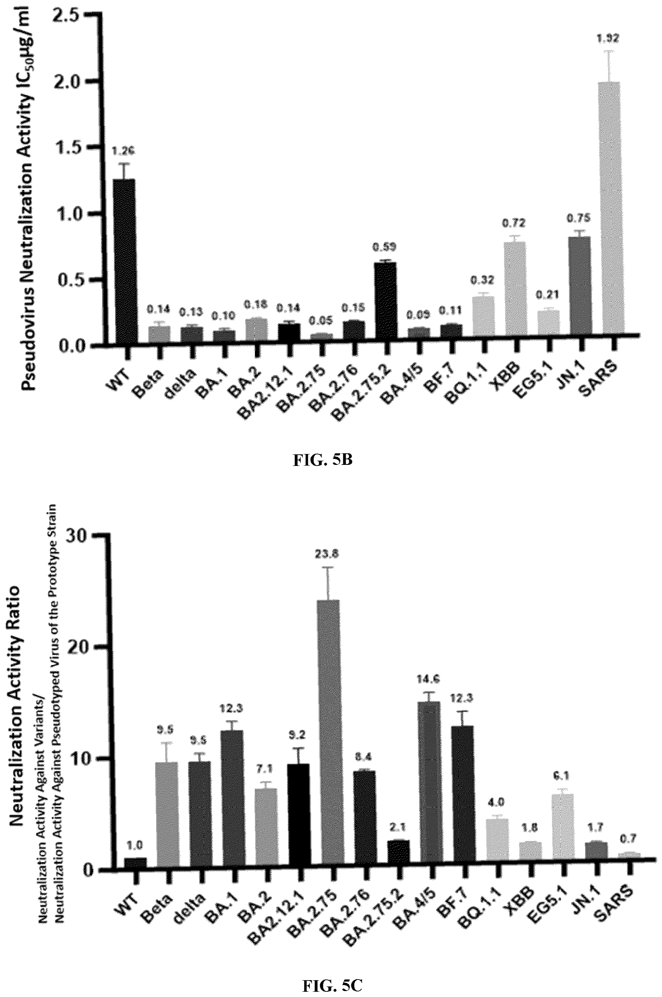

FIGs. 5A-5C schematically illustrate the results of pseudovirus neutralization assays for the ACE2-Fc fusion protein of an embodiment of the present disclosure against the prototype SARS-CoV-2 strain and a variant thereof. FIG. 5A: The test of mutants including Alpha, Beta, Gamma, Delta, and Omicron strains. FIG. 5B: The test of mutants including Beta, Delta, BA.1, BA.2, BA2.12.1, BA.2.75, BA.2.76, BA.2.75.2, BA.4/5, BF.7, BQ.1.1, XBB, EG.1, JN.1and SARS. FIG. 5C: The ratio of neutralizing activity (pseudovirus neutralizing activity against variants/neutralizing activity against original pseudovirus). FIGs. 5A-5C demonstrate that the ACE2-Fc fusion protein of the present disclosure exhibits neutralizing activity against pseudoviruses of all currently known variants, and the neutralizing activity against variants is higher than that against the prototype strain

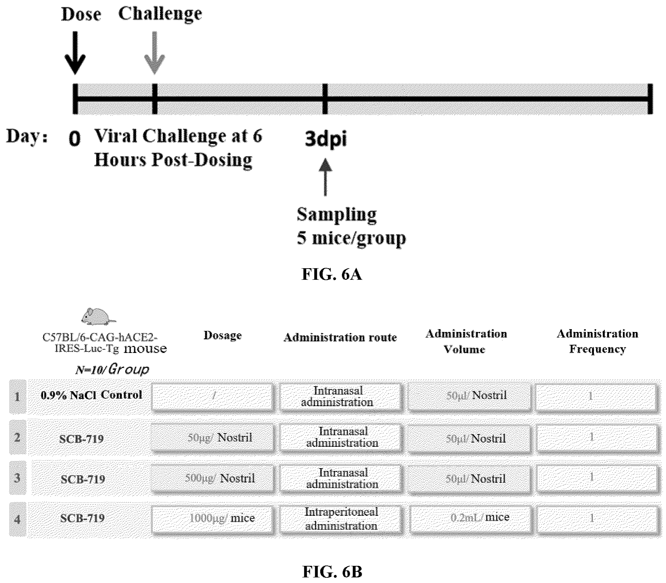

FIGs. 6A-6B show the design of the challenge experiment. FIG. 6A shows the Delta variant challenge study in transgenic mice expressing human ACE2. Forty mice were divided into 4 groups, 10 mice in each group. 5 mice from each group were sampled respectively for the measurement of body weight, lung viral load, and for lung pathological analysis and scoring. FIG. 6B: Group 1 (Control) mice were administered saline/vehicle intranasally. In Group 2, mice were administered 0.1 ml of ACE2-Fc fusion protein intranasally at a dose of 5 mg/kg. In Group 3, mice were administered 0.1 ml of ACE2-Fc fusion protein intranasally at a dose of 50 mg/kg. In Group 4, mice were administered 0.2 ml of ACE2-Fc fusion protein intraperitoneally at a dose of 50 mg/kg.

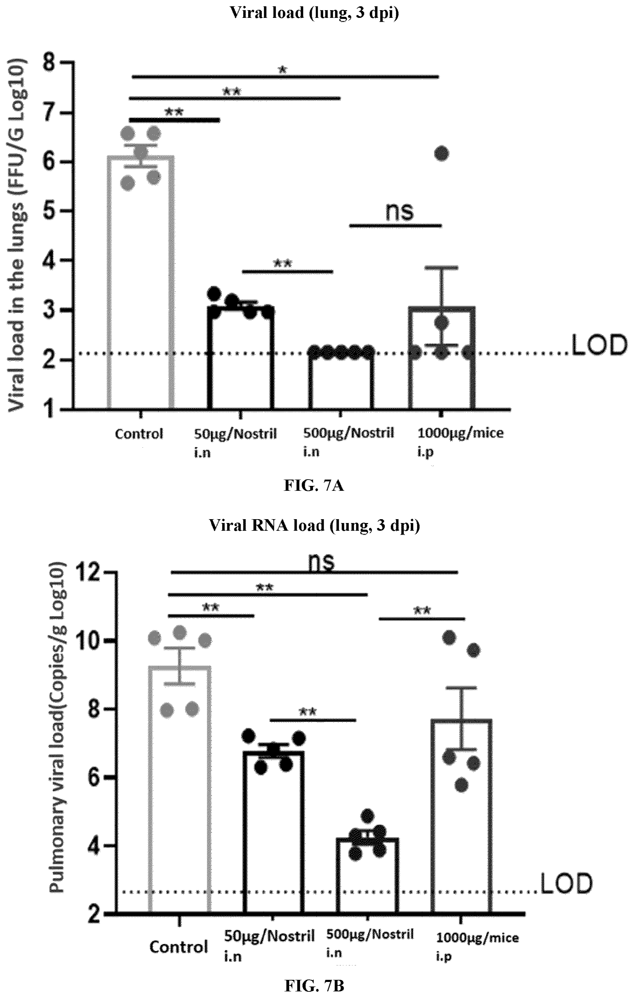

FIGs. 7A-7B exemplarily show the results of viral challenge experiments (pharmacodynamics experiments) conducted in transgenic mice using intranasal (i.n.) and intraperitoneal (i.p.) routes of administration. (Note: i.n. denotes intranasal administration; i.p. denotes intraperitoneal administration). FIG. 7A displays the results of the infectious viral load assay in the lungs. FIG. 7B displays the results of the viral RNA load assay in the lungs.

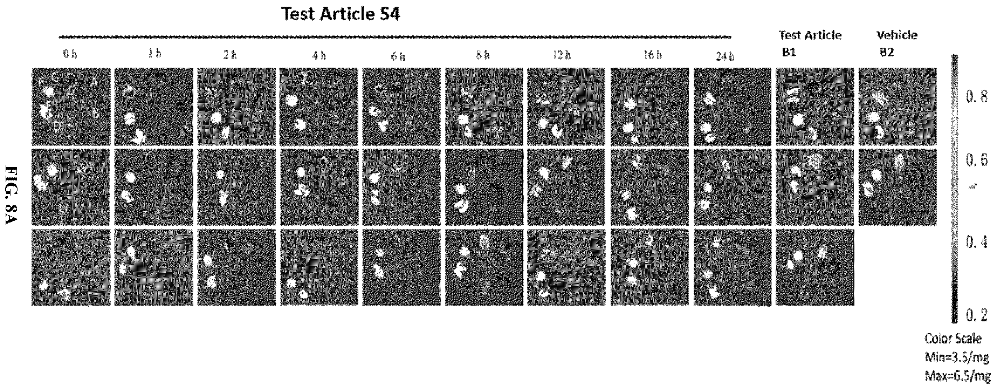

FIG. 8A exemplarily shows the organ distribution imaging results of Test Article B1, Vehicle B2, and Test Article S4 at various time points (with group sizes of n=3 or n=2). A: liver; B: spleen; C: kidney; D: heart; E: lung; F: brain; G: blood; H: nasal cavity.

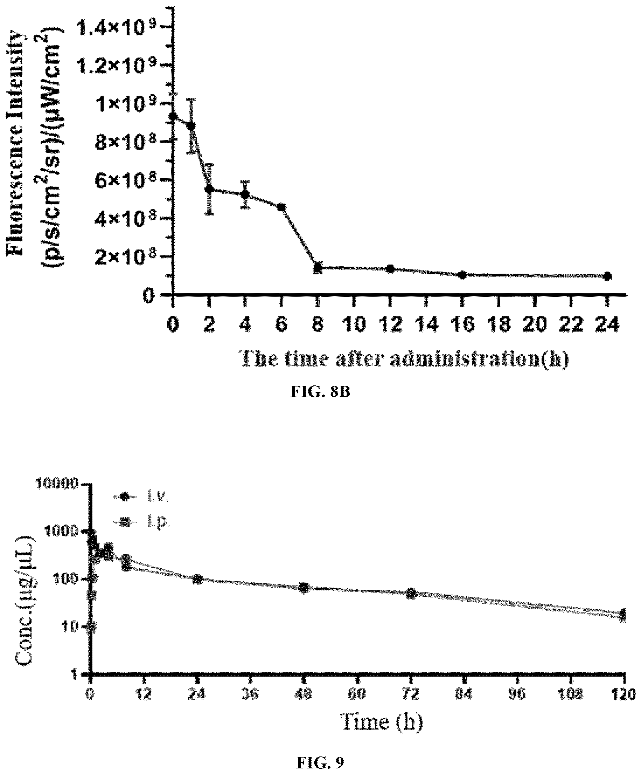

FIG. 8B exemplarily shows the fluorescence signal intensity in the nasal cavity at different time points for test article S4 ((p/s/cm²/sr)/(µW/cm²), Mean ± SEM., n = 3).
FIG. 9 exemplarily shows the pharmacokinetic (PK) and *in vitro* potency profiles of the ACE2-Fc fusion protein following IV/IP administration.

### Detailed Description of the Invention

The present disclosure provides a fully human ACE2-Fc fusion protein for the prevention, treatment of coronavirus infection and prevention of coronavirus transmission, pharmaceutical composition and preparations and kits containing it, as well as the manufacturing and usage methods. In some embodiments, a nasal spray formulation containing the ACE2-Fc fusion protein demonstrates a surprisingly broad preventive effect against coronavirus infection. Specifically, by coating the entire surface of the nasal cavity, the ACE2-Fc fusion protein receptor efficiently and competitively blocks the virus from binding to ACE2 on the surface of nasal mucosal epithelial cells, thereby effectively inhibiting viral invasion. This implies that, in the face of emerging SARS-CoV-2 variants where existing vaccines offer limited protection, intranasal delivery of the ACE2-Fc soluble receptor provides a flexible, rapid, and effective approach to achieve passive immunity against viral invasion. This strategy is particularly critical for high-risk populations (e.g., healthcare workers, border/customs personnel) and in confined, poorly ventilated settings with high crowd density (e.g., aircraft, high-speed trains, and other public transport). Furthermore, ACE2-Fc fusion protein binds to viruses within infected individuals, reducing viral shedding in expelled respiratory droplets (e.g., sneezes, saliva) and thereby blocking transmission, especially household transmission. Additionally, by binding to the virus within an infected individual, the ACE2-Fc fusion protein reduces the viral load in the body to achieve a therapeutic effect. This therapeutic effect can be realized through either intranasal or systemic administration.

Globally, SARS-CoV-2 infections have now surpassed 500 million cases, with cumulative deaths exceeding 6 million. More concerningly, following waves driven by variants such as Delta (India), Beta (South Africa), and Gamma (Brazil), the emergence of highly transmissible mutants-notably Omicron since late 2021-has persistently increased infection rates while exhibiting significant immune escape from licensed vaccines and neutralizing antibodies. This has compelled the implementation of diverse countermeasures worldwide. However, subsequent mutations beyond Omicron BA.1 have generated increasingly transmissible subvariants, including BA.2, BA.2.12.1, BA.4, BA.5, XBB, EG.5, and JN.1. Therefore, there is an urgent global need to rapidly develop highly effective and safe intervention strategies to address the challenges of SARS-CoV-2's rapid mutation rate and high immune evasion. Current vaccine development against emerging variants lags behind the pace of viral evolution. We must therefore develop alternative approaches beyond vaccines to block infection by any SARS-CoV-2 variant.

The ACE2-Fc fusion protein disclosed in this publication is intended to be a powerful supplement to address the current shortcomings in vaccine development and solve the problems of rapid mutation rate, high immune escape and strong infectious ability of new coronavirus, which is expected to effectively alleviate the current situation of new coronavirus rampant worldwide.

Key Technologies to be Addressed: First, regarding the application of the ACE2-Fc fusion protein for protection against SARS-CoV-2 variants. ACE2 is the key receptor for the virus to enter host cells. In the face of constant viral mutation and immune escape from vaccines and neutralizing antibodies, the protective efficacy of vaccines and therapeutic monoclonal antibodies is continuously diminishing. However, the receptor for viral entry into host cells remains unchanged. Furthermore, studies have shown that strains with stronger infectivity exhibit higher affinity for the ACE2 receptor. Therefore, using the ACE2-Fc fusion protein as a soluble receptor to block viral invasion can effectively address any existing and future SARS-CoV-2 variants, a strategy determined by the fundamental mechanism of the infection. In contrast, existing monoclonal antibodies could lose their protective efficacy against current and future variants at any time. Second, the feasibility of the ACE2-Fc fusion protein as a nasal spray formulation for routine use, specifically addressing its suitability in terms of storage and usage temperatures.

SARS-CoV-2 initiates infection by entering the human body via the nasal mucosa of the upper respiratory tract, establishing the nasal cavity as its primary entry portal. Intranasal delivery of the ACE2-Fc soluble receptor, which competitively blocks viral binding to endogenous ACE2 on nasal epithelial cells, enables coating of the entire nasal surface. This strategy effectively suppresses viral invasion, particularly against emerging variants where existing vaccines offer limited protection. Critically, passive immunization through intranasal ACE2-Fc administration provides flexible, rapid, and effective protection against viral entry. This approach is especially valuable for High-risk groups (e.g., healthcare workers, border control personnel), and in confined, poorly ventilated, congregate environments (e.g., aircraft, high-speed trains, and other public transport).

FIG. 1 exemplarily explains the principle that ACE2-Fc fusion protein solves the problem of rapid mutation rate of novel coronavirus strong transmission of mutant strains, and untimely update of vaccines against mutations. ACE2 has been established as the functional host receptor for severe acute respiratory syndrome coronavirus (SARS-CoV-2). A schematic diagram of the SARS-CoV-2 virus infecting host cells via the ACE2 receptor is shown in FIG. 1. Because while viral mutations drive immune evasion against vaccines and neutralizing antibodies, the receptor required for host cell entry remains invariant. Furthermore, studies demonstrate that strains with stronger infectivity exhibit higher binding affinity to ACE2. This lays the foundation for targeting the ACE2 receptor to block cellular infection by any SARS-CoV-2 variant.

FIGs. 3A-3D further exemplarily explain the principle of how ACE2-Fc blocks SARS-CoV-2 virus infection. The soluble human ACE2-Fc fusion protein was developed using Fc fusion protein technology and formulated as a nasal spray. Through intranasal administration, the ACE2-Fc fusion protein coats the upper respiratory tract, primarily the nasal cavity, which is the first line of defense against viral invasion, forming a biologically functional "invisible mask". When airborne viruses or virus-laden droplets are present, the first step for the virus to invade the human body is by infecting cells in the upper respiratory tract. At this time, the ACE2-Fc fusion protein covering the nasal cavity and upper respiratory tract can competitively bind with any mutant strain of SARS-CoV-2 virus, thereby blocking the site on the virus's S protein for binding with host cells, and blocking the virus from entering host cells via the S protein to infect the human body.

On one aspect, the present disclosure provides a fusion protein comprising a plurality of recombinant polypeptides, which includes a human ACE2 protein or a fragment thereof, and a human IgG Fc protein or a functional variant thereof. The fusion protein specifically binds to wild - type, variant or mutant human ACE2 receptors through one or more binding sites, and can be used for the treatment of coronavirus infections (e.g., prophylactic or therapeutic). The disclosure also provides methods of manufacture and use thereof. The nasal spray formulation containing the ACE2-Fc fusion protein has shown a surprisingly broad effect in preventing coronavirus infection. Specifically, by applying the soluble ACE2-Fc fusion protein receptor to the entire nasal surface, it can efficiently and competitively block the binding of the virus to ACE2 on the surface of nasal mucosal epithelial cells, thereby effectively inhibiting viral invasion. FIGs. 2A-2H show that the disclosed ACE2-Fc fusion protein has good affinity to the S protein of different strains.

In some embodiments, fusion protein described in the present disclosure is a soluble protein, wherein the fusion protein comprises human ACE2 protein or a fragment thereof and human IgG Fc protein or a functional variant thereof.

The fusion protein specifically binds to wild-type, variant or mutant human ACE2 receptors through one or more binding sites.

In some embodiments, the human ACE2 protein or a fragment thereof comprised in the fusion protein of the present disclosure comprises the human ACE2 ectodomain or a fragment thereof.

In some embodiments, the human ACE2 protein or a fragment thereof comprised in the fusion protein described in the present disclosure can be the wild-type human ACE2 extracellular domain or a fragment thereof, or a variant or mutant of the human ACE2 extracellular domain or a fragment thereof, provided that such variant or mutant of the human ACE2 extracellular domain or a fragment thereof retains the ability to bind to wild-type, variant, or mutant human ACE2 receptors.

In some embodiments, the human ACE2 protein or a fragment thereof comprised in the fusion protein described in the present disclosure comprises an amino acid sequence as set forth in any one of SEQ ID NO: 5, 7, 11, 12, 13 and 14 or an amino acid sequence having at least about 75%, 80%, 85%, 90%, 92%, 95%, 96%, 97%, 98%, or 99% sequence identity thereto, or a fragment of any of the aforementioned sequences.

In some embodiments, the human ACE2 protein or a fragment thereof comprised in the fusion protein described in the present disclosure comprises an amino acid sequence as set forth in SEQ ID NO: 5, or an amino acid sequence having at least about 75%, 80%, 85%, 90%, 92%, 95%, 96%, 97%, 98%, or 99% sequence identity thereto, or a fragment of either thereof.

In some embodiments, the human IgG Fc protein or a functional variant thereof included in the fusion protein described in the present disclosure comprises an Fc region sequence selected from any one or more of human IgG1 Fc, IgG2 Fc, IgG3 Fc, and IgG4 Fc, or a functional variant or a fragment thereof.

In some embodiments, the human IgG Fc protein or a functional variant thereof included in the fusion protein described in the present disclosure is selected from a human IgG1 Fc region sequence or a functional variant or a fragment thereof

In some embodiments, the human IgG Fc protein or a functional variant thereof included in the fusion protein described in the present disclosure comprises an amino acid sequence as set forth in SEQ ID NO: 6 or SEQ ID NO: 8, or an amino acid sequence having at least about 75%, 80%, 85%, 90%, 92%, 95%, 96%, 97%, 98%, or 99% sequence identity thereto, or a fragment of either thereof.

In some embodiments, the human IgG Fc protein or a functional variant thereof included in the fusion protein described in the present disclosure comprises an amino acid sequence as set forth in SEQ ID NO: 6, or an amino acid sequence having at least about 75%, 80%, 85%, 90%, 92%, 95%, 96%, 97%, 98%, or 99% sequence identity thereto, or a fragment of either thereof.

In some embodiments, the fusion protein optionally comprises a signal peptide, e.g. the signal peptide is as shown in SEQ ID NO. 9.

In some embodiments, the fusion protein optionally comprises a peptide linker, wherein the human ACE2 protein or a fragment thereof and the human IgG Fc protein or a functional variant thereof are directly linked or linked via the peptide linker.

In some embodiments, the fusion protein optionally comprises a mutated sequence. e.g. the mutated sequence is as shown in SEQ ID NO. 10.

In some embodiments, the fusion protein described in the present disclosure comprises an amino acid sequence as set forth in any one of SEQ ID NO: 1-18, or an amino acid sequence having at least about 75%, 80%, 85%, 90%, 92%, 95%, 96%, 97%, 98% or 99% sequence identity thereto, or a fragment thereof, or a combination thereof; for example, comprising an amino acid sequence as set forth in any one of SEQ ID NOs: 1-4 or 15-18, or an amino acid sequence having at least about 75%, 80%, 85%, 90%, 92%, 95%, 96%, 97%, 98%, or 99% sequence identity thereto, or a fragment thereof.

In some embodiments, the fusion protein described in the present disclosure further comprises a detectable label.

In one aspect, the present disclosure provides a pharmaceutical composition comprising the fusion protein described herein and optionally a pharmaceutically acceptable carrier.

In some embodiments, the pharmaceutical composition of the present disclosure comprises the fusion protein according to any one of claims 1 to 15 at a concentration of about 0.1 mg/ml to about 100 mg/ml, for example about 0.50 mg/ml to about 20.00 mg/ml, such as about 1.25 mg/ml, about 2.50 mg/ml, or about 5.00 mg/ml.

In some embodiments, the pharmaceutically acceptable carrier included in the pharmaceutical composition of the present disclosure is selected from one or more of the following: buffers, tonicity agents, or a combination thereof.

In some embodiments, the pharmaceutical composition of the present disclosure, wherein the buffer in the pharmaceutical composition of the present disclosure comprises one or more selected from the group consisting of sodium dihydrogen phosphate monohydrate, disodium hydrogen phosphate dihydrate, or a combination thereof.

In some embodiments, the tonicity agent in the pharmaceutical composition of the present disclosure comprises one or more selected from the group consisting of sodium chloride, potassium chloride, glycerol, glucose, sorbitol, sucrose, xylitol, and mannitol.

In some embodiments, the tonicity agent in the pharmaceutical composition of the present disclosure comprises sodium chloride and/or sucrose.

In some embodiments, the pharmaceutically acceptable carriers as described in the present disclosure optionally comprises a stabilizer, wherein the stabilizer is selected from the group consisting of: proteins, peptides, or hydrolysates, e.g., albumin, gelatin; sugars, e.g., sucrose, lactose, sorbitol; amino acids, e.g., sodium glutamate; or a combination thereof, for example, wherein the stabilizer comprises sucrose.

In some embodiments, the pharmaceutically acceptable carrier as described in the present disclosure optionally comprises a bacteriostatic agent, wherein the bacteriostatic agent is selected from the group consisting of benzoic acid, sorbic acid, thimerosal, and phenylethyl alcohol, or a combination thereof, for example, wherein the bacteriostatic agent comprises phenylethyl alcohol and/or thimerosal.

In some embodiments, the pharmaceutical composition described in the present disclosure is a dosage form suitable for administration via a route selected from intramuscular, intradermal, subcutaneous, intravenous, intraarterial, intraarticular, intraperitoneal, intranasal, sublingual, tonsillar, oropharyngeal, or other parenteral and mucosal routes, preferably being a dosage form for intranasal administration, for example as a nasal spray.

In one aspect, the present disclosure provides the use of the fusion protein or pharmaceutical composition of the present disclosure for the prevention and treatment of infection by coronavirus SARS-CoV-2 and a variant thereof and/or for the prevention of transmission of coronavirus SARS-CoV-2 and a variant thereof from an infected subject.

In some embodiments, the fusion protein or pharmaceutical composition of the present disclosure is administered intranasally for preventing and treating infection by coronavirus SARS-CoV-2 and a variant thereof, and/or for preventing transmission of coronavirus SARS-CoV-2 and a variant thereof from an infected subject.

In one aspect, the present disclosure provides the use of the fusion protein or pharmaceutical composition of the present disclosure in the preparation of a medicament for the prevention and treatment of infection by coronavirus SARS-CoV-2 and a variant thereof and/or for the prevention of transmission of coronavirus SARS-CoV-2 and a variant thereof from an infected subject.

In one aspect, the present disclosure provides a method for preventing or treating infection by coronavirus SARS-CoV-2 and a variant thereof, and/or preventing transmission of coronavirus SARS-CoV-2 and a variant thereof from an infected subject, the method comprising administering to a subject a therapeutically effective amount of the fusion protein or pharmaceutical composition of the present disclosure.

In some embodiments, the administration is intranasal.

In one aspect, the present disclosure provides a kit for preventing or treating infection by coronavirus SARS-CoV-2 and a variant thereof, and/or for preventing transmission of coronavirus SARS-CoV-2 and a variant thereof from an infected subject, the kit comprising:
the fusion protein or pharmaceutical composition of the present disclosure;
a container; and
an optional package insert or label with instructions for prevention and/or treatment.

In the present disclosure, the coronavirus SARS-CoV-2 and a variant thereof described include, but are not limited to, SARS-CoV-2 Hu-1, Alpha, Beta, Gamma, Delta, Mu, Omicron, JN.1; and unknown variants of coronavirus SARS-CoV-2.

In one aspect, the present disclosure provides a nucleic acid encoding the fusion protein or a fragment thereof of the present disclosure, a vector comprising the nucleic acid; and a host cell comprising the nucleic acid or the vector.

In some embodiments, the vector is phFC(IM).

In some embodiments, the host cell is a CHO cell, e.g., GH-CHO.

In one aspect, the present disclosure provides a method for producing the fusion protein described herein, the method comprising:
(a) culturing recombinant cells in suspension culture, wherein the recombinant cells contain a vector comprising a nucleic acid molecule encoding the fusion protein of the present disclosure; and
(b) isolating the fusion protein from the suspension culture.

In some embodiments, the present disclosure provides a method for producing the fusion protein described herein, the method comprising:
Conducting one or more stages of cell expansion, followed by:
(a) culturing recombinant cells in suspension culture, wherein the recombinant cells contain an expression vector comprising a nucleic acid molecule encoding the fusion protein of the present disclosure; and
(b) isolating the fusion protein from the suspension culture.

In some embodiments, the present disclosure provides a method for producing the fusion protein described herein, the method further comprising:
(1) Rapid capture of samples or enrichment of the fusion protein,
(2) One or more stages of purification of the fusion protein, and
(3) Concentration and buffer exchange to obtain the stock solution of the fusion protein.

Similar to other enveloped RNA viruses (e.g., HIV, RSV, and influenza), coronaviruses including SARS-CoV-2 possess trimeric surface antigens on their viral envelopes, which enable entry into diverse host cells via specific cell surface receptors during infection. Like SARS-CoV-1, SARS-CoV-2 utilizes its trimeric viral surface antigen (S protein) to bind to its specific cellular receptor ACE2, thereby gaining entry into host cells of the mammalian respiratory system. The fusion protein provided in the present disclosure can bind to the human ACE2 receptor, thereby blocking viral attachment to ACE2 and consequently preventing and/or eliminating viral infection. The ACE2-Fc fusion protein-containing compositions (e.g., nasal sprays) provided herein can broadly and effectively inhibit infection by viruses that enter human cells via the ACE2 receptor. In some aspects, such ACE2-Fc fusion protein-containing compositions can broadly and effectively suppress the invasion of SARS-CoV- 2 variants and mutants.

In some embodiments, the present disclosure provides a method for treating a subject in need thereof, comprising administering to the subject a therapeutically effective amount of a fusion protein, wherein the fusion protein comprises a sequence having at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to SEQ ID NO: 1 or SEQ ID NO: 2.

The present disclosure further provides a pharmaceutical composition comprising the fusion protein disclosed herein, a method for producing the fusion protein disclosed herein, a method for treating a subject using the fusion protein and/or pharmaceutical composition disclosed herein, and related kits.

All publications mentioned in this application, including patent documents, scientific articles, and databases, are incorporated by reference in their entirety for all purposes, to the same extent as if each individual publication were individually incorporated by reference. In the event of any inconsistency or conflict between the definitions set forth in the present disclosure and those in any patents, applications, published applications, or other publications incorporated by reference herein, the definitions provided in the present disclosure shall prevail. The section headings used herein are for organizational purposes only and shall not be construed as limiting the subject matter.

### I. ACE2-Fc fusion protein for anti-coronavirus infection

The present disclosure discloses an ACE2-Fc fusion protein and compositions comprising the same for preventing coronavirus infection. In some embodiments, the composition containing the ACE2-Fc fusion protein may be a nasal spray, which can coat the nasal cavity and upper respiratory tract, wherein the ACE2-Fc fusion protein can competitively bind to SARS-CoV-2 and any mutant strains thereof, thereby blocking the binding site of the viral S protein with host cells, and blocking the virus from infecting the human body by entering host cells via the S protein. Based on the mechanism by which SARS-CoV-2 enters host cells to cause infection, a person skilled in the art will fully understand that any mutant strains mentioned in the context of the ACE2-Fc fusion protein competitively binding to SARS-CoV-2 and any mutant strains thereof includes both known SARS-CoV-2 mutant strains and unknown SARS-CoV-2 mutant strains, wherein the said unknown SARS-CoV-2 mutant strains can be SARS-CoV-2 mutant strains that already exist but have not yet been discovered and identified by humans, or can be new SARS-CoV-2 mutant strains that do not currently exist but will emerge in the future due to viral mutation.

Coronaviruses are a family of positive-sense single-stranded RNA viruses known to cause severe respiratory diseases. They possess the largest genomes (26-32 kb) among known RNA viruses and are phylogenetically classified into four genera (α, β, γ, δ), with β-coronaviruses further subdivided into four lineages (A, B, C, D). Currently, viruses of the coronavirus family known to infect humans are from the α-coronavirus and β-coronavirus genera. In addition, it is believed that γ-coronaviruses and δ-coronaviruses may infect humans in the future. Non-limiting examples of β-coronaviruses include Middle East respiratory syndrome coronavirus (MERS-CoV), severe acute respiratory syndrome coronavirus (SARS-CoV), human coronavirus HKU1 (HKU1-CoV), human coronavirus OC43 (OC43-CoV), mouse hepatitis virus (MHV-CoV), bat SARS-like coronavirus WIV1 (WIV1-CoV), and human coronavirus HKU9 (HKU9-CoV). Non-limiting examples of α-coronaviruses include Human coronavirus 229E (229E-CoV), human coronavirus NL63 (NL63-CoV), porcine epidemic diarrhea virus (PEDV), and transmissible gastroenteritis coronavirus (TGEV). A non-limiting example of δ-coronaviruses is Porcine deltacoronavirus (SDCV).

The present disclosure provides a list of severe acute respiratory syndrome-associated coronaviruses
▪ *Bat coronavirus* Cp/Yunnan2011
▪ *Bat coronavirus* RaTG13
▪ *Bat coronavirus* Rp/Shaanxi2011
   ∘ Bat SARS-related coronavirus HKU3
      ▪ *Bat SARS-related coronavirus* HKU3-1
      ▪ *Bat SARS-related coronavirus* HKU3-10
      ▪ *Bat SARS-related coronavirus* HKU3-11
      ▪ *Bat SARS-related coronavirus* HKU3-12
      ▪ *Bat SARS-related coronavirus* HKU3-13
      ▪ *Bat SARS-related coronavirus* HKU3-2
      ▪ *Bat SARS-related coronavirus* HKU3-3
      ▪ *Bat SARS-related coronavirus* HKU3-4
      ▪ *Bat SARS-related coronavirus* HKU3-5
      ▪ *Bat SARS-related coronavirus* HKU3-6
      ▪ *Bat SARS-related coronavirus* HKU3-7
      ▪ *Bat SARS-related coronavirus* HKU3-8
      ▪ *Bat SARS-related coronavirus* HKU3-9
▪ *Bat* *SARS-related coronavirus* Rp1
▪ *Bat* *SARS-related coronavirus* Rp2
   ∘ *Bat SARS CoV* Rfl/2004
      ▪ *Bat Cov 273*/*2005*
   ∘ *Bat SARS CoV* Rm1/2004
      ▪ *Bat Cov 279*/*2005*
▪ *Bat* *SARS CoV* Rp3/2004
▪ *Bat SARS-like coronavirus*
▪ *Bat SARS-like coronavirus Rs3367*
▪ *Bat SARS-like coronavirus RsSHC014*
▪ *Bat SARS-like coronavirus WIV1*
▪ *Bat SARS-like coronavirus YNLF_31C*
▪ *Bat SARS-like coronavirus YNLF 34C*
▪ BtRf-BetaCoV/HeB2013
▪ BtRf-BetaCoV/JL2012
▪ BtRf-BetaCoV/SX2013
▪ BtRs-BetaCoV/GX2013
▪ BtRs-BetaCoV/HuB2013
▪ BtRs-BetaCoV/YN2013
▪ Civet SARS CoV 007/2004
▪ Civet SARS CoV SZ16/2003
▪ Civet SARS CoV SZ3/2003
   ∘ Recombinant SARSr-CoV
      ▪ SARS coronavirus ExoN1
      ▪ SARS coronavirus MA15
      ▪ SARS coronavirus MA15 ExoN1
      ▪ SARS coronavirus wtic-MB
▪ Rhinolophus sinicus coronavirus
▪ Bat SARS coronavirus
▪ SARS-CoV A001
▪ SARS-CoV A013
▪ SARS-CoV A021
▪ SARS-CoV A022
▪ SARS-CoV A030
▪ SARS-CoV A031
▪ SARS-CoV AS
▪ SARS-CoV B012
▪ SARS-CoV B024
▪ SARS-CoV B029
▪ SARS-CoV B033
▪ SARS-CoV B039
▪ SARS-CoV B040
▪ SARS-CoV BJ01
▪ SARS-CoV BJ02
▪ SARS-CoV BJ03
▪ SARS-CoV BJ04
▪ SARS-CoV BJ162
▪ SARS-CoV BJ182-12
▪ SARS-CoV BJ182-4
▪ SARS-CoV BJ182-8
▪ SARS-CoV BJ182a
▪ SARS-CoV BJ182b
▪ SARS-CoV BJ202
▪ SARS-CoV BJ2232
▪ SARS-CoV BJ302
▪ SARS-CoV C013
▪ SARS-CoV C014
▪ SARS-CoV C017
▪ SARS-CoV C018
▪ SARS-CoV C019
▪ SARS-CoV C025
▪ SARS-CoV C028
▪ SARS-CoV C029
▪ SARS-CoV CDC#200301157
▪ SARS-CoV civet010
▪ SARS-CoV civet014
▪ SARS-CoV civet019
▪ SARS-CoV civet020
▪ SARS-CoV CS21
▪ SARS-CoV CS24
▪ SARS-CoV CUHK-AG01
▪ SARS-CoV CUHK-AG02
▪ SARS-CoV CUHK-AG03
▪ SARS-CoV CUHK-L2
▪ SARS-CoV CUHK-Sul0
▪ SARS-CoV CUHK-W1
▪ SARS-CoV cw037
▪ SARS-CoV cw049
▪ SARS-CoV ES191
▪ SARS-CoV ES260
▪ SARS-CoV FRA
   ∘ SARS-CoV Frankfurt 1
      ▪ SARS-CoV Frankfurtl-v01
▪ SARS-CoV GD01
▪ SARS-CoV GD03T0013
▪ SARS-CoV GD322
▪ SARS-CoV GD69
▪ SARS-CoV GDH-BJH01
▪ SARS-CoV GZ-A
▪ SARS-CoV GZ-B
▪ SARS-CoV GZ-C
▪ SARS-CoV GZ-D
▪ SARS-CoV GZ02
▪ SARS-CoV GZ0401
▪ SARS-CoV GZ0402
▪ SARS-CoV GZ0403
▪ SARS-CoV GZ43
▪ SARS-CoV GZ50
▪ SARS-CoV GZ60
▪ SARS-CoV HB
▪ SARS-CoV HC/SZ/61103
▪ SARS-CoV HGZ8L1-A
▪ SARS-CoV HGZ8L1-B
▪ SARS-CoV HGZ8L2
▪ SARS-CoV HHS-2004
▪ SARS-CoV HKU-36871
▪ SARS-CoV HKU-39849
▪ SARS-CoV HKU-65806
▪ SARS-CoV HKU-66078
▪ SARS-CoV Hong Kong/03/2003
▪ SARS-CoV HPZ-2003
▪ SARS-CoV HSR 1
▪ SARS-CoV HSZ-A
▪ SARS-CoV HSZ-Bb
▪ SARS-CoV HSZ-Bc
▪ SARS-CoV HSZ-Cb
▪ SARS-CoV HSZ-Cc
▪ SARS-CoV HSZ2-A
▪ SARS-CoV HZS2-Bb
▪ SARS-CoV HZS2-C
▪ SARS-CoV HZS2-D
▪ SARS-CoV HZS2-E
▪ SARS-CoV HZS2-Fb
▪ SARS-CoV HZS2-Fc
▪ SARS-CoV JMD
▪ SARS-CoV LC1
▪ SARS-CoV LC2
▪ SARS-CoV LC3
▪ SARS-CoV LC4
▪ SARS-CoV LC5
▪ SARS-CoV LLJ-2004
▪ SARS-CoV NS-1
▪ SARS-CoV P2
▪ SARS-CoV PC4-115
▪ SARS-CoV PC4-127
▪ SARS-CoV PC4-13
▪ SARS-CoV PC4-136
▪ SARS-CoV PC4-137
▪ SARS-CoV PC4-145
▪ SARS-CoV PC4-199
▪ SARS-CoV PC4-205
▪ SARS-CoV PC4-227
▪ SARS-CoV PC4-241
▪ SARS-CoV PUMC01
▪ SARS-CoV PUMC02
▪ SARS-CoV PUMC03
▪ SARS-CoV Rs 672/2006
▪ SARS-CoV sf098
▪ SARS-CoV sf099
▪ SARS-CoV ShanghaiQXC1
▪ SARS-CoV ShanghaiQXC2
▪ SARS-CoV Shanhgai LY
▪ SARS-CoV Sin0409
▪ SARS-CoV Sin2500
▪ SARS-CoV Sin2677
▪ SARS-CoV Sin2679
▪ SARS-CoV Sin2748
▪ SARS-CoV Sin2774
▪ SARS-CoV Sin3408
▪ SARS-CoV Sin3408L
▪ SARS-CoV Sin3725V
▪ SARS-CoV Sin3765V
▪ SARS-CoV Sin842
▪ SARS-CoV Sin845
▪ SARS-CoV Sin846
▪ SARS-CoV Sin847
▪ SARS-CoV Sin848
▪ SARS-CoV Sin849
▪ SARS-CoV Sin850
▪ SARS-CoV Sin852
▪ SARS-CoV Sin WNV
▪ SARS-CoV Sino1-11
▪ SARS-CoV Sino3-11
▪ SARS-CoV SinP1
▪ SARS-CoV SinP2
▪ SARS-CoV SinP3
▪ SARS-CoV SinP4
▪ SARS-CoV SinP5
▪ SARS-CoV SoD
▪ SARS-CoV SZ1
▪ SARS-CoV SZ13
▪ SARS-CoV Taiwan
▪ SARS-CoV Taiwan JC-2003
▪ SARS-CoV Taiwan TC1
▪ SARS-CoV Taiwan TC2
▪ SARS-CoV Taiwan TC3
▪ SARS-CoV TJ01
▪ SARS-CoV TJF
▪ SARS-CoV Tor2
   ∘ SARS-CoV TW
      ▪ SARS-CoV TW-GD1
      ▪ SARS-CoV TW-GD2
      ▪ SARS-CoV TW-GD3
      ▪ SARS-CoV TW-GD4
      ▪ SARS-CoV TW-GD5
      ▪ SARS-CoV TW-HP1
      ▪ SARS-CoV TW-HP2
      ▪ SARS-CoV TW-HP3
      ▪ SARS-CoV TW-HP4
      ▪ SARS-CoV TW-JC2
      ▪ SARS-CoV TW-KC1
      ▪ SARS-CoV TW-KC3
      ▪ SARS-CoV TW-PH1
      ▪ SARS-CoV TW-PH2
      ▪ SARS-CoV TW-YM1
      ▪ SARS-CoV TW-YM2
      ▪ SARS-CoV TW-YM3
      ▪ SARS-CoV TW-YM4
▪ SARS-CoV TW1
▪ SARS-CoV TW10
▪ SARS-CoV TW11
▪ SARS-CoV TW2
▪ SARS-CoV TW3
▪ SARS-CoV TW4
▪ SARS-CoV TW5
▪ SARS-CoV TW6
▪ SARS-CoV TW7
▪ SARS-CoV TW8
▪ SARS-CoV TW9
▪ SARS-CoV TWC
▪ SARS-CoV TWC2
▪ SARS-CoV TWC3
▪ SARS-CoV TWH
▪ SARS-CoV TWJ
▪ SARS-CoV TWK
▪ SARS-CoV TWS
▪ SARS-CoV TWY
▪ SARS-CoV Urbani
▪ SARS-CoV Vietnam
▪ SARS-CoV WF188
▪ SARS-CoV WH20
▪ SARS-CoV WHU
▪ SARS-CoV xw002
▪ SARS-CoV ZJ01
▪ SARS-CoV ZJ02
▪ SARS-CoV ZJ0301
▪ SARS-CoV ZMY 1
▪ SARS-CoV ZS-A
▪ SARS-CoV ZS-B
▪ SARS-CoV ZS-C
▪ Bat-SARSr-CoV RsSHC014
▪ SARS-related β-coronavirus Rp3/2004
▪ Severe Acute Respiratory Syndrome Coronavirus 2

The following table shows exemplary SARS-CoV-2 strains

| **Name** | | **Geographic distribution** | **Significant Mutations** | **Influence** | **Sequence** |
|---|---|---|---|---|---|
| D614G | | Global spread | D614G | Increased Transmissibility, Dominant Transmission Since June 2020 | P0DTC2 |
| B.1.1.7 | 501Y.V1 | UK / Worldwide (but predominantly in the US) | D614G, N501Y, P681H | Increased Transmissibility | B.1.1.7 Lineage |
| B.1.351 | 501.V2 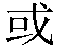 N501Y.V2 | South Africa | N501Y, **E484K***, K417N | Increased Transmissibility, *** Immune Escape Mutations *** | B.1.351 Lineage |
| B.1.1.248 | P1 | Brazil | N501Y, **E484K***, K417T | Increased Transmissibility, *** Immune Escape Mutations** * | P1 Lineage |

The coronavirus genome is capped, polyadenylated, and coated with nucleocapsid protein. Coronavirus particles comprise a viral envelope containing a type I fusion glycoprotein known as the spike (S) protein. Most coronaviruses share a common genomic structure, wherein the replicase gene is located in the 5' region of the genome, while the structural genes are situated in the 3' region.

The coronavirus spike (S) protein is a class I fusion glycoprotein initially synthesized as a precursor protein. A single precursor S polypeptide forms homotrimers, undergoes glycosylation and signal peptide removal in the Golgi apparatus, and is cleaved by cellular proteases to generate separate S1 and S2 polypeptide chains. These chains remain associated as S1/S2 protomers within the homotrimeric complex, and are therefore a trimer of heterodimers. The S1 subunit, positioned distally to the viral membrane, contains the receptor-binding domain (RBD) that mediates viral attachment to host receptors. The S2 subunit contains the fusion machinery, such as the fusion peptide, two characteristic heptad repeat sequences (HR1 and HR2) of fusion glycoproteins, a central helix, a transmembrane domain, and a cytosolic tail domain.

ACE2, as the receptor for SARS-CoV-2, connects with the spike protein on the SARS-CoV-2 envelope via its extracellular domain. At this time, the transmembrane serine protease (TMPRSS2) on the cell membrane surface cleaves the S protein into two subunits, S1 and S2, and exposes the fusion peptide, to achieve the purpose of fusion with the cell membrane and release of genetic material for replication.

The viral antigen or immunogen comprises a protease cleavage site, wherein the protease is optionally selected from furin, trypsin, factor Xa, or cathepsin L. During viral assembly, Furin protease can cleave its S protein into different subunits (S1 and S2), and is then released to infect other cells. SARS-CoV lacks a Furin recognition site, so it is possible that due to the difference in S protein cleavage and assembly mechanisms, SARS-CoV-2 has a stronger membrane fusion capability and higher cell entry efficiency. Studies have found that the binding ability of SARS-CoV-2 to ACE2 is 10-20 times that of SARS, resulting in the high transmissibility of SARS-CoV-2.

In some embodiments, the ACE2-Fc fusion protein comprises an ACE2 protein or polypeptide. In some embodiments, the ACE2 protein is a primate ACE2 protein, such as a human ACE2 protein or a fragment thereof. In some embodiments, the human ACE2 protein or a fragment thereof is a full-length protein of the extracellular domain of human ACE2 or a fragment protein thereof. In some embodiments, the non-dimerizing Fc domain is an IgG, IgM, IgD, IgA, or IgE Fc region, or a variant, mutant, or a fragment thereof. In some embodiments, the non-dimerizing Fc domain is an IgG1, IgG2, IgG3, or IgG4 Fc region, or a variant, mutant, or a fragment thereof. In some embodiments, the Fc protein is a primate Fc protein, such as a human IgG Fc protein or a functional variant thereof. In some embodiments, the human IgG Fc or a fragment thereof is a full-length protein of the extracellular domain of human IgG Fc or a fragment protein thereof. In some embodiments, the ACE2-Fc fusion protein comprises: a human ACE2 protein or a fragment thereof and a human IgG Fc protein or a functional variant thereof. In some embodiments, the ACE2-Fc fusion protein comprises a full-length protein/polypeptide of the extracellular domain of human ACE2 or a fragment protein/polypeptide thereof and a human IgG Fc or a fragment protein/polypeptide thereof.

In some embodiments, the ACE2-Fc fusion protein comprises the sequence as set forth in SEQ ID NO: 1 or a fragment thereof. In some embodiments, the ACE2-Fc fusion protein comprises: an amino acid sequence having at least or about 75%, 80%, 85%, 90%, 92%, 95%, 96%, 97%, 98%, or 99% sequence identity to SEQ ID NO: 1 as shown below, or a fragment thereof. In some embodiments, the ACE2-Fc fusion protein comprises an amino acid fragment (RS) connecting the ACE2 and Fc sequences.

MSSSSWLLLSLVAVTAAQSTIEEQAKTFLDKFNHEAEDLFYQSSLASWNYNTNITEENVQNMN
NAGDKWSAFLKEQSTLAQMYPLQEIQNLTVKLQLQALQQNGSSVLSEDKSKRLNTILNTMSTI
YSTGKVCNPDNPQECLLLEPGLNEIMANSLDYNERLWAWESWRSEVGKQLRPLYEEYVVLKNE
MARANHYEDYGDYWRGDYEVNGVDGYDYSRGQLIEDVEHTFEEIKPLYEHLHAYVRAKLMNAY
PSYISPIGCLPAHLLGDMWGRFWTNLYSLTVPFGQKPNIDVTDAMVDQAWDAQRIFKEAEKFF
VSVGLPNMTQGFWENSMLTDPGNVQKAVCHPTAWDLGKGDFRILMCTKVTMDDFLTAHHEMGH
IQYDMAYAAQPFLLRNGANEGFHEAVGEIMSLSAATPKHLKSIGLLSPDFQEDNETEINFLLK
QALTIVGTLPFTYMLEKWRWMVFKGEIPKDQWMKKWWEMKREIVGVVEPVPHDETYCDPASLF
HVSNDYSFIRYYTRTLYQFQFQEALCQAAKHEGPLHKCDISNSTEAGQKLFNMLRLGKSEPWT
LALENVVGAKNMNVRPLLNYFEPLFTWLKDQNKNSFVGWSTDWSPYADQSIKVRISLKSALGD
KAYEWNDNEMYLFRSSVAYAMRQYFLKVKNQMILFGEEDVRVANLKPRISFNFFVTAPKNVSD
IIPRTEVEKAIRMSRSRINDAFRLNDNSLEFLGIQPTLGPPNQPPRSEPKSCDKTHTCPPCPA
PELLGGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPPSRDE
LTKNQVSLTCLVKGFYPSDIAVEWESNGQPENNYKTTPPVLDSDGSFFLYSKLTVDKSRWQQG
NVFSCSVMHEALHNHYTQKSLSLSPGKDSNLWN (SEQ ID NO: 1)

In some embodiments, the ACE2-Fc fusion protein comprises the sequence as set forth in SEQ ID NO: 2 or a fragment thereof. In some embodiments, the ACE2-Fc fusion protein comprises: an amino acid sequence having at least or about 75%, 80%, 85%, 90%, 92%, 95%, 96%, 97%, 98%, or 99% sequence identity to SEQ ID NO: 2 as shown below; or a fragment thereof.

QSTIEEQAKTFLDKFNHEAEDLFYQSSLASWNYNTNITEENVQNMNNAGDKWSAFLKEQSTLA
QMYPLQEIQNLTVKLQLQALQQNGSSVLSEDKSKRLNTILNTMSTIYSTGKVCNPDNPQECLL
LEPGLNEIMANSLDYNERLWAWESWRSEVGKQLRPLYEEYVVLKNEMARANHYEDYGDYWRGD
YEVNGVDGYDYSRGQLIEDVEHTFEEIKPLYEHLHAYVRAKLMNAYPSYISPIGCLPAHLLGD
MWGRFWTNLYSLTVPFGQKPNIDVTDAMVDQAWDAQRIFKEAEKFFVSVGLPNMTQGFWENSM
LTDPGNVQKAVCHPTAWDLGKGDFRILMCTKVTMDDFLTAHHEMGHIQYDMAYAAQPFLLRNG
ANEGFHEAVGEIMSLSAATPKHLKSIGLLSPDFQEDNETEINFLLKQALTIVGTLPFTYMLEK
WRWMVFKGEIPKDQWMKKWWEMKREIVGVVEPVPHDETYCDPASLFHVSNDYSFIRYYTRTLY
QFQFQEALCQAAKHEGPLHKCDISNSTEAGQKLFNMLRLGKSEPWTLALENVVGAKNMNVRPL
LNYFEPLFTWLKDQNKNSFVGWSTDWSPYADQSIKVRISLKSALGDKAYEWNDNEMYLFRSSV
AYAMRQYFLKVKNQMILFGEEDVRVANLKPRISFNFFVTAPKNVSDIIPRTEVEKAIRMSRSR
INDAFRLNDNSLEFLGIQPTLGPPNQPPRSEPKSCDKTHTCPPCPAPELLGGPSVFLFPPKPK
DTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVMHEALHNHYT
QKSLSLSPGK (SEQ ID NO: 2)

In some embodiments, the ACE2-Fc fusion protein comprises the sequence as set forth in SEQ ID NO: 3, or a fragment thereof. In some embodiments, the ACE2-Fc fusion protein comprises an amino acid sequence having at least or about 75%, 80%, 85%, 90%, 92%, 95%, 96%, 97%, 98%, or 99% sequence identity to SEQ ID NO: 3 as shown below, or a fragment thereof.

MSSSSWLLLSLVAVTAAQSTIEEQAKTFLDKFNHEAEDLFYQSSLASWNYNTNITEENVQNMN
NAGDKWSAFLKEQSTLAQMYPLQEIQNLTVKLQLQALQQNGSSVLSEDKSKRLNTILNTMSTI
YSTGKVCNPDNPQECLLLEPGLNEIMANSLDYNERLWAWESWRSEVGKQLRPLYEEYVVLKNE
MARANHYEDYGDYWRGDYEVNGVDGYDYSRGQLIEDVEHTFEEIKPLYEHLHAYVRAKLMNAY
PSYISPIGCLPAHLLGDMWGRFWTNLYSLTVPFGQKPNIDVTDAMVDQAWDAQRIFKEAEKFF
VSVGLPNMTQGFWENSMLTDPGNVQKAVCHPTAWDLGKGDFRILMCTKVTMDDFLTAHHEMGH
IQYDMAYAAQPFLLRNGANEGFHEAVGEIMSLSAATPKHLKSIGLLSPDFQEDNETEINFLLK
QALTIVGTLPFTYMLEKWRWMVFKGEIPKDQWMKKWWEMKREIVGVVEPVPHDETYCDPASLF
HVSNDYSFIRYYTRTLYQFQFQEALCQAAKHEGPLHKCDISNSTEAGQKLFNMLRLGKSEPWT
LALENVVGAKNMNVRPLLNYFEPLFTWLKDQNKNSFVGWSTDWSPYADQSIKVRISLKSALGD
KAYEWNDNEMYLFRSSVAYAMRQYFLKVKNQMILFGEEDVRVANLKPRISFNFFVTAPKNVSD
IIPRTEVEKAIRMSRSRINDAFRLNDNSLEFLGIQPTLGPPNQPPRSEPKSCDKTHTCPPCPA
PELLGGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPPSRDE
LTKNQVSLTCLVKGFYPSDIAVEWESNGQPENNYKTTPPVLDSDGSFFLYSKLTVDKSRWQQG
NVFSCSVMHEALHNHYTQKSLSLSPGK (SEQ ID NO: 3)

In some embodiments, the ACE2-Fc fusion protein comprises the sequence as set forth in SEQ ID NO: 4 or a fragment thereof. In some embodiments, the ACE2-Fc fusion protein comprises an amino acid sequence having at least or about 75%, 80%, 85%, 90%, 92%, 95%, 96%, 97%, 98%, or 99% sequence identity to SEQ ID NO: 4 as shown below, or a fragment thereof.

QSTIEEQAKTFLDKFNHEAEDLFYQSSLASWNYNTNITEENVQNMNNAGDKWSAFLKEQSTLA
QMYPLQEIQNLTVKLQLQALQQNGSSVLSEDKSKRLNTILNTMSTIYSTGKVCNPDNPQECLL
LEPGLNEIMANSLDYNERLWAWESWRSEVGKQLRPLYEEYVVLKNEMARANHYEDYGDYWRGD
YEVNGVDGYDYSRGQLIEDVEHTFEEIKPLYEHLHAYVRAKLMNAYPSYISPIGCLPAHLLGD
MWGRFWTNLYSLTVPFGQKPNIDVTDAMVDQAWDAQRIFKEAEKFFVSVGLPNMTQGFWENSM
LTDPGNVQKAVCHPTAWDLGKGDFRILMCTKVTMDDFLTAHHEMGHIQYDMAYAAQPFLLRNG
ANEGFHEAVGEIMSLSAATPKHLKSIGLLSPDFQEDNETEINFLLKQALTIVGTLPFTYMLEK
WRWMVFKGEIPKDQWMKKWWEMKREIVGVVEPVPHDETYCDPASLFHVSNDYSFIRYYTRTLY
QFQFQEALCQAAKHEGPLHKCDISNSTEAGQKLFNMLRLGKSEPWTLALENVVGAKNMNVRPL
LNYFEPLFTWLKDQNKNSFVGWSTDWSPYADQSIKVRISLKSALGDKAYEWNDNEMYLFRSSV

AYAMRQYFLKVKNQMILFGEEDVRVANLKPRISFNFFVTAPKNVSDIIPRTEVEKAIRMSRSR
INDAFRLNDNSLEFLGIQPTLGPPNQPPRSEPKSCDKTHTCPPCPAPELLGGPSVFLFPPKPK
DTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVMHEALHNHYT
QKSLSLSPGKDSNLWN (SEQ ID NO: 4)

In some embodiments, the ACE2-Fc fusion protein comprises the sequence as set forth in SEQ ID NO: 15 or a fragment thereof. In some embodiments, the ACE2-Fc fusion protein comprises an amino acid sequence having at least or about 75%, 80%, 85%, 90%, 92%, 95%, 96%, 97%, 98%, or 99% sequence identity to SEQ ID NO: 15 as shown below, or a fragment thereof. In some embodiments, the ACE2-Fc fusion protein comprises an amino acid fragment VS connecting the ACE2 and Fc sequences.

MSSSSWLLLSLVAVTAAQSTIEEQAKTFLDKFNHEAEDLFYQSSLASWNYNTNITEENVQNMN
NAGDKWSAFLKEQSTLAQMYPLQEIQNLTVKLQLQALQQNGSSVLSEDKSKRLNTILNTMSTI
YSTGKVCNPDNPQECLLLEPGLNEIMANSLDYNERLWAWESWRSEVGKQLRPLYEEYVVLKNE
MARANHYEDYGDYWRGDYEVNGVDGYDYSRGQLIEDVEHTFEEIKPLYEHLHAYVRAKLMNAY
PSYISPIGCLPAHLLGDMWGRFWTNLYSLTVPFGQKPNIDVTDAMVDQAWDAQRIFKEAEKFF
VSVGLPNMTQGFWENSMLTDPGNVQKAVCHPTAWDLGKGDFRILMCTKVTMDDFLTAHHEMGH
IQYDMAYAAQPFLLRNGANEGFHEAVGEIMSLSAATPKHLKSIGLLSPDFQEDNETEINFLLK
QALTIVGTLPFTYMLEKWRWMVFKGEIPKDQWMKKWWEMKREIVGVVEPVPHDETYCDPASLF
HVSNDYSFIRYYTRTLYQFQFQEALCQAAKHEGPLHKCDISNSTEAGQKLFNMLRLGKSEPWT
LALENVVGAKNMNVRPLLNYFEPLFTWLKDQNKNSFVGWSTDWSPYADQSIKVRISLKSALGD
KAYEWNDNEMYLFRSSVAYAMRQYFLKVKNQMILFGEEDVRVANLKPRISFNFFVTAPKNVSD
IIPRTEVEKAIRMSRSRINDAFRLNDNSLEFLGIQPTLGPPNQPPVSEPKSCDKTHTCPPCPA
PELLGGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPPSRDE
LTKNQVSLTCLVKGFYPSDIAVEWESNGQPENNYKTTPPVLDSDGSFFLYSKLTVDKSRWQQG
NVFSCSVMHEALHNHYTQKSLSLSPGKDSNLWN (SEQ ID NO: 15)

In some embodiments, the ACE2-Fc fusion protein comprises the sequence as set forth in SEQ ID NO: 16 or a fragment thereof. In some embodiments, the ACE2-Fc fusion protein comprises an amino acid sequence having at least or about 75%, 80%, 85%, 90%, 92%, 95%, 96%, 97%, 98%, or 99% sequence identity to SEQ ID NO: 16 as shown below, or a fragment thereof.

QSTIEEQAKTFLDKFNHEAEDLFYQSSLASWNYNTNITEENVQNMNNAGDKWSAFLKEQSTLA
QMYPLQEIQNLTVKLQLQALQQNGSSVLSEDKSKRLNTILNTMSTIYSTGKVCNPDNPQECLL
LEPGLNEIMANSLDYNERLWAWESWRSEVGKQLRPLYEEYVVLKNEMARANHYEDYGDYWRGD
YEVNGVDGYDYSRGQLIEDVEHTFEEIKPLYEHLHAYVRAKLMNAYPSYISPIGCLPAHLLGD
MWGRFWTNLYSLTVPFGQKPNIDVTDAMVDQAWDAQRIFKEAEKFFVSVGLPNMTQGFWENSM
LTDPGNVQKAVCHPTAWDLGKGDFRILMCTKVTMDDFLTAHHEMGHIQYDMAYAAQPFLLRNG
ANEGFHEAVGEIMSLSAATPKHLKSIGLLSPDFQEDNETEINFLLKQALTIVGTLPFTYMLEK
WRWMVFKGEIPKDQWMKKWWEMKREIVGVVEPVPHDETYCDPASLFHVSNDYSFIRYYTRTLY
QFQFQEALCQAAKHEGPLHKCDISNSTEAGQKLFNMLRLGKSEPWTLALENVVGAKNMNVRPL
LNYFEPLFTWLKDQNKNSFVGWSTDWSPYADQSIKVRISLKSALGDKAYEWNDNEMYLFRSSV
AYAMRQYFLKVKNQMILFGEEDVRVANLKPRISFNFFVTAPKNVSDIIPRTEVEKAIRMSRSR
INDAFRLNDNSLEFLGIQPTLGPPNQPPVSEPKSCDKTHTCPPCPAPELLGGPSVFLFPPKPK
DTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVMHEALHNHYT
QKSLSLSPGK (SEQ ID NO: 16)

In some embodiments, the ACE2-Fc fusion protein comprises the sequence as set forth in SEQ ID NO: 17 or a fragment thereof. In some embodiments, the ACE2-Fc fusion protein comprises an amino acid sequence having at least or about 75%, 80%, 85%, 90%, 92%, 95%, 96%, 97%, 98%, or 99% sequence identity to SEQ ID NO: 17 as shown below, or a fragment thereof.

MSSSSWLLLSLVAVTAAQSTIEEQAKTFLDKFNHEAEDLFYQSSLASWNYNTNITEENVQNMN
NAGDKWSAFLKEQSTLAQMYPLQEIQNLTVKLQLQALQQNGSSVLSEDKSKRLNTILNTMSTI
YSTGKVCNPDNPQECLLLEPGLNEIMANSLDYNERLWAWESWRSEVGKQLRPLYEEYVVLKNE
MARANHYEDYGDYWRGDYEVNGVDGYDYSRGQLIEDVEHTFEEIKPLYEHLHAYVRAKLMNAY
PSYISPIGCLPAHLLGDMWGRFWTNLYSLTVPFGQKPNIDVTDAMVDQAWDAQRIFKEAEKFF
VSVGLPNMTQGFWENSMLTDPGNVQKAVCHPTAWDLGKGDFRILMCTKVTMDDFLTAHHEMGH
IQYDMAYAAQPFLLRNGANEGFHEAVGEIMSLSAATPKHLKSIGLLSPDFQEDNETEINFLLK
QALTIVGTLPFTYMLEKWRWMVFKGEIPKDQWMKKWWEMKREIVGVVEPVPHDETYCDPASLF
HVSNDYSFIRYYTRTLYQFQFQEALCQAAKHEGPLHKCDISNSTEAGQKLFNMLRLGKSEPWT
LALENVVGAKNMNVRPLLNYFEPLFTWLKDQNKNSFVGWSTDWSPYADQSIKVRISLKSALGD
KAYEWNDNEMYLFRSSVAYAMRQYFLKVKNQMILFGEEDVRVANLKPRISFNFFVTAPKNVSD
IIPRTEVEKAIRMSRSRINDAFRLNDNSLEFLGIQPTLGPPNQPPVSEPKSCDKTHTCPPCPA
PELLGGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPPSRDE
LTKNQVSLTCLVKGFYPSDIAVEWESNGQPENNYKTTPPVLDSDGSFFLYSKLTVDKSRWQQG
NVFSCSVMHEALHNHYTQKSLSLSPGK (SEQ ID NO: 17)

In some embodiments, the ACE2-Fc fusion protein comprises the sequence as set forth in SEQ ID NO: 18 or a fragment thereof. In some embodiments, the ACE2-Fc fusion protein comprises an amino acid sequence having at least or about 75%, 80%, 85%, 90%, 92%, 95%, 96%, 97%, 98%, or 99% sequence identity to SEQ ID NO: 18 as shown below, or a fragment thereof.

QSTIEEQAKTFLDKFNHEAEDLFYQSSLASWNYNTNITEENVQNMNNAGDKWSAFLKEQSTLA
QMYPLQEIQNLTVKLQLQALQQNGSSVLSEDKSKRLNTILNTMSTIYSTGKVCNPDNPQECLL
LEPGLNEIMANSLDYNERLWAWESWRSEVGKQLRPLYEEYVVLKNEMARANHYEDYGDYWRGD
YEVNGVDGYDYSRGQLIEDVEHTFEEIKPLYEHLHAYVRAKLMNAYPSYISPIGCLPAHLLGD
MWGRFWTNLYSLTVPFGQKPNIDVTDAMVDQAWDAQRIFKEAEKFFVSVGLPNMTQGFWENSM
LTDPGNVQKAVCHPTAWDLGKGDFRILMCTKVTMDDFLTAHHEMGHIQYDMAYAAQPFLLRNG
ANEGFHEAVGEIMSLSAATPKHLKSIGLLSPDFQEDNETEINFLLKQALTIVGTLPFTYMLEK
WRWMVFKGEIPKDQWMKKWWEMKREIVGVVEPVPHDETYCDPASLFHVSNDYSFIRYYTRTLY
QFQFQEALCQAAKHEGPLHKCDISNSTEAGQKLFNMLRLGKSEPWTLALENVVGAKNMNVRPL
LNYFEPLFTWLKDQNKNSFVGWSTDWSPYADQSIKVRISLKSALGDKAYEWNDNEMYLFRSSV
AYAMRQYFLKVKNQMILFGEEDVRVANLKPRISFNFFVTAPKNVSDIIPRTEVEKAIRMSRSR
INDAFRLNDNSLEFLGIQPTLGPPNQPPVSEPKSCDKTHTCPPCPAPELLGGPSVFLFPPKPK
DTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVMHEALHNHYT
QKSLSLSPGKDSNLWN (SEQ ID NO: 18)

In some embodiments, the ACE2-Fc fusion protein comprises the ACE2 extracellular domain sequence as set forth in SEQ ID NO: 5 or a fragment thereof. In some embodiments, the ACE2-Fc fusion protein comprises an amino acid sequence having at least or about 75%, 80%, 85%, 90%, 92%, 95%, 96%, 97%, 98%, or 99% sequence identity to SEQ ID NO: 5 as shown below, or a fragment thereof.

QSTIEEQAKTFLDKFNHEAEDLFYQSSLASWNYNTNITEENVQNMNNAGDKWSAFLKEQSTLA
QMYPLQEIQNLTVKLQLQALQQNGSSVLSEDKSKRLNTILNTMSTIYSTGKVCNPDNPQECLL
LEPGLNEIMANSLDYNERLWAWESWRSEVGKQLRPLYEEYVVLKNEMARANHYEDYGDYWRGD
YEVNGVDGYDYSRGQLIEDVEHTFEEIKPLYEHLHAYVRAKLMNAYPSYISPIGCLPAHLLGD
MWGRFWTNLYSLTVPFGQKPNIDVTDAMVDQAWDAQRIFKEAEKFFVSVGLPNMTQGFWENSM
LTDPGNVQKAVCHPTAWDLGKGDFRILMCTKVTMDDFLTAHHEMGHIQYDMAYAAQPFLLRNG
ANEGFHEAVGEIMSLSAATPKHLKSIGLLSPDFQEDNETEINFLLKQALTIVGTLPFTYMLEK
WRWMVFKGEIPKDQWMKKWWEMKREIVGVVEPVPHDETYCDPASLFHVSNDYSFIRYYTRTLY

QFQFQEALCQAAKHEGPLHKCDISNSTEAGQKLFNMLRLGKSEPWTLALENVVGAKNMNVRPL
LNYFEPLFTWLKDQNKNSFVGWSTDWSPYADQSIKVRISLKSALGDKAYEWNDNEMYLFRSSV
AYAMRQYFLKVKNQMILFGEEDVRVANLKPRISFNFFVTAPKNVSDIIPRTEVEKAIRMSRSR
INDAFRLNDNSLEFLGIQPTLGPPNQPP (SEQ ID NO: 5)

In some embodiments, the ACE2-Fc fusion protein comprises the ACE2 extracellular domain sequence as set forth in SEQ ID NO: 11 or a fragment thereof. In some embodiments, the ACE2-Fc fusion protein comprises an amino acid sequence having at least or about 75%, 80%, 85%, 90%, 92%, 95%, 96%, 97%, 98%, or 99% sequence identity to SEQ ID NO: 11 as shown below, or a fragment thereof.

MSSSSWLLLSLVAVTAAQSTIEEQAKTFLDKFNHEAEDLFYQSSLASWNYNTNITEENVQNMN
NAGDKWSAFLKEQSTLAQMYPLQEIQNLTVKLQLQALQQNGSSVLSEDKSKRLNTILNTMSTI
YSTGKVCNPDNPQECLLLEPGLNEIMANSLDYNERLWAWESWRSEVGKQLRPLYEEYVVLKNE
MARANHYEDYGDYWRGDYEVNGVDGYDYSRGQLIEDVEHTFEEIKPLYEHLHAYVRAKLMNAY
PSYISPIGCLPAHLLGDMWGRFWTNLYSLTVPFGQKPNIDVTDAMVDQAWDAQRIFKEAEKFF
VSVGLPNMTQGFWENSMLTDPGNVQKAVCHPTAWDLGKGDFRILMCTKVTMDDFLTAHHEMGH
IQYDMAYAAQPFLLRNGANEGFHEAVGEIMSLSAATPKHLKSIGLLSPDFQEDNETEINFLLK
QALTIVGTLPFTYMLEKWRWMVFKGEIPKDQWMKKWWEMKREIVGVVEPVPHDETYCDPASLF
HVSNDYSFIRYYTRTLYQFQFQEALCQAAKHEGPLHKCDISNSTEAGQKLFNMLRLGKSEPWT
LALENVVGAKNMNVRPLLNYFEPLFTWLKDQNKNSFVGWSTDWSPYADQSIKVRISLKSALGD
KAYEWNDNEMYLFRSSVAYAMRQYFLKVKNQMILFGEEDVRVANLKPRISFNFFVTAPKNVSD
IIPRTEVEKAIRMSRSRINDAFRLNDNSLEFLGIQPTLGPPNQPPRS (SEQ ID NO: 11)

In some embodiments, the ACE2-Fc fusion protein comprises the ACE2 extracellular domain sequence as set forth in SEQ ID NO: 12 or a fragment thereof. In some embodiments, the ACE2-Fc fusion protein comprises an amino acid sequence having at least or about 75%, 80%, 85%, 90%, 92%, 95%, 96%, 97%, 98%, or 99% sequence identity to SEQ ID NO: 12 as shown below, or a fragment thereof.

MSSSSWLLLSLVAVTAAQSTIEEQAKTFLDKFNHEAEDLFYQSSLASWNYNTNITEENVQNMN
NAGDKWSAFLKEQSTLAQMYPLQEIQNLTVKLQLQALQQNGSSVLSEDKSKRLNTILNTMSTI
YSTGKVCNPDNPQECLLLEPGLNEIMANSLDYNERLWAWESWRSEVGKQLRPLYEEYVVLKNE
MARANHYEDYGDYWRGDYEVNGVDGYDYSRGQLIEDVEHTFEEIKPLYEHLHAYVRAKLMNAY
PSYISPIGCLPAHLLGDMWGRFWTNLYSLTVPFGQKPNIDVTDAMVDQAWDAQRIFKEAEKFF
VSVGLPNMTQGFWENSMLTDPGNVQKAVCHPTAWDLGKGDFRILMCTKVTMDDFLTAHHEMGH
IQYDMAYAAQPFLLRNGANEGFHEAVGEIMSLSAATPKHLKSIGLLSPDFQEDNETEINFLLK
QALTIVGTLPFTYMLEKWRWMVFKGEIPKDQWMKKWWEMKREIVGVVEPVPHDETYCDPASLF
HVSNDYSFIRYYTRTLYQFQFQEALCQAAKHEGPLHKCDISNSTEAGQKLFNMLRLGKSEPWT
LALENVVGAKNMNVRPLLNYFEPLFTWLKDQNKNSFVGWSTDWSPYADQSIKVRISLKSALGD
KAYEWNDNEMYLFRSSVAYAMRQYFLKVKNQMILFGEEDVRVANLKPRISFNFFVTAPKNVSD
IIPRTEVEKAIRMSRSRINDAFRLNDNSLEFLGIQPTLGPPNQPPVS (SEQ ID NO: 12)

In some embodiments, the ACE2-Fc fusion protein comprises the ACE2 extracellular domain sequence as set forth in SEQ ID NO: 13 or a fragment thereof. In some embodiments, the ACE2-Fc fusion protein comprises an amino acid sequence having at least or about 75%, 80%, 85%, 90%, 92%, 95%, 96%, 97%, 98%, or 99% sequence identity to SEQ ID NO: 13 as shown below, or a fragment thereof.

QSTIEEQAKTFLDKFNHEAEDLFYQSSLASWNYNTNITEENVQNMNNAGDKWSAFLKEQSTLA
QMYPLQEIQNLTVKLQLQALQQNGSSVLSEDKSKRLNTILNTMSTIYSTGKVCNPDNPQECLL
LEPGLNEIMANSLDYNERLWAWESWRSEVGKQLRPLYEEYVVLKNEMARANHYEDYGDYWRGD
YEVNGVDGYDYSRGQLIEDVEHTFEEIKPLYEHLHAYVRAKLMNAYPSYISPIGCLPAHLLGD
MWGRFWTNLYSLTVPFGQKPNIDVTDAMVDQAWDAQRIFKEAEKFFVSVGLPNMTQGFWENSM
LTDPGNVQKAVCHPTAWDLGKGDFRILMCTKVTMDDFLTAHHEMGHIQYDMAYAAQPFLLRNG
ANEGFHEAVGEIMSLSAATPKHLKSIGLLSPDFQEDNETEINFLLKQALTIVGTLPFTYMLEK
WRWMVFKGEIPKDQWMKKWWEMKREIVGVVEPVPHDETYCDPASLFHVSNDYSFIRYYTRTLY
QFQFQEALCQAAKHEGPLHKCDISNSTEAGQKLFNMLRLGKSEPWTLALENVVGAKNMNVRPL

LNYFEPLFTWLKDQNKNSFVGWSTDWSPYADQSIKVRISLKSALGDKAYEWNDNEMYLFRSSV
AYAMRQYFLKVKNQMILFGEEDVRVANLKPRISFNFFVTAPKNVSDIIPRTEVEKAIRMSRSR
INDAFRLNDNSLEFLGIQPTLGPPNQPPRS (SEQ ID NO: 13)

In some embodiments, the ACE2-Fc fusion protein comprises the ACE2 extracellular domain sequence as set forth in SEQ ID NO: 14 or a fragment thereof. In some embodiments, the ACE2-Fc fusion protein comprises an amino acid sequence having at least or about 75%, 80%, 85%, 90%, 92%, 95%, 96%, 97%, 98%, or 99% sequence identity to SEQ ID NO: 14 as shown below, or a fragment thereof.

QSTIEEQAKTFLDKFNHEAEDLFYQSSLASWNYNTNITEENVQNMNNAGDKWSAFLKEQSTLA
QMYPLQEIQNLTVKLQLQALQQNGSSVLSEDKSKRLNTILNTMSTIYSTGKVCNPDNPQECLL
LEPGLNEIMANSLDYNERLWAWESWRSEVGKQLRPLYEEYVVLKNEMARANHYEDYGDYWRGD
YEVNGVDGYDYSRGQLIEDVEHTFEEIKPLYEHLHAYVRAKLMNAYPSYISPIGCLPAHLLGD
MWGRFWTNLYSLTVPFGQKPNIDVTDAMVDQAWDAQRIFKEAEKFFVSVGLPNMTQGFWENSM
LTDPGNVQKAVCHPTAWDLGKGDFRILMCTKVTMDDFLTAHHEMGHIQYDMAYAAQPFLLRNG
ANEGFHEAVGEIMSLSAATPKHLKSIGLLSPDFQEDNETEINFLLKQALTIVGTLPFTYMLEK
WRWMVFKGEIPKDQWMKKWWEMKREIVGVVEPVPHDETYCDPASLFHVSNDYSFIRYYTRTLY
QFQFQEALCQAAKHEGPLHKCDISNSTEAGQKLFNMLRLGKSEPWTLALENVVGAKNMNVRPL
LNYFEPLFTWLKDQNKNSFVGWSTDWSPYADQSIKVRISLKSALGDKAYEWNDNEMYLFRSSV
AYAMRQYFLKVKNQMILFGEEDVRVANLKPRISFNFFVTAPKNVSDIIPRTEVEKAIRMSRSR
INDAFRLNDNSLEFLGIQPTLGPPNQPPVS (SEQ ID NO: 14)

In some embodiments, the ACE2-Fc fusion protein comprises the ACE2 extracellular domain sequence as set forth in SEQ ID NO: 7 or a fragment thereof. In some embodiments, the ACE2-Fc fusion protein comprises an amino acid sequence having at least or about 75%, 80%, 85%, 90%, 92%, 95%, 96%, 97%, 98%, or 99% sequence identity to SEQ ID NO: 7 as shown below, or a fragment thereof.

MSSSSWLLLSLVAVTAAQSTIEEQAKTFLDKFNHEAEDLFYQSSLASWNYNTNITEENVQNMN
NAGDKWSAFLKEQSTLAQMYPLQEIQNLTVKLQLQALQQNGSSVLSEDKSKRLNTILNTMSTI
YSTGKVCNPDNPQECLLLEPGLNEIMANSLDYNERLWAWESWRSEVGKQLRPLYEEYVVLKNE
MARANHYEDYGDYWRGDYEVNGVDGYDYSRGQLIEDVEHTFEEIKPLYEHLHAYVRAKLMNAY
PSYISPIGCLPAHLLGDMWGRFWTNLYSLTVPFGQKPNIDVTDAMVDQAWDAQRIFKEAEKFF
VSVGLPNMTQGFWENSMLTDPGNVQKAVCHPTAWDLGKGDFRILMCTKVTMDDFLTAHHEMGH
IQYDMAYAAQPFLLRNGANEGFHEAVGEIMSLSAATPKHLKSIGLLSPDFQEDNETEINFLLK
QALTIVGTLPFTYMLEKWRWMVFKGEIPKDQWMKKWWEMKREIVGVVEPVPHDETYCDPASLF
HVSNDYSFIRYYTRTLYQFQFQEALCQAAKHEGPLHKCDISNSTEAGQKLFNMLRLGKSEPWT
LALENVVGAKNMNVRPLLNYFEPLFTWLKDQNKNSFVGWSTDWSPYADQSIKVRISLKSALGD
KAYEWNDNEMYLFRSSVAYAMRQYFLKVKNQMILFGEEDVRVANLKPRISFNFFVTAPKNVSD
IIPRTEVEKAIRMSRSRINDAFRLNDNSLEFLGIQPTLGPPNQPP (SEQ ID NO: 7)

In some embodiments, the ACE2-Fc fusion protein comprises the Fc sequence as set forth in SEQ ID NO: 6 or a fragment thereof. In some embodiments, the ACE2-Fc fusion protein comprises an amino acid sequence having at least or about 75%, 80%, 85%, 90%, 92%, 95%, 96%, 97%, 98%, or 99% sequence identity to SEQ ID NO: 6 as shown below, or a fragment thereof.

EPKSCDKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEKTISKAKG
QPREPQVYTLPPSRDELTKNQVSLTCLVKGFYPSDIAVEWESNGQPENNYKTTPPVLDSDGSF
FLYSKLTVDKSRWQQGNVFSCSVMHEALHNHYTQKSLSLSPGK (SEQ ID NO: 6)

In some embodiments, the ACE2-Fc fusion protein comprises the Fc tag sequence as set forth in SEQ ID NO: 8 or a fragment thereof. In some embodiments, the ACE2-Fc fusion protein comprises an amino acid sequence having at least or about 75%, 80%, 85%, 90%, 92%, 95%, 96%, 97%, 98%, or 99% sequence identity to SEQ ID NO: 8 as shown below, or a fragment thereof.

EPKSCDKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEKTISKAKG
QPREPQVYTLPPSRDELTKNQVSLTCLVKGFYPSDIAVEWESNGQPENNYKTTPPVLDSDGSF
FLYSKLTVDKSRWQQGNVFSCSVMHEALHNHYTQKSLSLSPGKDSNLWN (SEQ ID NO: 8)

In some embodiments, the fusion protein of the present disclosure further comprises a signal peptide. In some embodiments, the signal peptide comprises the amino acid sequence as set forth in SEQ ID NO: 9 or a fragment thereof. In some embodiments, the signal peptide comprises an amino acid sequence having at least or about 75%, 80%, 85%, 90%, 92%, 95%, 96%, 97%, 98%, or 99% sequence identity to SEQ ID NO: 9, or a fragment thereof.

In some embodiments, the fusion protein of the present disclosure further comprises a mutation sequence for C-terminal modification to facilitate tracking and detection. The mutation sequence included in the ACE2-Fc fusion protein of the present disclosure comprises the sequence as set forth in SEQ ID NO: 10 or a fragment thereof. In some embodiments, the ACE2-Fc fusion protein comprises an amino acid sequence having at least or about 75%, 80%, 85%, 90%, 92%, 95%, 96%, 97%, 98%, or 99% sequence identity to SEQ ID NO: 10, or a fragment thereof.

In some embodiments, the fusion protein of the present disclosure comprises a non-dimerizing Fc domain comprising a sequence with one or more cysteine substitutions compared to human IgG1 Fc, optionally wherein the cysteine substitutions are selected from C5S, C11S, and C14S, wherein the positions of substitution are relative to SEQ ID NO: 6, and optionally wherein the cysteine is substituted with serine. In some embodiments, the fusion protein of the present disclosure comprises a non-dimerizing Fc domain comprising a sequence having at least 90%, at least 91%, at least 92%, at least 93%, at least 94%, at least 95%, at least 96%, at least 97%, at least 98%, at least 99%, or 100% sequence identity to SEQ ID NO: 5.

In some embodiments, the ACE2 protein/peptide is produced from a codon-optimized nucleic acid sequence. In some embodiments, the ACE2 protein/peptide is produced from a non-codon-optimized nucleic acid sequence.

It is contemplated that the ACE2 provided herein may form a fusion protein with Fc, and may also bind to other proteins or peptides, for example through linkage, to form further recombinant polypeptides including fusion peptides. In some embodiments, the ACE2-Fc fusion protein comprises ACE2 and an Fc protein or polypeptide. In some embodiments, the ACE2 is a primate ACE2, such as a human ACE2 protein or a fragment thereof. In some embodiments, the human ACE2 protein or a fragment thereof is a full-length extracellular domain protein of human ACE2 or a fragment protein thereof. In some embodiments, the Fc is a primate Fc, such as a human IgG Fc protein or a functional variant thereof. In some embodiments, the human IgG Fc or a fragment thereof is a full-length extracellular domain protein of human IgG Fc or a fragment protein thereof. In some embodiments, the ACE2-Fc fusion protein comprises the human ACE2 protein or a fragment thereof and a human IgG Fc protein or a functional variant thereof. In some embodiments, the ACE2-Fc fusion protein comprises the full-length extracellular domain protein/polypeptide of human ACE2 or a fragment protein/polypeptide thereof, and the human IgG Fc protein or a functional variant protein/polypeptide thereof. In some embodiments, the ACE2 protein/polypeptide is directly linked to the Fc protein/polypeptide. In some embodiments, the ACE2 or polypeptide and Fc protein or polypeptide are linked via one or more amino acids. In some embodiments, one or more peptide linkers (e.g., glycine-serine linkers, such as arginine-serine peptide linkers) may be used to connect ACE2 to Fc. In some embodiments, the linker comprises a non-dimerizing Fc domain or a fragment thereof capable of binding an Fc receptor (e.g., FcRn), thereby increasing the half-life of the fusion protein and its complexes (e.g., human serum half-life). In some embodiments, the recombinant ACE2-Fc may form dimers or multimers (e.g., trimers, tetramers, pentamers, hexamers) as long as it retains desired properties (e.g., pre-fusion conformation), and may include any stabilizing mutations (or combinations thereof) provided in the present disclosure. In some embodiments, the non-dimerizing Fc domain or a fragment thereof in a trimeric complex exists as a monomer, that is, the Fc domain or a fragment thereof does not form interchain covalent bonds such as disulfide bonds, and does not form oligomers through direct non-covalent Fc interactions between domains. In some embodiments, the recombinant polypeptide or fusion protein comprises a first sequence selected from any one as set forth in SEQ ID NOs: 5, 7, 11, 12, 13 or 14, wherein the first sequence is connected to a second sequence selected from any one as set forth in SEQ ID NOs: 6 and 8. In some embodiments, the recombinant polypeptide or fusion protein comprises a first sequence selected from any one as set forth in SEQ ID NOs: 11 or 13, wherein the first sequence is connected to a second sequence selected from any one as set forth in SEQ ID NOs: 6 and 8. In some embodiments, the first sequence and the second sequence are connected via a linker, wherein the linker is one amino acid or a plurality of amino acids, for example two amino acids: -R-S- or -V-S-. In some embodiments, the linker comprises a sequence containing glycine-X-Y repeats.

Papain digestion of antibodies yields two identical antigen-binding fragments, termed Fab fragments, and a residual Fc fragment (a name reflecting its facile crystallizability). Each Fab fragment consists of an entire light chain, the variable region of a heavy chain, and the first constant domain (CH1) of a heavy chain. Each Fab fragment is monovalent in antigen binding, containing a single antigen-binding site. The Fc fragment comprises the carboxy-terminal portions of two heavy chains linked together by disulfide bonds. The effector functions of antibodies are determined by sequences in the Fc region, which is also recognized by Fc receptors (FcRs) on certain cell types.

The Fc domain of an Fc fusion protein can bind to Fc receptors on the surface of immune cells and exert diverse biological functions, such as transplacental and transmucosal transport, inflammatory responses, antibody-dependent cellular phagocytosis (ADCP), antibody-dependent cell-mediated cytotoxicity (ADCC), complement-dependent cytotoxicity (CDC), promotion of dendritic cell (DC) maturation, modulation of cytokine secretion, and regulation of B cell proliferation and differentiation.

**Table 1: Major Fc receptors and their corresponding biological functions**

| **Receptor** | **Antibody Ligand** | **Expressing Cells** | **Ligand Affinity (L/mol)** | **Functions** |
|---|---|---|---|---|
| FcyR I (CD64) | IgG | Macrophages, monocyte, etc | 10⁷~10⁵ | Phagocytic action, cell activation, mediating ADCC effect |
| FcyR IIa (CD32) | IgG | Macrophages, neutrophils, etc | <10⁷ | Phagocytic effect, degranulation effect, mediating ADCC effect |
| FcyR IIb (CD32) | IgG | B cells, monocytes, etc | <10⁷ | Inhibit cell activation |
| FcyR IIIa (CD16a) | IgG | Macrophages, monocytes, etc | 2*10⁷ | Mediate ADCC effect and promote the secretion of cytokines by macrophages |
| FcyR IIIb (CD16b) | IgG | Neutrophils, eosinophils, etc | <10⁷ | Induced bactericidal effect |
| FcεRI | IgE | Mast cells, basophils, etc | > 10¹⁰ | Degranulation effect, release of proinflammatory mediators or cytokines |
| FcεRII (CD23) | IgE | B cell, T Cell, etc | 10⁶ | Regulatory effect |
| FcαRI (CD89) | IgA | Neutrophils, nuclear cells, etc | 2* 10⁷ | Phagocytic action, mediates ADCC effect |
| FcRn | IgG | Epithelial cells, endothelial cells | 2*10⁸ | Transport maternal IgG into the fetus; Protect IgG from degradation |

The ability of antibodies to interact with the neonatal Fc receptor (FcRn) through pH-dependent binding via their Fc (fragment, crystallizable) region can confer extended serum half-life. In some embodiments, the present disclosure provides engineered Fc-fusion molecules wherein the Fc domain prolongs the serum half-life of therapeutic proteins or peptides. Owing to the homodimeric nature of antibody Fc regions, naturally occurring IgG antibodies and engineered Fc-fusion molecules comprising native IgG sequences are typically bivalent and monospecific. For certain therapeutic applications disclosed herein, the engineered Fc polypeptides and their fusion polypeptides may retain beneficial attributes conferred by monomeric, non-dimerizing Fc while achieving flexibility and specificity.

Immunoglobulin G (IgG) is the most abundant immunoglobulin class in human serum. The IgG structure comprises two light chains and two heavy chains, each light chain consisting of two domains, each heavy chain consisting of four domains. The antigen-binding site is located in the Fab region (antigen-binding fragment), which contains a variable light chain (VL) domain and a variable heavy chain (VH) domain, a constant light chain (CL) domain and a constant heavy chain 1 (CH1) domain. The Fc region comprises the CH2 and CH3 domain regions of the heavy chains, and the two heavy chains are linked together via disulfide bonds (-S-S-) in the hinge region.

The FcRn (neonatal Fc receptor) binding site of IgG is located in the Fc region of the antibody, therefore the prolonged serum half-life characteristic of antibodies is retained in the Fc fragment. The isolated Fc fragment can be considered as a homodimer of heavy chains containing CH2 and CH3 domains. In some embodiments, the monomeric non-dimerizing Fc domain of the present disclosure comprises one or more mutations in the heavy chain Fc region to stabilize the Fc domain or its fusion polypeptide in aqueous solution/serum. In some embodiments, compared to the corresponding wild-type Fc domain, the monomeric non-dimerizing Fc domain contains three or more mutations in the Fc amino acid sequence. In some embodiments, compared to the corresponding wild-type Fc domain, the monomeric non-dimerizing Fc domain contains two mutations in the Fc amino acid sequence.

In some embodiments, the Fc domain or fusion polypeptide of the present disclosure comprises an immunoglobulin portion (e.g., hinge, CH2, and/or CH3 domains), wherein any or all cysteine residues located within the hinge domain of the immunoglobulin portion are mutated to eliminate disulfide bond formation involving one or more or all of these cysteine residues. For example, in a human IgG1 Fc domain according to the EU index, any one or more of cysteine residues at positions 220, 226, and 229 (corresponding to positions 233, 239, and 242, respectively, under Kabat numbering) may be substituted with another amino acid residue such as serine. In some embodiments, the Fc domain or fusion polypeptide of the present disclosure comprises a cysteine at position 220, which is substituted with, for example, serine. In some embodiments, the Fc domain or fusion polypeptide of the present disclosure comprises a cysteine at position 226, which is substituted with, for example, serine. In some embodiments, the Fc domain or fusion polypeptide of the present disclosure comprises a cysteine at position 229, which is substituted with, for example, serine. In some embodiments, the Fc domain or fusion polypeptide of the present disclosure further comprises one or more other amino acid substitutions, insertions, and/or deletions. In some embodiments, the Fc domain or fusion polypeptide of the present disclosure further comprises a proline at position 238 (corresponding to position 251 under Kabat numbering), which is substituted with, for example, serine.

In some embodiments, the Fc domain or fusion polypeptide disclosed herein exhibits elevated binding affinity to FcRn within a first pH range compared to a corresponding human IgG1 Fc domain or fusion polypeptide. In some embodiments, the Fc domain or fusion polypeptide of the present disclosure demonstrates rapid dissociation from FcRn within a second pH range compared to the corresponding human IgG1 Fc domain or fusion polypeptide. In some embodiments, the first pH range is lower than the second pH range. In some embodiments, the first pH range is below about 7.0 and the second pH range is above about 7.0. In some embodiments, the first pH range is between about 5.5 and about 6.5, and the second pH range is between about 7.0 and about 8.0. In some embodiments, the first pH range is about 6.0 and the second pH range is about 7.4.

In some embodiments, the Fc domain or fusion polypeptide of the present disclosure comprises mutations at positions 217, 228, 243, 262, 273, 274, 262, 273, 274, 288, 290, 298, 305, 309, 310, 321, 326, 344, 353, 356, 363, 364, 368, 375, 388, 389, 390, 397, 398, 399, 401, 405, 407, 409, 410, 413, 424, 438, and 442 (EU index numbering for human IgG1 Fc), corresponding to positions 227, 241, 256, 275, 286, 287, 305, 307, 317, 324, 328, 329, 340, 345, 365, 374, 377, 386, 387, 391, 398, 416, 417, 418, 425, 426, 427, 430, 436, 438, 440, 441, 444, 455, 469, and 473, respectively, under Kabat numbering. In some embodiments, the Fc domain or fusion polypeptide comprises one or more amino acid substitutions selected from: P217R, P228K, F243V, V262I, V273L, K274V, K288V, K288D, K288I, K288F, K290L, S298N, V305I, L309E, H310S, C321V, K326G, R344T, P353K, P353D, D356P, V363N, S364N, L368W, L368G, S375Y, E388G, N389V, N390L, V397L, L398W, D399K, D401G, F405E, F405K, F405Q, F405R, F405V, Y407S, K409N, K409D, L410N, D413R, S424G, Q438S, and S442K.

In some embodiments, the Fc domain or fusion polypeptide of the present disclosure comprises mutations at positions 221, 234, 297, 306, 312, 315, 306, 312, 315, 325, 343, 356, 401, 406, and 421 (EU index numbering for human IgG1 Fc), corresponding to positions 234, 247, 314, 325, 331, 334, 344, 364, 377, 430, 437, and 452, respectively, according to Kabat numbering. In some embodiments, the Fc domain or fusion polypeptide of the present disclosure comprises one or more amino acid substitutions selected from the group consisting of: N421H.

In some embodiments, the Fc domain or fusion polypeptide of the present disclosure comprises mutations at positions 221, 224, 270, 271, 273, 290, 271, 273, 290, 294, 305, 315, 319, 332, 343, 349, 357, 364, 368, 391, 405, 409, 424, 426, 435, 437, 438, 441, and 447 in the human IgG1 Fc domain according to the EU index of Kabat, which correspond to positions 234, 237, 283, 284, 286, 307, 311, 324, 334, 338, 341, 364, 370, 378, 387, 391, 419, 436, 440, 455, 457, 466, 468, 469, 472, and 478, respectively, according to Kabat numbering. In some embodiments, the Fc domain or fusion polypeptide of the present disclosure comprises one or more amino acid substitutions selected from the group consisting of: D221A, H224Y, D270Y, P27lS, V273L, K290L, E294D, V305F, N315D, Y319S, 1332M, P343W, Y349E, E357V, S364N, L368G, Y391R, F405E, K409V, S424G, S426G, H435P, T437G, Q438S, Q438P, Q438K, L441T, and K447F.

In some embodiments, the Fc domain or fusion polypeptide of the present disclosure comprises one or more amino acid substitutions, insertions, and/or deletions at any one or more of positions 252, 254, 256, 428, and 434 in the human IgG1 Fc domain according to the EU index of Kabat, which correspond to positions 265, 267, 269, 459, and 465, respectively, according to Kabat numbering. In some embodiments, the Fc domain or fusion polypeptide of the present disclosure comprises one or more amino acid substitutions selected from the group consisting of: M252Y, S254T, T256E, M428L, and N434S.

In some embodiments, the fusion proteins described in the present disclosure may incorporate an immunoglobulin Fc domain monomer or a fragment of an Fc domain to extend the serum half-life of the polypeptide. In some embodiments, the fusion proteins disclosed herein are incapable of forming dimers (e.g., homodimers or heterodimers) through interactions between any two Fc domain monomers within the non-covalent complexes disclosed in the present disclosure. Instead, the disclosed fusion proteins may undergo trimerization (independent of their Fc domains), thereby forming a trimeric complex comprising three Fc domain monomers, which may have identical or different sequences.

In some embodiments, the Fc domain may be mutated to lack effector functions, typically being a "dead" Fc domain that does not bind Fc receptors such as FcγRI, FcγRIIa, FcγRIIb, FcγRIIIa, FcγRIIIb, and/or FcγRIV. For example, the Fc domain may comprise specific amino acid substitutions known to minimize interactions between the Fc domain and Fcγ receptors. In some embodiments, the Fc domain is derived from an IgG1 antibody and includes the amino acid substitutions L234A, L235A, and G237A. In some embodiments, the Fc domain is derived from an IgG1 antibody and includes the amino acid substitutions D265A, K322A, and N434A. The aforementioned amino acid positions are defined according to Kabat (Sequences of Proteins of Immunological Interest, 5th Ed. Public Health Service, National Institutes of Health, Bethesda, Md. (1991)). The Kabat numbering for amino acid residues in a given antibody may be determined by aligning homologous regions of the antibody sequence with the "standard" Kabat-numbered sequence. Furthermore, in some embodiments, the Fc domain does not induce any immune system-related responses. For instance, the Fc domain within the polypeptide dimer may be modified to reduce interaction or binding between the Fc domain and Fcy receptors.

In some cases, the fusion proteins described herein comprise an Fc region of a human immunoglobulin or a fragment or variant thereof. In some embodiments, the Fc domain of the fusion protein is the Fc region of human IgG1 or a fragment or variant thereof. In some embodiments, the Fc domain of the fusion protein is N-terminally truncated compared to the native Fc region of a human immunoglobulin. In some embodiments, the Fc domain of the fusion protein does not include the CH1 domain of the native Fc region of a human immunoglobulin.

In some cases, the Fc domain of the fusion protein comprises one or more amino acid substitutions, deletions, or insertions relative to the native human IgG1 sequence. In some embodiments, the Fc domain of the fusion protein comprises substitutions at one or more amino acid positions within the CH2 domain. In some embodiments, the Fc domain of the fusion protein comprises substitutions at one or more amino acid positions in the DE loop. In specific embodiments, the Fc domain of the fusion protein comprises a substitution of the naturally occurring amino acid at position 297, wherein the substitution detectably reduces and/or eliminates glycosylation at position 297. In specific embodiments, the Fc domain of the fusion protein comprise a substitution of asparagine with cysteine at position 297 of the antibody heavy chain. In other embodiments, the Fc domain of the fusion protein lacks glycosylation at position 297. In some embodiments, the Fc domain of the fusion protein comprises an N297Q substitution. In each of these cases, the numbering system for the constant region is the EU index numbering system as defined in Kabat.

In some embodiments, the amino acid substitutions, deletions, or insertions may improve the expression, purification, or stability profile of the fusion protein. In some embodiments, the amino acid substitutions, deletions, or insertions may improve the binding affinity and specificity profile of the fusion protein. In some cases, the Fc domain of the fusion protein does not contain any cysteine residues capable of forming interchain disulfide bonds.
In some embodiments, the Fc domain of the fusion protein or complex comprises the amino acid sequence set forth in SEQ ID NO: 5. In some embodiments, the Fc domain of the fusion protein or complex comprises an amino acid sequence having at least or about 75%, 80%, 85%, 90%, 92%, 95%, 96%, 97%, 98%, or 99% sequence identity to SEQ ID NO: 5, or a fragment thereof.

In some embodiments, the recombinant fusion protein comprises the ACE2 protein or a fragment thereof as described in Section I. In some embodiments, the recombinant fusion protein comprises the ACE2 protein as described in Section I connected to the Fc protein or a functional variant thereof as described in the present disclosure.

In some embodiments, the Fc protein is produced from a codon-optimized nucleic acid sequence. In some embodiments, the Fc protein is produced from a non-codon-optimized nucleic acid sequence.

In some embodiments, the ACE2-Fc fusion protein provided herein is further conjugated to a coupling moiety selected from the group consisting of: detectable labels, drugs, toxins, cytokines, radionuclides, enzymes, gold nanoparticles/nanorods, magnetic nanoparticles, viral envelope proteins or VLPs, or combinations thereof. In some embodiments, the ACE2-Fc fusion protein is connected to the coupling moiety via a conjugation linkage.

### II. Polynucleotides and vectors

The present disclosure further provides a polynucleotide (nucleic acid molecule) encoding the ACE2-Fc fusion protein described herein, and a vector for genetically engineering cells to express the ACE2-Fc fusion protein.

In some embodiments, a polynucleotide encoding the fusion protein described herein is provided. In some aspects, the polynucleotides comprises a single nucleic acid sequence, such as that encoding the ACE2-Fc fusion protein polypeptide. In other instances, the polynucleotide comprises a first nucleic acid sequence encoding a recombinant polypeptide, particularly ACE2, and a second nucleic acid sequence encoding a recombinant polypeptide of Fc.

In some embodiments, the polynucleotide encoding the fusion protein comprises at least one promoter that is operably linked to control the expression of the recombinant polypeptide. In some embodiments, the polynucleotide comprises two, three or more promoters that are operably linked to control the expression of recombinant polypeptides.

In some embodiments, for example, when a polynucleotide comprises two or more nucleic acid coding sequences, e.g., a first nucleic acid sequence encoding ACE2 and a second nucleic acid sequence encoding a recombinant polypeptide of Fc, at least one promoter may be operably linked to control the expression of two or more nucleic acid sequences. In some embodiments, the polynucleotide comprises two, three or more promoters that are operably linked to control the expression of recombinant polypeptides.

In some embodiments, expression of the recombinant polypeptide is inducible or conditional. Accordingly, in some aspects, the polynucleotide encoding the recombinant polypeptide comprises a conditional promoter, enhancer, or transactivator. In some such aspects, the conditional promoter, enhancer, or transactivator is an inducible promoter, enhancer, or transactivator, or a repressible promoter, enhancer, or transactivator. For example, in some embodiments, the inducible or conditional promoter may be used to restrict expression of the recombinant polypeptide to a specific microenvironment. In some embodiments, expression driven by the inducible or conditional promoter is regulated by exposure to an exogenous agent (e.g., heat, radiation, or a drug).

Where the polynucleotide comprises more than one nucleic acid sequence encoding a recombinant polypeptide, the polynucleotide may further include a nucleic acid sequence encoding a peptide linker between one or more of the nucleic acid sequences. In some cases, the intervening nucleic acid encodes a peptide that mediates separation of the translation products of the nucleic acid sequences during or after translation. In some embodiments, the peptide comprises an internal ribosome entry site (IRES), a self-cleaving peptide, or a ribosome-skipping peptide (e.g., T2A peptide).

In some embodiments, a polynucleotide encoding recombinant polypeptide is introduced into compositions containing cultured cells (e.g., host cells), e.g., via retroviral transduction, transfection or transformation. In some embodiments, this allows expression (e.g., production) of recombinant polypeptides. In some embodiments, the expressed recombinant polypeptides are purified.

In some embodiments, the polynucleotide (nucleic acid molecule) provided herein encodes a nucleic acid sequence of fusion protein of ACE2-Fc as described herein. In some embodiments, the polynucleotide (nucleic acid molecule) provided in the present disclosure encode a nucleic acid sequence for fusion protein of ACE2-Fc as described in the present disclosure **I.** In some embodiments, the polynucleotide (nucleic acid molecule) provided in the present disclosure encodes a recombinant polypeptide containing ACE2 as described in the present disclosure. In some embodiments, the polynucleotide (nucleic acid molecule) provided herein encodes a recombinant polypeptide containing Fc as described herein. A vector or construct containing nucleic acid molecule as described in the present disclosure is also provided. In some embodiments, the vector or construct contains one or more promoters that are operably linked to the nucleic acid molecule encoding the recombinant polypeptide to drive its expression. In some embodiments, the promoter is operably linked to one or more nucleic acid molecules, such as those encoding a polypeptide comprising no ACE2.

In some embodiments, the vector is a viral vector. In some embodiments, the viral vector is a retroviral vector. In some embodiments, the retroviral vector is a lentiviral vector. In some embodiments, the retroviral vector is a γ-retroviral vector.

In some embodiments, a vector or construct includes a single promoter that drives the expression of one or more nucleic acid molecules of a polynucleotide. In some embodiments, such promoters can be polycistronic (bicistronic or tricistronic, see e.g., US Patent No. 6, 060, 273). For example, in some embodiments, transcription units can be engineered into bicistronic units containing the IRES (internal ribosome entry site), which allows co-expression of gene products (e.g., encoding different recombinant polypeptides) through a message from a single promoter. In some embodiments, the vector provided in the present disclosure is bicistronic, allowing the vector to comprise and express two nucleic acid sequences. In some embodiments, the vector provided in the present disclosure is tricistronic, allowing the vector to comprise and express three nucleic acid sequences.

In some embodiments, a single promoter directs expression of an RNA comprising two or three genes (e.g., encoding a chimeric signaling receptor and a recombinant receptor) within a single open reading frame (ORF), wherein the genes are separated by sequences encoding self-cleaving peptides (e.g., 2A sequences), or protease recognition sites (e.g., furin cleavage sites). Consequently, the ORF encodes a single polypeptide that is processed into individual proteins either during translation (for 2A sequences), or post-translationally. In certain cases, the peptide (e.g., T2A) can cause ribosomal skipping, wherein the ribosome bypasses formation of a peptide bond at the C-terminus of the 2A element, resulting in separation between the 2A sequence's terminus and the downstream peptide (see, e.g., de Felipe, Genetic Vaccines and Therapy 2:13 (2004*) and* de Felipe et al., Traffic 5:616-626 (2004*),* each incorporated herein by reference in their entirety). Numerous 2A elements are known in the art. Non-limiting examples of 2A sequences suitable for use in the methods and nucleic acids disclosed herein include those derived from: Foot-and-mouth disease virus (F2A), Equine rhinitis A virus (E2A), Thosea asigna virus (T2A), and Porcine teschovirus-1 (P2A), as described in U.S. Patent Publication No. 20070116690.

### III. ACE2-Fc Fusion Protein Composition and Drug Product

In some embodiments, the present disclosure provides an ACE2-Fc fusion protein composition, i.e., a composition comprising the ACE2-Fc fusion protein (pharmaceutical composition), wherein the fusion protein comprises a sequence selected from the group consisting of SEQ ID NO: 1 through SEQ ID NO: 18, or any combination thereof. In some embodiments, the present disclosure provides a composition comprising a recombinant fusion protein having the sequence as set forth in SEQ ID NO: 1, or a fragment, variant, or mutant thereof. The fusion protein composition disclosed herein, comprising a recombinant polypeptide selected from the group consisting of SEQ ID NOs: 5-8 or a fragment, variant, or mutant thereof, can be used in formulations.

In some embodiments, a single dose of the ACE2-Fc fusion protein composition may include approximately 0.1 mg to approximately 100 mg of ACE2-Fc fusion protein, preferably approximately 0.25 mg to approximately 35 mg of ACE2-Fc fusion protein, and preferably approximately 0.25 mg to approximately 15 mg of ACE2-Fc fusion protein. In some embodiments, a single dose comprises 0.25, 0.3, 0.40, 0.45, 0.50, 0.55, 0.60, 0.65, 0.75, 0.80, 0.85, 0.90, 0.95, 1.00, 1.05, 1.10, 1.25, 1.30, 1.35, 1.40, 1.45, 1.50, 1.55, 1.60, 1.65, 1.70, 1.75, 1.80, 1.85, 1.90, 2.00, 2.05, 2.10, 2.15, 2.20, 2.25, 2.30, 2.35, 2.40, 2.45, 2.50, 2.75, 3.00, 3.25, 3.5, 3.75, or 4 mg ACE2-Fc fusion protein. In some embodiments, a single dose comprises 0.5 mg ACE2-Fc fusion protein. In other embodiments, a single dose consisted of 1 mg ACE2-Fc fusion protein. In a further embodiment, the dose comprises 2.0 mg ACE2-Fc fusion protein.

In some embodiments, an ACE2-Fc fusion protein composition provided by the present disclosure may comprise from approximately 0.1 mg/ml to approximately 100 mg/ml of ACE2-Fc fusion protein. Preferably, in some embodiments, the ACE2-Fc fusion protein composition may comprise from approximately 0.1 mg/ml to approximately 95 mg/ml of ACE2-Fc fusion protein. Preferably, in some embodiments, the ACE2-Fc fusion protein composition may comprise from approximately 0.1 mg/ml to approximately 85 mg/ml of ACE2-Fc fusion protein. Preferably, in some embodiments, the ACE2-Fc fusion protein composition may comprise from approximately 0.1 mg/ml to approximately 75 mg/ml of ACE2-Fc fusion protein. Preferably, in some embodiments, the ACE2-Fc fusion protein composition may comprise from approximately 0.1 mg/ml to approximately 65, 55, 45, 35, 25, or 15 mg/ml of ACE2-Fc fusion protein. Preferably, in some embodiments, the ACE2-Fc fusion protein composition may comprise from approximately 0.1 mg/ml to approximately 5, 6, 7, 8, 9, or 10 mg/ml of ACE2-Fc fusion protein. Preferably, in some embodiments, the ACE2-Fc fusion protein composition may comprise from approximately 0.2, 0.3, 0.4, 0.5, 0.6, 0.7, 0.8, 0.9 or 1 mg/ml to approximately 5 mg/ml of ACE2-Fc fusion protein. Preferably, in some embodiments, the ACE2-Fc fusion protein composition may comprise from approximately 0.2, 0.3, 0.4, 0.5, 0.6, 0.7, 0.8, 0.9, or 1 mg/ml to approximately 4 mg/ml of ACE2-Fc fusion protein. Preferably, in some embodiments, the ACE2-Fc fusion protein composition may comprise from approximately 0.2, 0.3, 0.4, 0.5, 0.6, 0.7, 0.8, 0.9, or 1 mg/ml to approximately 3 mg/ml of ACE2-Fc fusion protein. Preferably, in some embodiments, the ACE2-Fc fusion protein composition may comprise from approximately 0.2, 0.3, 0.4, 0.5, 0.6, 0.7, 0.8, 0.9, or 1 mg/ml to approximately 2 mg/ml of ACE2-Fc fusion protein. Preferably, in some embodiments, the ACE2-Fc fusion protein composition may comprise from approximately 0.2, 0.3, 0.4, 0.5, 0.6, 0.7, 0.8, 0.9, or 1 mg/ml to approximately 1.5 mg/ml of ACE2-Fc fusion protein. Preferably, in some embodiments, the ACE2-Fc fusion protein composition may comprise from approximately 1 mg/ml to approximately 5 mg/ml of ACE2-Fc fusion protein. Preferably, in some embodiments, the ACE2-Fc fusion protein composition may comprise approximately 1.00, 1.25, 1.50, 1.75, 2.00, 2.25, 2.50, 2.75, 3.00, 3.25, 3.50, 3.75, 4.00, 4.25, 4.50, 4.75, 5.00, 5.25, 5.50, 5.75, 6.00, 6.25, 6.50, 6.75, 7.00, 7.25, 7.50, 7.75, 8.00, 8.25, 8.50, 8.75, 9.00, 9.25, 9.50, 9.75 or 10.00 mg/ml of ACE2-Fc fusion protein. Preferably, in some embodiments, the ACE2-Fc fusion protein composition may comprise approximately 1.00 mg/ml of ACE2-Fc fusion protein. Preferably, in some embodiments, the ACE2-Fc fusion protein composition may comprise approximately 1.25 mg/ml of ACE2-Fc fusion protein. Preferably, in some embodiments, the ACE2-Fc fusion protein composition may comprise approximately 2.50 mg/ml of ACE2-Fc fusion protein. Preferably, in some embodiments, the ACE2-Fc fusion protein composition may comprise approximately 5.00 mg/ml of ACE2-Fc fusion protein. Preferably, in some embodiments, the ACE2-Fc fusion protein composition may comprise approximately 10.00 mg/ml of ACE2-Fc fusion protein.

In some aspects, the fusion protein disclosed herein may be used in combination with other agents (e.g., vaccines) or pharmaceutical products capable of eliciting a protective immune response. For example, a quadrivalent influenza nasal spray vaccine may protect against influenza A (H1N1) virus, influenza A (H3N2) virus, and two influenza B viruses. The fusion protein described in the present disclosure may be administered concurrently or sequentially with vaccines or other pharmaceutical products targeting other viruses that infect humans via the ACE2 pathway in the upper respiratory tract (oral/nasal) or influenza.

The combination use provides protection against a plurality of pathogens. In some aspects, the combined use may provide protection against a plurality of strains of the same pathogen. The said combination use is necessary for minimizing the number of immunizations required to confer protection against diverse pathogens or pathogenic strains, reducing administrative costs, and improving vaccination coverage rates. For example, this may prove especially beneficial when vaccinating infants or children.

A composition containing the disclosed ACE2-Fc fusion protein and a pharmaceutically acceptable carrier is also provided. In some embodiments, the disclosed composition comprises the ACE2-Fc fusion protein provided herein and an optional pharmaceutically acceptable carrier such as water, buffer, or saline. In some embodiments, the disclosed composition comprises the ACE2-Fc fusion protein provided herein and PBS buffer or saline. In some embodiments, the disclosed composition comprises the ACE2-Fc fusion protein provided herein and PB buffer or saline. In some embodiments, the disclosed composition comprises the ACE2-Fc fusion protein provided herein and disodium phosphate dihydrate, disodium phosphate monohydrate, sodium chloride, sucrose, phenylethyl alcohol, and water; specifically, 0.1-15 mg/ml ACE2-Fc fusion protein, and 0.5-10 mg/ml disodium phosphate dihydrate, 0.01-5 mg/ml disodium phosphate monohydrate, 0.1-10 mg/ml sodium chloride, 10-100 mg/ml sucrose, 0.25-5 mg/ml phenylethyl alcohol, and an appropriate amount of water. In some embodiments, the disclosed composition comprises 1 - 5 mg/ml ACE2-Fc fusion protein, and 2.13 mg disodium phosphate dihydrate, 0.82 mg/ml disodium phosphate monohydrate, 2.92 mg/ml sodium chloride, 50 mg/ml sucrose, 2.50 mg/ml phenylethanol, and an appropriate amount of water. In some embodiments, the disclosed composition comprises 1.25 mg/ml ACE2-Fc fusion protein, and 2.13 mg disodium phosphate dihydrate, 0.82 mg/ml disodium phosphate monohydrate, 2.92 mg/ml sodium chloride, 50 mg/ml sucrose, and 2.50 mg/ml phenylethanol. In some embodiments, the disclosed composition comprises 2.0 mg/ml ACE2-Fc fusion protein, and 2.13 mg disodium phosphate dihydrate, 0.82 mg/ml disodium phosphate monohydrate, 2.92 mg/ml sodium chloride, 50 mg/ml sucrose, and 2.50 mg/ml phenylethanol. In some embodiments, the disclosed composition comprises 2.5 mg/ml ACE2-Fc fusion protein, and 2.13 mg disodium phosphate dihydrate, 0.82 mg/ml disodium phosphate monohydrate, 2.92 mg/ml sodium chloride, 50 mg/ml sucrose, and 2.50 mg/ml phenylethanol. In some embodiments, the disclosed composition comprises 5.0 mg/ml ACE2-Fc fusion protein, and 2.13 mg disodium phosphate dihydrate, 0.82 mg/ml disodium phosphate monohydrate, 2.92 mg/ml sodium chloride, 50 mg/ml sucrose, and 2.50 mg/ml phenylethanol. In some embodiments, the disclosed composition comprises the ACE2-Fc fusion protein provided herein and disodium hydrogen phosphate, e.g., disodium hydrogen phosphate dihydrate, sodium dihydrogen phosphate, e.g., disodium hydrogen phosphate monohydrate, sodium chloride and Tween 80.

In some embodiments, ACE2-Fc fusion protein composition comprises the protein nanoparticles provided herein and an optional pharmaceutically acceptable carrier. In some embodiments, ACE2-Fc fusion protein composition comprises the VLPs provided herein and an optional pharmaceutically acceptable carrier. In some embodiments, ACE2-Fc fusion protein composition comprises isolated nucleic acids provided herein and an optional pharmaceutically acceptable carrier. In some embodiments, ACE2-Fc fusion protein composition comprises a publicly available carrier and an optional pharmaceutically acceptable carrier. In some embodiments, ACE2-Fc fusion protein composition comprises a virus provided herein and an optional pharmaceutically acceptable carrier. In some embodiments, ACE2-Fc fusion protein composition comprises a pseudovirus provided herein and an optional pharmaceutically acceptable carrier. In some embodiments, ACE2-Fc fusion protein composition comprises a cell provided herein and an optional pharmaceutically acceptable carrier.

In some embodiments, ACE2-Fc fusion protein is prophylactic. In some embodiments, ACE2-Fc fusion protein is therapeutic. In some embodiments, ACE2-Fc fusion protein is a prophylactic and therapeutic agent. Such pharmaceutical composition may be administered to a subject via a variety of modes of administration known to those skilled in the art, e.g., intramuscular, intradermal, subcutaneous, intravenous, intra-arterial, intra-articular, intraperitoneal, intranasal, sublingual, tonsillar, oropharyngeal, or other parenteral and mucosal routes. Preferably, ACE2-Fc fusion protein composition is administered intranasally. Actual methods used to prepare administrable compositions are known or readily apparent to those skilled in the ar and are described in greater detail in publications such as Remingtons Pharmaceutical Sciences, 19th Ed., Mack Publishing Company, Easton, Pa., 1995.

Therefore, the ACE2-Fc fusion protein described herein can be formulated with a pharmaceutically acceptable carrier to help maintain biological activity while also facilitating increased stability during storage at acceptable temperature ranges. Potential carriers include, but are not limited to, physiologically balanced media phosphate buffered saline, water, emulsions (e.g., oil/water or water/oil emulsion), various types of wetting agents, cryoprotective additives or stabilizers such as proteins, peptides or hydrolysates (e.g., albumin, gelatin), sugars (e.g., sucrose, lactose, sorbitol), amino acids (e.g., sodium glutamate), or other protective agents. The resulting aqueous solution may be used as is or lyophilized. Lyophilized preparation is mixed with sterile solution prior to single or multiple dose administration. In some embodiments, phosphate buffered saline solutions include monobasic sodium phosphate monohydrate and dibasic sodium phosphate dihydrate.

In some embodiments, the ACE2-Fc fusion protein composition of the present invention comprises an aqueous carrier as a solvent. Suitable carriers include e.g., sterile water, saline, phosphate buffered saline, and Ringer's solution. In some embodiments, the compositionis isotonic. In some embodiments, the ACE2-Fc fusion protein composition of the present invention comprises phosphate buffered saline (PBS), such as NaCl, KCl, Na₂HPO₄, and KH₂PO₄, or for another example, NaCl, Na₂HPO₄, and NaH₂PO₄.

The formulation composition, particularly liquid preparations, may comprise one or more bacteriostatic agents to inhibit microbial growth and prevent/minimize degradation during storage, including but not limited to effective concentrations (typically 1% w/v) of benzyl alcohol, phenol, m-cresol, chlorobutanol, methylparaben, and/or propylparaben. The bacteriostatic agents may also include one or more types from the categories of essential oils, high salt, high sugar, high acid, and anaerobic agents. In some embodiments, the bacteriostatic agents described in the present disclosure include one or more of benzoic acid, sorbic acid, thimerosal, and phenylethanol. In some embodiments, the bacteriostatic agents described include thimerosal and/or phenylethanol. Bacteriostatic agents may be contraindicated in some patients; therefore, lyophilized formulations may be reconstituted in solutions containing or not containing such ingredients.

The compositions of the present invention may comprise pharmaceutically acceptable carrier substances required to approximate physiological conditions, such as pH regulators and buffering agents, tonicity agents, isotonic agents, wetting agents, etc., such as sodium acetate, sodium lactate, sodium chloride, potassium chloride, calcium chloride, sorbitan monolaurate, and triethanolamine oleate. The isotonic agents are selected from one or more of glucose, dextrose, sucrose, glycerol, mannitol, sodium chloride, and potassium chloride, with dextrose being preferred.

In some embodiments, ACE2-Fc fusion protein composition comprises pharmaceutically acceptable excipients, including, for example, solvents, fillers, buffers, tonicity agents, and bacteriostatic agents (Pramanick et al., Pharma Times, 45:65-77, 2013, which is incorporated herein by reference in its entirety for all purposes). In some embodiments, the fusion protein composition of the present disclosure may include an excipient that acts as one or more of the solvents, fillers, buffers, and tonicity agents (e.g., sodium chloride in saline may be used as both an aqueous carrier and tonicity agent).

The ACE2-Fc fusion protein composition described herein may include a buffering agent. Buffering agents control pH to inhibit active agent degradation during handling, storage, and optionally reconstitution. Suitable buffers include e.g., salts containing acetate, citrate, phosphate or sulfate. Other suitable buffers include e.g., amino acids such as arginine, glycine, histidine and lysine. Buffering agents may further include hydrochloric acid or sodium hydroxide. In some embodiments, buffers maintain the composition pH in the range of 6 to 9. In some embodiments, pH is greater than (lower limit) 6, 7 or 8. In some embodiments, pH is less than (upper limit) 9, 8 or 7. That is, pH is in the range of about 6 to 9, where the lower limit is less than the upper limit. In some embodiments, the pH is between about 5.8 and 6.8.

The ACE2-Fc fusion protein composition disclosed herein may include a tonicity agent. Suitable tonicity agents include, but are not limited to, glucose, glycerol, sodium chloride, sucrose, and mannitol.

The ACE2-Fc fusion protein composition disclosed herein may comprise a tonicity agent selected from one or more of sodium chloride, potassium chloride, glycerol, glucose, sorbitol, sucrose, xylitol, mannitol, such as sodium chloride and/or sucrose. The ACE2-Fc fusion protein composition disclosed herein may include a solvent. The solvent is selected from one or more of ethanol, propylene glycol, and purified water, such as water.

The ACE2-Fc fusion protein composition disclosed herein may comprise a filler. Fillers are particularly useful when the pharmaceutical composition is lyophilized prior to administration. In some embodiments, the filler functions as a protective agent, helping to stabilize the active agent and prevent its degradation during freezing or spray-drying processes and/or during storage. Suitable fillers include sugars (mono-, di-, and polysaccharides), such as sucrose, lactose, trehalose, mannitol, sorbitol, glucose and raffinose.

The ACE2-Fc fusion protein composition described herein may include a preservative, suitable preservatives include, for example, antioxidants and antibiotics. The preservatives comprise, but are not limited to, one or more selected from the group consisting of: benzalkonium chloride, methylparaben, propylparaben, potassium sorbate, and sodium benzoate, with potassium sorbate being preferred. The suitable antioxidants may be selected from one or more of the group consisting of: disodium EDTA (EDTA-2Na), butylated hydroxyanisole (BHA), 2,6-di-tert-butyl-4-methylphenol (BHT), sodium metabisulfite, potassium metabisulfite, butylated hydroxyanisole, L-ascorbyl palmitate, sodium thiosulfate, and vitamin E. However, in preferred embodiments, the fusion protein composition of the present disclosure is manufactured under sterile conditions and packaged in single-use containers, thereby obviating the need for preservatives.

The ACE2-Fc fusion protein compositions described herein may include antibiotics. Antibiotics are substances or drugs that kill bacteria, microorganisms, or inhibit their activity. Suitable antibiotics and/or bacteriostatic agents may include one or more of the following: β-lactam antibiotics (including penicillin, cephalosporins, carbapenems, β-lactam/β-lactamase inhibitor combinations, and monobactams), aminoglycosides, tetracyclines, fluoroquinolones, folate pathway inhibitors, chloramphenicol, glycopeptides (including vancomycin and teicoplanin), and macrolides. The antibiotics may also include chemically synthesized drugs, such as one or more of sulfonamides, nitrofurans, and quinolones. The ACE2-Fc fusion protein composition described herein may not include bacteriostatic agents/antibiotics.

The ACE2-Fc fusion protein compositions described herein may include pharmaceutically acceptable flavorings or other ingredients for adjusting taste or smell.

In some embodiments, the composition may be provided as a sterile composition. The pharmaceutical composition typically comprises a therapeutically effective amount of the disclosed ACE2-Fc fusion protein and may be prepared using conventional techniques. Generally, the quantity of the ACE2-Fc fusion protein composition per dose is selected to effectively block viral entry by acting as a soluble receptor while avoiding significant adverse effects. In some embodiments, the composition may be provided in unit dosage form for blocking ACE2 receptor-binding viral invasion in the upper respiratory nasal cavity of a subject. The unit dosage form contains a single pre-selected, a single pre-selected dose suitable for administration to the subject, or a suitable labeled or measured multiple of two or more pre-selected unit doses, and/or a metering mechanism for administering the unit dose or a multiples thereof. In other embodiments, the composition further comprises one or more adjuvants.

### IV. Method for Blocking Viral Entry via Receptor Blocking

In some embodiments, the present disclosure provides a method for preventing coronavirus infection in a subject, including administering an effective amount of a composition to the subject, wherein the composition includes a recombinant fusion protein freely selected from the group consisting of SEQ ID NO: 1-18. In some embodiments, the present disclosure provides a method for preventing coronavirus infection in a subject, wherein the recombinant fusion protein comprises the ACE2 protein or a fragment thereof, and the method includes administering an effective amount of a composition to the subject, wherein the composition comprises a recombinant fusion protein selected from the group consisting of SEQ ID NO: 1-18.

The disclosed fusion protein (e.g., an ACE2-Fc fusion protein, e.g., a human ACE2-Fc fusion protein disclosed herein, a nucleic acid molecule (e.g., an RNA molecule) or a vector encoding a human ACE2-Fc fusion protein disclosed herein, or a protein nanoparticle or a virus-like particle comprising a human ACE2-Fc fusion protein disclosed herein) may be administered to a subject to block infection by a virus that infects a human via the ACE2 pathway (i.e., prevent viral infection). In a specific example, the subject is a human. Such blocking can inhibit the subsequent binding of the corresponding coronavirus to ACE2 in the subject and can be used to prevent, treat, or suppress an infection and a disease associated with the corresponding coronavirus.

The disclosed fusion protein or pharmaceutical composition may be administered via a route selected from intramuscular, intradermal, subcutaneous, intravenous, intraarterial, intraarticular, intraperitoneal, intranasal, sublingual, tonsillar, oropharyngeal, or other parenteral and mucosal routes, preferably via intranasal administration.

A subject may be selected for defense against the risk of coronavirus infection, for example, because of exposure or potential exposure to the coronavirus.

A typical subject for prevention using the therapy and method of the present invention includes a human, a non-human primate, and another animal. To identify a subject for prevention or treatment according to the method of the invention, an acceptable screening method is employed to determine a risk factor associated with a target or suspected disease or condition, or to determine the status of an existing disease or condition in the subject. This screening method includes, for example, a routine examination to determine an environmental, familial, occupational, and other such risk factor that may be associated with a target or suspected disease or condition, and a diagnostic method, for example, various ELISA and other immunoanalytical methods for detecting and/or characterizing a coronavirus infection. This and another conventional method allows a clinician to select a subject who requires prevention or treatment of a disease using the method and pharmaceutical composition of the present invention. In accordance with this method and principle, the composition may be administered according to the teaching of the present disclosure or another conventional method, as a standalone prophylactic or therapeutic regimen, or as a follow-up, adjunctive, or coordinated therapeutic regimen to another treatment.

The administration of the disclosed fusion protein (e.g., an ACE2-Fc fusion protein such as the human ACE2-Fc fusion protein described herein, the disclosed nucleic acid molecule (e.g., an RNA molecule) or vector encoding the human ACE2-Fc fusion protein, or a protein nanoparticle or virus-like particle comprising the disclosed human ACE2-Fc fusion protein) may be for a prophylactic or therapeutic purpose. When provided prophylactically, the disclosed therapeutic agent is administered prior to any symptom, for example, before an infection occurs. The prophylactic administration of the disclosed therapeutic agent is for preventing or ameliorating any subsequent infection. When provided therapeutically, the disclosed therapeutic agent is administered at or after the onset of a symptom of a disease or an infection, for example, after the development of a symptom of a coronavirus infection that corresponds to a coronavirus S antigen, or after a diagnosis of a coronavirus infection. Thus, the therapeutic agent may be provided before an anticipated exposure to a coronavirus, in order to attenuate the expected severity, duration, or extent of an infection and/or an associated disease symptom after exposure or suspected exposure to the virus, or after the actual onset of an infection.

The fusion protein and the fusion protein composition described in the present disclosure can effectively assist a subject (preferably a human) in preventing a coronavirus infection. The actual dosage of the disclosed fusion protein will vary according to a factor such as a disease sign and a specific status of the subject (e.g., the age, size, health status, degree of a symptom, susceptibility factor, etc. of the subject), the time and route of administration, another concurrently administered drug or therapy, and the specific pharmacology of the composition for eliciting a desired activity or biological response in the subject. A dosage regimen can be adjusted to provide an optimal prophylactic or therapeutic response.

The fusion protein and the fusion protein composition described herein may be used concomitantly or sequentially with one or more immunogenic compositions comprising the disclosed immunogen.

In some embodiments, an effective amount of the disclosed ACE2-Fc fusion protein composition is administered in a single dose or in a series of doses separated by one or more intervals. The interval may be measured in an hour, a day, or a week. In some embodiments, the composition comprising an effective amount of the disclosed ACE2-Fc fusion protein may be administered at an interval of 4 hours, 6 hours, 8 hours, 12 hours, 24 hours, 48 hours, or 72 hours. In some embodiments, it may be administered 1, 2, or 3 times a day for multiple consecutive days, for example, once a day or twice a day for multiple consecutive days, for example, 28 days, and for another example, twice a day for one week, two weeks, three weeks, four weeks, or more. In some embodiments, it may be administered once every two or three days for multiple consecutive days.

The suitability of a selected parameter of the fusion protein, for example, the formulation, dosage, regimen, etc., may be determined by taking an aliquot of serum from a subject and determining the antibody titer during the administration. In addition, the clinical status of the subject may be monitored to obtain an intended effect, for example, preventing an infection or ameliorating a disease state (e.g., reducing viral load). If such monitoring indicates that the ACE2-Fc fusion protein is suboptimal, the subject may be reinforced with an additional dose of the ACE2-Fc fusion protein composition, and the use parameter of the ACE2-Fc fusion protein composition may be improved in a manner that is expected to enhance infection blocking.

Typically, each single human dose will comprise 0.01 to 20 mg of the ACE2-Fc fusion protein, for example from about 0.05 mg to about 10 mg, for example from about 0.1 mg to about 8 mg, such as about 0.50 mg, about 1.00 mg, about 1.50 mg, about 2.00 mg, about 2.50 mg, about 3.00 mg, about 3.50 mg, about 4.00 mg, about 4.50 mg, about 5.00 mg, about 5.50 mg, about 6.00 mg, about 6.50 mg, about 7.00 mg, about 7.50 mg, or about 8.00 mg of the ACE2-Fc fusion protein.

For protein therapy, a typical single human dose may comprise about 0.01 to 3 mg of the ACE2-Fc fusion protein. In some embodiments, a single dose of the ACE2-Fc fusion protein composition may comprise about 0.02, 0.03, 0.04, 0.05, 0.06, 0.07, 0.08, 0.09, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.30, 0.31, 0.32, 0.33, 0.34, 0.35, 0.36, 0.37, 0.39, 0.40 to 3 mg of ACE2-Fc fusion protein. In some embodiments, single dose of the ACE2-Fc fusion protein composition may comprise about 0.05 to 0.06, 0.07, 0.08, 0.09, 0.1, 0.15, 0.2, 0.25, 0.3, 0.35, 0.4, 0.45, 0.5, 0.55, 0.6, 0.65, 0.7, 0.75, 0.8, 0.85, 0.9, 0.95, 1, 1.2, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, or 3.0 mg ACE2-Fc fusion protein. In some embodiments, a single dose of the ACE2-Fc fusion protein composition may comprise about 0.01, 0.02, 0.03, 0.04, 0.05, 0.06, 0.07, 0.08, 0.09, 0.1, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.50, 0.55, 0.6, 0.65, 0.70, 0.75, 0.80, 0.85, 0.90, 0.95, 1.00, 1.20, 1.25, 1.30, 1.40, 1.50, 1.60, 1.70, 1.80, 1.90, 2.00, 2.10, 2.20, 2.30, 2.40, 2.50, 2.60, 2.70, 2.80, 2.90, or 3.00 mg ACE2-Fc fusion protein. A single dose according to the present disclosure refers to the quantity administered at one time, which may comprise one spray or a plurality of sprays per nostril, such as two or three sprays.

The amount of the fusion protein composition to be used is selected based on the subject population (e.g., infants or elderly individuals). The optimal dosage for a specific component can be determined through standard studies that monitor antibody titers and other responses in a subject. It should be understood that the therapeutically effective amount of the disclosed fusion protein may encompass quantities that are ineffective in blocking viral infection when administered as a single dose but become effective when administered as a plurality of doses.

Following administration of the fusion protein disclosed herein, a subject exhibits a reduced viral load, e.g., pulmonary viral load (relative to a control group or a pre-administration level), and this response indicates the delivery of an effective dose of the fusion protein to the subject. In some embodiments, the antibody response in a subject will be determined under conditions evaluating an effective dose/immunization regimen. In most cases, it is sufficient to assess antibody titers in serum or plasma obtained from the subject. A decision as to whether to alter the amount of the therapeutic agent administered to an individual may be based at least in part on the antibody titer level. The antibody titer level may be determined based on, for example, an immunoassay that measures the concentration of antibodies in serum binding to an antigen (including, for instance, a recombinant coronavirus S antigen such as S-Trimer).

The method can be effective without complete elimination, reduction, or prevention of coronavirus infection. For example, compared to coronavirus infection in the absence of the fusion protein administration, the use of the disclosed fusion protein can reduce or inhibit the coronavirus infection by a desired extent, such as by at least 10%, at least 20%, at least 50%, at least 60%, at least 70%, at least 80%, at least 90%, at least 95%, at least 98%, or even by at least 100% (eliminating or preventing detectable infected cells).

The publicly disclosed fusion protein can also be administered via nucleic acid (Hoecke and Kenny Roose, J Transl Med (2019) 17:54, https://doi.org/10.1186/s12967-019-1804-8, is incorporated by reference in its entirety for all purposes). In some embodiments, the fusion protein disclosed herein is administered using a DNA method.

In some embodiments, administration of a therapeutically effective amount of one or more of the disclosed fusion protein to a subject can block viral infection in the subject. To evaluate the effect of blocking viral infection, serum may be collected from the subject at appropriate time points after administration, frozen, and stored for a neutralization assay. Methods for determining neutralizing activity are known to those of ordinary skill in the art and are further described herein, including but not limited to a plaque reduction neutralization test (PRNT), a microneutralization assay, a flow cytometry-based assay, and a single-cycle infection assay. In some embodiments, a panel of coronavirus pseudotyped virus may be used to measure serum neutralizing activity.

### V. Article of Manufacture or Kit

The present disclosure further provides an article of manufacture or kit comprising the disclosed recombinant polypeptide or protein composition. The article may comprise a container and a label or package insert affixed to or associated with the container. Suitable container includes, for example, a bottle, a vial, a syringe, a test tube, an intravenous solution bag, and the like. The container may be fabricated from various materials (for example, glass or plastic). In some embodiments, the container is provided with a sterile access port. An exemplary container includes an intravenous solution bag or a vial comprising a stopper penetrable by a hypodermic injection needle. The article or kit may further comprise a package insert indicating the utility of the article for the prevention or treatment of a specified disorder, including but not limited to those described herein (e.g., a coronavirus infection). Additionally or alternatively, the article or kit may further comprise another container (or the same container) containing a pharmaceutically acceptable buffer, or other excipient, or adjuvant. It may further include other materials, for example, other buffer, diluent, filter, needle, and/or syringe.

The label or package insert may indicate that the composition is intended for the prevention or treatment of coronavirus infection in a subject. The label or package insert affixed to or associated with the container may indicate instructions for the reconstitution and/or use of the preparation. Furthermore, the label or package insert may indicate that the preparation is for or intended to be administered via nasal spray delivery for the prevention of coronavirus infection in an individual.

In some embodiments, the container contains an ACE2-Fc fusion protein or composition thereof, which is either alone or in combination with another composition effective for treating, preventing, and/or diagnosing a disorder. The article or kit may comprise: (a) a first container comprising a composition contained therein (i.e., a first agent), wherein the composition includes an immunogenic ACE2-Fc fusion protein or recombinant polypeptide, or a composition thereof; and (b) a second container comprising a composition contained therein (i.e., a second agent), wherein the composition includes another agent, such as an adjuvant or other therapeutic agent. The article or kit may further include instructions on the label or package insert for treating a subject with the second agent in an effective amount.

### VI. Terminology

Unless otherwise defined, the meaning of all specialized terms, symbols, and other technical and scientific term or terminology used herein is intended to be the same as the meaning commonly understood by one of ordinary skill in the art to which the claimed subject matter pertains. In some instances, terms with ordinarily understood meanings may be explicitly defined in The present disclosure for clarity and/or ease of reference, and such definitions included herein do not necessarily imply substantive differences from what is commonly understood in the art. The terms "polypeptide" and "protein" are used interchangeably herein to refer to a polymer of an amino acid residue, without limitation to a minimum length. A polypeptide (including the provided receptor and other polypeptide, for example a linker or peptide) may comprise an amino acid residue, including a natural and/or non-natural amino acid residue. The term also includes post-translational modification of a polypeptide, for example glycosylation, sialylation, acetylation, and phosphorylation. In some aspects, a polypeptide may contain modifications relative to the native or natural sequence, provided that the protein retains its desired activity. Such modifications may be introduced intentionally (e.g., through site-directed mutagenesis) or occur adventitiously (e.g., through mutations in the protein-producing host or errors arising from PCR amplification).

As used herein, the term "subject" refers to a mammal, such as a human or other animal, and is typically a human. In some embodiments, the subject (e.g., a patient) to whom one or more therapeutic agents, cells, cell populations, or compositions are administered is a mammal, typically a primate, such as a human. In some embodiments, the primate may be a monkey or ape. The subject may be male or female and may fall within any appropriate age range, including infant, juvenile, adolescent, adult, and geriatric subjects. In some embodiments, the subject is a non-primate mammal, such as a rodent.

As used herein, the term "treatment" (including its grammatical variants such as "treat" or "treating") refers to the complete or partial amelioration or reduction of a disease, disorder, or condition, or symptoms, adverse effects, outcomes, or phenotypes associated therewith. The desired effects of treatment include, but are not limited to: prevention of disease occurrence or recurrence, alleviation of symptoms, reduction of any direct or indirect pathological consequences of the disease, prevention of metastasis, slowing the rate of disease progression, improvement or remission of the disease state, and mitigation or improvement of prognosis. This term does not imply complete cure of the disease or complete elimination of any symptom or effects on all symptoms or outcomes.

As used herein, "delaying progression of disease" refers to retarding, impeding, decelerating, slowing, stabilizing, suppressing, and/or postponing the advancement of a disease (e.g., cancer). The duration of delay may vary depending on the disease history and/or the individual receiving treatment. In some embodiments, a sufficient or significant delay may effectively constitute prevention, as the individual may not develop the disease. For example, the development of advanced cancer, for example metastasis, may be delayed.

As used herein, "prevention" includes providing prevention for the occurrence or recurrence of a disease in a subject who may be susceptible to the disease but have not yet been diagnosed with the disease. In some embodiments, the provided cells and compositions are used to delay disease progression or slow disease advancement.

As used herein, "inhibition" of a function or activity refers to a reduction in the function or activity when compared to other identical conditions except for the condition or parameter of interest, or when compared to another condition. For example, a cell that inhibits tumor growth would reduce the rate of tumor growth compared to the growth rate observed in the absence of the cell.

In the context of administration, an "effective amount" of an agent (e.g., a drug preparation, cells, or composition) is an effective amount for the dose/quantity and period of time required to achieve the intended effect (e.g., a therapeutic or preventive effect).

A "therapeutically effective amount" of an agent (e.g., a drug preparation or cells) is an effective amount for the dose and period of time required to achieve the intended therapeutic effect (e.g., for treating a disease, disorder, or condition) and/or the pharmacokinetic or pharmacodynamic effect of the treatment. The therapeutically effective amount may vary depending on factors including, for example, the subject's disease status, age, sex, and body weight, as well as the administered cell population. In some embodiments, the provided methods involve administration of cells and/or compositions at an effective amount (e.g., a therapeutically effective amount).

A "prophylactically effective amount" refers to an effective amount for the dose and period of time required to achieve the intended preventive effect. Typically, but not necessarily, the prophylactically effective amount will be less than the therapeutically effective amount since a preventive dose is administered to a subject prior to or in an early stage of a disease. In some aspects with low tumor burden, the prophylactically effective amount may exceed the therapeutically effective amount. For a vaccine or other agent, an effective amount is sufficient to elicit a desired response, such as reduction or elimination of a sign or symptom of a disorder or disease (e.g., pneumonia). For example, this may be an amount necessary to inhibit viral replication, or detectably alter an external symptom of viral infection. Generally, this amount will be sufficient to measurably inhibit replication or infectivity of a virus (e.g., SARS-CoV-2). When administered to a subject, such dosage typically achieves a target tissue concentration that has demonstrated viral replication inhibition in vitro. In some embodiments, an "effective amount" is an amount that treats (including prevention) one or more symptoms and/or underlying causes of any disorder or disease, such as for treating coronavirus infection. In some embodiments, the effective amount is a therapeutically effective amount. In other embodiments, the effective amount is an amount that prevents the development of one or more signs or symptoms of a specific disease or disorder, such as a sign or symptom associated with coronavirus infection.

As used herein, the terms "antigen" and "immunogen" are used interchangeably to refer to a substance capable of inducing an immune response in a subject, typically a protein. The term further refers to an immunologically active protein that, when administered to a subject (either directly or via administration of a nucleotide sequence or vector encoding the protein), elicit a humoral and/or cellular-type immune response against the protein. Unless otherwise specified, the term "vaccine immunogen" may be used interchangeably with "protein antigen" or "immunogenic polypeptide."

The term "conservatively modified variant" applies to both amino acid and nucleic acid sequences. With respect to a given nucleic acid sequence, conservatively modified variants refer to those nucleic acids that encode identical or substantially identical amino acid sequences, or where the nucleic acid does not encode an amino acid sequence, substantially identical sequences. Owing to the degeneracy of the genetic code, a multitude of functionally identical nucleic acids encode any given protein. For polypeptide sequences, "conservatively modified variant" refers to variants having conservative amino acid substitutions, wherein an amino acid residue is replaced by another amino acid residue having a side chain with a similar charge. The art has defined families of amino acid residues having a side chain with a similar charge. These families include amino acid residues having a basic side chain (e.g., lysine, arginine, histidine), an acidic side chain (e.g., aspartic acid, glutamic acid), an uncharged polar side chain (e.g., glycine, asparagine, glutamine, serine, threonine, tyrosine, cysteine), a nonpolar side chain (e.g., alanine, valine, leucine, isoleucine, proline, phenylalanine, methionine, tryptophan), a β-branched side chain (e.g., threonine, valine, isoleucine), and an aromatic side chain (e.g., tyrosine, phenylalanine, tryptophan, histidine).

An epitope refers to an antigenic determinant. These are specific chemical groups or peptide sequences on an antigen molecule that elicit a specific immune response. For example, an epitope is the region of an antigen to which a B cell and/or T cell responds. An epitope may be formed by either contiguous amino acids or by non-contiguous amino acids brought into proximity by the tertiary folding of a protein.

Unless otherwise specified, a fusion protein of the present disclosure is a recombinant protein containing amino acid sequences from at least two unrelated proteins, joined together via peptide bonds to form a single protein. Accordingly, it does not include a naturally occurring coronavirus surface antigen. The unrelated amino acid sequences may be directly linked to each other or may be connected using a linker sequence. As used herein, proteins are considered "unrelated" if their amino acid sequences are not naturally joined by peptide bonds in their native environment (e.g., within a cell). For example, the amino acid sequences of ACE2 and Fc are not naturally connected by peptide bonds.

An immunogen is a protein or portion thereof capable of inducing an immune response in a mammal, such as a mammal infected with a pathogen or at risk of pathogen infection. Administration of the immunogen may result in protective immunity and/or active immunity against the target pathogen.

An immunogenic composition refers to a composition comprising an immunogenic polypeptide that induces a measurable cytotoxic T lymphocyte (CTL) response against a virus expressing the immunogenic polypeptide, or induces a measurable B-cell response (e.g., antibody production) against the immunogenic polypeptide.

Sequence identity or similarity between two or more nucleic acid sequences or two or more amino acid sequences is expressed in terms of the identity or similarity between the sequences. Sequence identity can be measured in terms of percent identity, with a higher percentage indicating greater sequence identity. Two sequences are "substantially identical" if they have a specified percentage of identical amino acid residues or nucleotides when compared and aligned for maximum correspondence over a specified region as measured by a comparison window, or by using one of the following sequence comparison algorithms, or by manual alignment and visual inspection (i.e., 60% sequence identity, preferably 65%, 70%, 75%, 80%, 85%, 90%, 95%, or 99% sequence identity over the specified region, or over the entire sequence if no region is specified). Optionally, the identity exists over a region of at least about 50 nucleotides (or 10 amino acids), or more preferably over a region of 100 to 500 or 1000 or more nucleotides (or 20, 50, 200, or more amino acid residues).

A vaccine refers to a pharmaceutical composition capable of eliciting a prophylactic or therapeutic immune response in a subject. In certain cases, the immune response is a protective immune response. Typically, a vaccine induces an antigen-specific immune response against an antigen of a pathogen (e.g., a viral pathogen), or a cellular component associated with a pathological condition. A vaccine may comprise: a polynucleotide (e.g., a nucleic acid encoding the disclosed antigen), a peptide or polypeptide (e.g., the disclosed antigen), a virus, a cell, or one or more cellular components. In selected embodiments, the vaccine, vaccine immunogen, or vaccine composition is expressed from a fusion construct and spontaneously assembles into nanoparticles that display the immunogenic polypeptide or protein on their surfaces.

Virus-like particles (VLPs) refer to non-replicating viral shells derived from any of several viruses. VLPs typically consist of one or more viral proteins, including but not limited to proteins designated as capsid, coat, shell, surface, and/or envelope proteins, or particle-forming polypeptides derived from such proteins. Following recombinant expression of the proteins in an appropriate expression system, VLPs can spontaneously self-assemble. Methods for producing specific VLPs are well known in the art. The presence of VLPs after recombinant expression of viral proteins can be detected using conventional techniques known in the field, such as electron microscopy, biophysical characterization, etc. See, for example, Baker et al. (1991) Biophys. J. 60:1445-1456 and Hagensee et al. (1994) J. Virol. 68:4503-4505, which are incorporated herein by reference in their entirety for all purposes. For instance, VLPs may be isolated by density gradient centrifugation and/or identified by its characteristic density band. Alternatively, cryo-electron microscopy may be performed on vitrified aqueous samples of the VLP preparation in question, with images recorded under appropriate exposure conditions.

The term "about" or "approximately" as used in the present disclosure refers to a usual range of error for a given value that is readily known to a person skilled in the art. Any reference in the present disclosure to a value or parameter that is "about" or "approximately" a specified amount includes (and describes) an embodiment directed to that precise value or parameter itself. As used in the present disclosure, the singular forms "a," "an," and "the" include plural referents unless the context explicitly dictates otherwise. For example, the term "a" or "an" means "at least one" or "one or more."

In the present disclosure, various aspects of the claimed subject matter are presented in range format. It should be understood that the description in range format is for convenience and brevity only, and should not be construed as an inflexible limitation on the scope of the claimed subject matter. Accordingly, the description of a range should be considered to have specifically disclosed all possible subranges and individual numerical values within that range. For example, when a range of values is provided, it should be understood that every intervening value between the upper and lower limits of that range - as well as any other stated or intervening values in that range - is encompassed within the claimed subject matter. The upper and lower limits of these smaller ranges may independently be included in the smaller ranges and are also encompassed within the claimed subject matter, subject to any specifically excluded limits in the stated range. Where the range includes one or both of the stated limits, ranges excluding either or both of those included limits are also included in the claimed subject matter. This applies regardless of the breadth of the range.

As used herein, the term "composition" refers to any mixture of two or more products, substances, or compounds (including cells). It may be in the form of a solution, suspension, liquid, powder, paste, aqueous formulation, non-aqueous formulation, or any combination thereof.

The term "vector" as used in the present disclosure refers to a nucleic acid molecule capable of propagating another nucleic acid to which it is linked. This term encompasses vectors as selfreplicating nucleic acid structures, as well as vectors that are incorporated into the genome of a host cell into which they have been introduced. Certain vectors are capable of directing expression of nucleic acids operably linked to them. Such vectors are referred to herein as "expression vectors."

### VII. Exemplary Embodiments

Embodiment 1. A fusion protein comprising a plurality of recombinant polypeptides, wherein the fusion protein comprises a human ACE2 protein or a fragment thereof and a human IgG Fc protein or a functional variant thereof.

Embodiment 2. The fusion protein according to embodiment 1, wherein the human ACE2 protein or a fragment thereof comprises a human ACE2 extracellular domain or a fragment thereof.

Embodiment 3. The fusion protein according to embodiment 1 or 2, wherein the human ACE2 protein or a fragment thereof comprises an amino acid sequence selected from the group consisting of SEQ ID NO: 5, SEQ ID NO: 7, SEQ ID NO: 11, SEQ ID NO: 12, SEQ ID NO: 13, and SEQ ID NO: 14, or an amino acid sequence having at least about 75%, 80%, 85%, 90%, 92%, 95%, 96%, 97%, 98%, or 99% sequence identity thereto, or a fragment thereof.

Embodiment 4. The fusion protein according to any one of embodiments 1 to 3, wherein the human ACE2 protein or a fragment thereof comprises: an amino acid sequence of SEQ ID NO: 5, or an amino acid sequence having at least about 75%, 80%, 85%, 90%, 92%, 95%, 96%, 97%, 98%, or 99% sequence identity to SEQ ID NO: 5, or a fragment thereof.

Embodiment 5. The fusion protein according to any one of embodiments 1 to 4, wherein the human IgG Fc protein or a functional variant thereof comprises an Fc region sequence selected from the group consisting of human IgG1 Fc, IgG2 Fc, IgG3 Fc, and IgG4 Fc, a functional variant, or a fragment thereof.

Embodiment 6. The fusion protein according to any one of embodiments 1 to 5, wherein the human IgG Fc protein or a functional variant thereof is selected from the group consisting of a human IgG1 Fc region sequence, a functional variant thereof, and a fragment thereof.

Embodiment 7. The fusion protein according to any one of embodiments 1 to 6, wherein the human IgG Fc protein or a functional variant thereof comprises: an amino acid sequence of SEQ ID NO: 6 or SEQ ID NO: 8, or an amino acid sequence having at least about 75%, 80%, 85%, 90%, 92%, 95%, 96%, 97%, 98%, or 99% sequence identity to SEQ ID NO: 6 or SEQ ID NO: 8, or a fragment thereof.

Embodiment 8. The fusion protein according to any one of embodiments 1 to 7, wherein the human IgG Fc protein or a functional variant thereof comprises: an amino acid sequence of SEQ ID NO: 6, or an amino acid sequence having at least about 75%, 80%, 85%, 90%, 92%, 95%, 96%, 97%, 98%, or 99% sequence identity to SEQ ID NO: 6, or a fragment thereof.

Embodiment 9. The fusion protein according to any one of embodiments 1 to 8, optionally comprising a signal peptide.

Embodiment 10. The fusion protein according to any one of embodiments 1 to 9, optionally comprising a peptide linker, wherein the human ACE2 protein or a fragment thereof and the human IgG Fc protein or a functional variant thereof are directly linked or linked via the peptide linker.

Embodiment 11. The fusion protein according to embodiment 10, wherein the peptide linker is selected from the group consisting of: an arginine-serine linker (-RS-), a valine-serine linker (-VS-), and a glycine-serine linker (-GS-).

Embodiment 12. The fusion protein according to any one of embodiments 1 to 11, optionally comprising a mutated sequence.

Embodiment 13. The fusion protein according to embodiment 1, comprising: an amino acid sequence selected from the group consisting of SEQ ID NO: 1 through SEQ ID NO: 18, or an amino acid sequence having at least about 75%, 80%, 85%, 90%, 92%, 95%, 96%, 97%, 98%, or 99% sequence identity thereto, or a fragment thereof, or a combination thereof.

Embodiment 14. The fusion protein according to embodiment 1, comprising: an amino acid sequence selected from the group consisting of SEQ ID NO: 1, SEQ ID NO: 2, SEQ ID NO: 3, SEQ ID NO: 4, SEQ ID NO: 15, SEQ ID NO: 16, SEQ ID NO: 17, and SEQ ID NO: 18, or an amino acid sequence having at least about 75%, 80%, 85%, 90%, 92%, 95%, 96%, 97%, 98%, or 99% sequence identity thereto, or a fragment thereof; for example, comprising an amino acid sequence selected from the group consisting of SEQ ID NO: 1, SEQ ID NO: 2, SEQ ID NO: 3, SEQ ID NO: 4, SEQ ID NO: 15, SEQ ID NO: 16, SEQ ID NO: 17, and SEQ ID NO: 18, or an amino acid sequence having at least about 75%, 80%, 85%, 90%, 92%, 95%, 96%, 97%, 98%, or 99% sequence identity thereto, or a fragment thereof.

Embodiment 15. The fusion protein according to any one of embodiments 1 to 14, further comprising a detectable label.

Embodiment 16. A pharmaceutical composition comprising: the fusion protein according to any one of embodiments 1 to 15, and optionally, a pharmaceutically acceptable carrier.

Embodiment 17. The pharmaceutical composition according to embodiment 16, comprising the fusion protein according to any one of embodiments 1 to 15 at a concentration of about 0.1 mg/ml to about 100 mg/ml, for example about 0.50 mg/ml to about 20.00 mg/ml, such as about 1.25 mg/ml, about 2.50 mg/ml, or about 5.00 mg/ml.

Embodiment 18. The pharmaceutical composition according to embodiment 16 or 17, wherein the pharmaceutically acceptable carrier comprises one or more selected from the group consisting of a buffer and a tonicity agent.

Embodiment 19. The pharmaceutical composition according to embodiment 18, wherein the buffer comprises one or more selected from the group consisting of sodium dihydrogen phosphate monohydrate, disodium hydrogen phosphate dihydrate, or a combination thereof.

Embodiment 20. The pharmaceutical composition according to embodiment 18 or 19, wherein the tonicity agent comprises one or more selected from the group consisting of sodium chloride, potassium chloride, glycerol, glucose, sorbitol, sucrose, xylitol, and mannitol.

Embodiment 21. The pharmaceutical composition according to any one of embodiments 18 to 20, wherein the tonicity agent comprises sodium chloride and/or sucrose.

Embodiment 22. The pharmaceutical composition according to any one of embodiments 18 to 21, wherein the pharmaceutically acceptable carrier optionally comprises a stabilizer selected from the group consisting of: proteins, peptides, or hydrolysates, e.g., albumin, gelatin; sugars, e.g., sucrose, lactose, sorbitol; amino acids, e.g., sodium glutamate; or a combination thereof, for example, wherein the stabilizer comprises sucrose.

Embodiment 23. The pharmaceutical composition according to any one of embodiments 18 to 22, wherein the pharmaceutically acceptable carrier optionally comprises a bacteriostatic agent selected from the group consisting of benzoic acid, sorbic acid, thimerosal, and phenylethyl alcohol, or a combination thereof, for example, wherein the bacteriostatic agent comprises phenylethyl alcohol and/or thimerosal.

Embodiment 24. The pharmaceutical composition according to any one of embodiments 18 to 23, formulated for administration via a route selected from intramuscular, intradermal, subcutaneous, intravenous, intraarterial, intraarticular, intraperitoneal, intranasal, sublingual, tonsillar, oropharyngeal, or other parenteral and mucosal routes, preferably formulated for intranasal administration, for example as a nasal spray.

Embodiment 25. The fusion protein according to any one of embodiments 1 to 15, or the pharmaceutical composition according to any one of embodiments 16 to 24, for use in: preventing or treating infection by coronavirus SARS-CoV-2 and a variant thereof; and/or preventing transmission of SARS-CoV- 2 and a variant thereof from an infected subject.

Embodiment 26. The fusion protein according to any one of embodiments 1 to 15, or the pharmaceutical composition according to any one of embodiments 16 to 24, for use according to embodiment 25, wherein the SARS-CoV-2 and a variant thereof comprise, but are not limited to: SARS-CoV-2 Hu-1, Alpha, Beta, Gamma, Delta, Mu, Omicron, JN.1; and hitherto unknown variants of coronavirus SARS-CoV-2.

Embodiment 27. The fusion protein according to any one of embodiments 1 to 15, or the pharmaceutical composition according to any one of embodiments 16 to 24, for use according to embodiment 25 or 26, wherein the fusion protein or pharmaceutical composition is administered via a route selected from intramuscular, intradermal, subcutaneous, intravenous, intraarterial, intraarticular, intraperitoneal, intranasal, sublingual, tonsillar, oropharyngeal, or other parenteral and mucosal routes, preferably via intranasal administration.

Embodiment 28. The fusion protein according to any one of embodiments 1 to 15, or the pharmaceutical composition according to any one of embodiments 16 to 24, for use according to any one of embodiments 25 to 27, wherein the fusion protein or pharmaceutical composition is administered intranasally as: a single dose, or a plurality of doses separated by one or more intervals measured in hours, days, or weeks.

Embodiment 29. The fusion protein according to any one of embodiments 1 to 15, or the pharmaceutical composition according to any one of embodiments 16 to 24, for use according to embodiment 28, wherein the interval is selected from the group consisting of 4 hours, 6 hours, 8 hours, 12 hours, 24 hours, 48 hours, or 72 hours.

Embodiment 30. The fusion protein according to any one of embodiments 1 to 15, or the pharmaceutical composition according to any one of embodiments 16 to 24, for use according to any one of embodiments 25 to 29, wherein the fusion protein or pharmaceutical composition is administered at a frequency of once or multiple times per day, for example once per day or twice per day.

Embodiment 31. Use of the fusion protein according to any one of embodiments 1 to 15, or the pharmaceutical composition according to any one of embodiments 16 to 24, in the manufacture of a medicament for: preventing or treating infection by coronavirus SARS-CoV-2 and a variant thereof; and/or preventing transmission of SARS-CoV-2 and a variant thereof from an infected subject.

Embodiment 32. The use according to embodiment 31, wherein the SARS-CoV-2 and a variant thereof comprise, but are not limited to: SARS-CoV-2 Hu-1, Alpha, Beta, Gamma, Delta, Mu, Omicron, JN.1, and hitherto unknown variants of coronavirus SARS-CoV-2.

Embodiment 33. A method for preventing or treating infection by coronavirus SARS-CoV-2 and a variant thereof, and/or preventing transmission of SARS-CoV-2 and a variant thereof from an infected subject, comprising administering to a subject a therapeutically effective amount of: the fusion protein according to any one of embodiments 1 to 15, or the pharmaceutical composition according to any one of embodiments 16 to 24.

Embodiment 34. The method according to embodiment 33, wherein the SARS-CoV-2 and a variant thereof comprise, but are not limited to: SARS-CoV-2 Hu-1, Alpha, Beta, Gamma, Delta, Mu, Omicron, JN.1, and hitherto unknown variants of coronavirus SARS-CoV-2.

Embodiment 35. The method according to embodiment 33 or 34, wherein the fusion protein or pharmaceutical composition is administered via a route selected from intramuscular, intradermal, subcutaneous, intravenous, intraarterial, intraarticular, intraperitoneal, intranasal, sublingual, tonsillar, oropharyngeal, or other parenteral and mucosal routes, preferably via intranasal administration.

Embodiment 36. The method according to any one of embodiments 33 to 35, wherein the fusion protein according to any one of embodiments 1 to 15 or the pharmaceutical composition according to any one of embodiments 16 to 24 is administered intranasally as: a single dose, or a plurality of doses separated by one or more intervals measured in hours, days, or weeks.

Embodiment 37. The method according to embodiment 36, wherein the interval is selected from 4 hours, 6 hours, 8 hours, 12 hours, 24 hours, 48 hours, or 72 hours.

Embodiment 38. The method according to any one of embodiments 33 to 37, wherein the fusion protein or pharmaceutical composition is administered at a frequency of once or multiple times per day, for example once per day or twice per day.

Embodiment 39. A kit for preventing or treating infection by coronavirus SARS-CoV-2 and a variant thereof, and/or preventing transmission of SARS-CoV- 2 and a variant thereof from an infected subject, comprising: the fusion protein according to any one of embodiments 1 to 15, or the pharmaceutical composition according to any one of embodiments 16 to 24;
a container; and
optionally, a package insert or label with instructions for prevention and/or treatment.

Embodiment 40. The kit according to embodiment 39, wherein the SARS-CoV-2 and a variant thereof comprise, but are not limited to: SARS-CoV-2 Hu-1, Alpha, Beta, Gamma, Delta, Mu, Omicron, JN.1, and hitherto unknown variants of coronavirus SARS-CoV-2.

Embodiment 41. A nucleic acid encoding the fusion protein according to any one of embodiments 1 to 15, or a fragment of the fusion protein.

Embodiment 42. A vector comprising the nucleic acid according to embodiment 41.

Embodiment 43. The vector according to embodiment 42, which is phFC(IM).

Embodiment 44. A host cell comprising: the nucleic acid according to embodiment 41, or the vector according to embodiment 42.

Embodiment 45. The host cell according to embodiment 44, which is a CHO cell, for example GH-CHO.

### Examples

The following examples are included for illustrative purposes only and are not intended to limit the scope of the present invention.

### METHOD

### Example 1: Production and Purification of ACE2-Fc Fusion Protein and Preparation as a Nasal Spray

The ACE2-Fc fusion protein with the amino acid sequence shown in SEQ ID NO: 4 (SCB-719) was produced and purified in this example and used for the biological tests conducted in the following examples. The SEQ ID NO: 4 synthesized in the example contains a C-terminal modification that facilitates tracking and detection without affecting the functional effect of the ACE2-Fc fusion protein. A person skilled in the art can fully understand that ACE2-Fc fusion proteins with or without this C-terminal modification, for example, SEQ ID NO: 1-3 and 15-18, can achieve the same or comparable biological functional effects. For rapid expression of the ACE2-Fc fusion protein (Liu et al., Scientific Reports 7(1): 8953, 2017, incorporated herein by reference in its entirety for all purposes), the cDNA encoding the ACE2-Fc fusion protein was subcloned into the expression vector phFC(IM), and was propagated in the Chinese Hamster Ovary (CHO) cell line GH-CHO.

Taking the 200L cell culture process as an example, the 200L cell culture process comprised a seed expansion stage and a fed-batch culture stage. Seed expansion stage: cells from one vial of a working cell bank (WCB) were thawed into a 250 ml shaker flask using CD118 medium, cultured for 3-5 days, and passaged and expanded, after being passaged and expanded in N-4 and N-3 stage shaker flasks, N-2 and N-1 stage amplification was performed in a WAVE reactor; Fed-batch culture stage: When the N-1 stage culture reached day 3, it was inoculated into a 200L reactor. From day 3 to day 15, CB7a and CB7b were supplemented daily at 2% and 0.2% of the initial culture volume, respectively. On day 4, the culture temperature was reduced from 37°C to 32°C. The glucose concentration was maintained between 2-10 g/L throughout the cultivation process. The culture was harvested when it reached day 14-16 or when the cell viability dropped below 90%. Harvesting was performed using a two-stage depth filtration system with Merck's D0SP and A1HC membrane filters, followed by sterile filtration, the clarified cell harvest was then transferred to downstream processing for purification.

To obtain the fusion protein in a highly purified form for pharmaceutical research. The purification process began by capturing the target sample from the cell culture harvest using Cytiva's affinity chromatography resin Mabselect PrismA. This was followed by a flow-through mode cation exchange chromatography step using Suzhou Saifen Technology company's cation exchange resin Monomix HC 60SP, with the primary purpose of removing target product aggregates. The collected cation exchange eluate was supplemented with 1% Tween 80 and 0.3% TNBP (final concentration) for solvent/detergent (S/D) viral inactivation. Subsequently, a bindand-elute mode anion exchange chromatography was performed using Merck's Eshmuno Q resin to further enhance sample purity by removing impurities such as host cell proteins (HCP) and DNA. This was followed by viral clearance using Asahi Kasei Corporation's Planova 20N nanofilter for virus removal. Finally, the sample was concentrated and diafiltered using a 50 kDa PES ultrafiltration membrane cassette from Hangzhou Cobetter Filtration Equipment Co., Ltd. The formulation buffer was exchanged into a solution containing 20 mM PB, 50 mM NaCl, and 5% sucrose. The final sample, with a concentration ≥10 g/L, was 0.2 µm sterile-filtered to obtain the drug substance. The ACE2-Fc fusion protein purification platform yielded the target protein with a purity exceeding 98%. The corresponding analytical chromatogram is shown in FIG. 4C. Stability analysis of the purified ACE2-Fc fusion protein indicated that the ACE2-Fc fusion protein remained stable at 25°C.

The drug substance of the ACE2-Fc fusion protein was processed by thawing, intermediate formulation, sterile filtration, aseptic filling and crimping, labeling and packaging to produce the ACE2-Fc fusion protein nasal spray.

The formulation development study for the ACE2-Fc fusion protein nasal spray was conducted in four stages: a pH screening test, a single-factor additive test, a Design of Experiments (DoE) test, and a formulation confirmation test. The formulation was determined primarily based on the trend of purity changes (as measured by SEC-HPLC) under accelerated conditions (25 ± 2°C) and high-temperature conditions (40 ± 2°C), with osmotic pressure of the solution as a secondary indicator. The formulation is shown in Table 2a-2e.

**Table 2a. Nasal Spray Formulation**

| **Ingredients** | **Content/ Concentration** | **Function** |
|---|---|---|
| ACE2-Fc fusion protein | 2.50 mg/ml | Active Ingredient |
| Sodium dihydrogen phosphate monohydrate | 2.13 mg/ml | Buffer agent |
| Disodium Hydrogen Phosphate Dihydrate | 0.82 mg/ml | Buffer agent |
| Sodium chloride | 2.92 mg/ml | Tonicity agent |
| Sucrose | 50.00 mg/ml | Stabilizer, Tonicity agent |

**Table 2b. Nasal Spray Formulation**

| **Ingredients** | **Content/ Concentration** | **Function** |
|---|---|---|
| ACE2-Fc fusion protein | 2.5 mg/ml | Active Ingredient |
| Sodium dihydrogen phosphate monohydrate | 2.13 mg/ml | Buffer agent |
| Disodium Hydrogen Phosphate Dihydrate | 0.82 mg/ml | Buffer agent |
| Sodium chloride | 2.92 mg/ml | Tonicity agent |
| Sucrose | 50 mg/ml | Stabilizer, Tonicity agent |
| phenethyl alcohol | 2.5 mg/ml | Bacteriostatic agent |

**Table 2c. Nasal Spray Formulation**

| **Ingredients** | **Content/ Concentration** | **Function** |
|---|---|---|
| ACE2-Fc fusion protein | 2.0 mg/ml | Active Ingredient |
| Sodium dihydrogen phosphate monohydrate | 2.13 mg/ml | Buffer agent |
| Disodium Hydrogen Phosphate Dihydrate | 0.82 mg/ml | Buffer agent |
| Sodium chloride | 2.92 mg/ml | Tonicity agent |
| Sucrose | 50 mg/ml | Stabilizer, Tonicity agent |
| Thiomersal | 0.10 mg/ml | Bacteriostatic agent |

**Table 2d. Nasal Spray Formulation**

| **Ingredients** | **Content/ Concentration** | **Function** |
|---|---|---|
| ACE2-Fc fusion protein | 2.5 mg/ml | Active Ingredient |
| Sodium dihydrogen phosphate monohydrate | 2.13 mg/ml | Buffer agent |
| Disodium Hydrogen Phosphate Dihydrate | 0.82 mg/ml | Buffer agent |
| Sodium chloride | 2.92 mg/ml | Tonicity agent |
| Sucrose | 50 mg/ml | Stabilizer, Tonicity agent |
| Thiomersal | 0.13 mg/ml | Bacteriostatic agent |

**Table 2e. Nasal Spray Formulation**

| **Ingredients** | **Content/ Concentration** | **Function** |
|---|---|---|
| ACE2-Fc fusion protein | 2.5 mg/ml | Active Ingredient |
| Sodium dihydrogen phosphate monohydrate | 2.13 mg/ml | Buffer agent |
| Disodium Hydrogen Phosphate Dihydrate | 0.82 mg/ml | Buffer agent |
| Sodium chloride | 2.92 mg/ml | Tonicity agent |
| Sucrose | 50 mg/ml | Stabilizer, Tonicity agent |
| Thiomersal | 0.15 mg/ml | Bacteriostatic agent |

| | | |
|---|---|---|
| Note: The formulation contains an appropriate quantity of water. | | |

The nasal spray formulation disclosed herein demonstrated that no significant trend in purity change was observed after storage for 6 months under long-term conditions (2-8°C) and for 4 months under accelerated conditions (25 ± 2°C), when compared to the initial time point (T0), and the results met the acceptance criteria. Following 1 month of storage under high-temperature conditions (40 ± 2°C), a clear decreasing trend in purity was observed, although it still remained within the acceptance criteria.

The nasal spray disclosed herein was a multi-dose sterile nasal spray (e.g., 100 µl per nostril, 2.8ml per bottle). It was manufactured using sterile (sterilized) packaging materials and an aseptic filling process, which ensured the sterility of the released product. The microbial barrier pump utilized featured unique sealing technology at the spray orifice, coupled with a dedicated filter membrane design in the air pathway. These features collectively ensured the sterility of the product throughout a plurality of uses.

### Example 2: The construction and production of pseudoviruses.

The spike (S) gene of SARS-CoV-2 Variants of Concern was codon-optimized for mammalian expression and synthesized by Genscript, followed by cloning into the pcDNA3.1(+) eukaryotic expression vector. Plasmids encoding the S glycoprotein of various SARS-CoV-2 variants, including Hu-1, Alpha (α), Beta (β), Gamma (γ), Delta (δ), Mu (µ), Omicron (o), and JN.1, were constructed. The lentiviral packaging plasmid psPAX2 and the lentiviral reporter plasmid pLVX-AcGFP-N1-Fluc, which expresses GFP and luciferase, were obtained from HonorGene (China). Pseudoviruses were generated by co-transfecting HEK 293T cells with psPAX2, pLVX-AcGFP-N1-Fluc, and plasmids encoding various S genes using Lipofectamine 3000 (Invitrogen, L3000-015). Supernatants were harvested at 24 ± 2 hours post-transfection, centrifuged at 1500 rpm for 5 minutes to remove cellular debris, and subsequently stored at - 80°C. Pseudovirus stocks were titrated by infecting 293T-ACE2 cells. Following a 44 to 48-hour incubation period at 37°C with 5% CO₂, luciferase activity was measured using a microplate reader (TECAN, Spark) after adding the Bright-Glo Luciferase Assay System (Promega, E2650). The TCID₅₀ of the pseudoviruses was then calculated according to the Reed-Muench method (Quantification ofSARS-CoV-2neutralizing antibody by a pseudotyped virus based assay. Nie J. et al. DOI: 10.21203/rs.3.pex-941/v11).

### Example 3: Neutralization test

Aliquots of test serum samples were first heat-inactivated at 56°C for 30 minutes and then clarified by centrifugation at 10,000 rcf for 5 minutes. The samples were serially diluted (3-fold) in assay medium (100 µL), and incubated with 650 TCID₅₀ of pseudovirus (50 µL) at 37°C for 1 hour. Virus-only controls (virus alone) and cell-only controls (background) were included. Subsequently, fresh trypsinized 293T-ACE2 cells were added to each well at a density of 20,000 cells per well in 100 µL. After incubation for 44 to 48 hours at 37°C in a 5% CO₂ incubator, cells were lysed, and luciferase activity was measured using the Bright-Glo Luciferase Assay System (Promega) according to the manufacturer's protocol. The IC₅₀ neutralizing antibody titer for a given serum sample was defined as the serum dilution that resulted in a 50% reduction in relative light units (RLU) compared to the virus-infected control wells. The detailed method was as reported by Quantification ofSARS-CoV-2neutralizing antibody by a pseudotyped virus based assay. Nie J. et al. DOI: 10.21203/rs.3.pex-941/v11.

### Results

The test results indicated that the ACE2-Fc fusion protein disclosed herein exhibited neutralizing activity against all currently known COVID-19 mutant strains, with higher neutralizing activity against the mutant strains compared to the wild-type original virus.

### Example 4: Neutralizing activity test of ACE2-Fc fusion protein with currently available SARS-CoV-2 mutant strains and the original strain pseudovirus

The results of FIGs. 5A-5C showed that ACE2-Fc fusion protein had neutralizing activity against all currently known COVID-19 mutant strains, and the neutralizing activity against the mutant strains was higher than that against the original wild-type strain. The experimental results confirmed that using the ACE2-Fc fusion protein as a biological protective barrier against SARS-CoV-2 infection can effectively counter any COVID-19 mutant strain. Regardless of the mutation sites in the viral strains, the fundamental mechanism by which the virus invades human cells remains unchanged. The ACE2-Fc fusion protein exhibited high neutralizing activity against all SARS-CoV-2 mutant strains, including both known and unknown mutants. This also suggested that the ACE2-Fc fusion protein disclosed herein may be used for preventing SARS-CoV-2 infection and transmission, as well as for therapeutic purposes.

### Example 5: A challenge study (pharmacodynamics study) was conducted in transgenic mice employing an intranasal administration route

As shown in FIGs. 6A-6B, Delta challenge assays were performed in transgenic mice carrying human ACE2. Forty mice were divided into 4 groups, 10 mice in each group. Body weight and lung viral load were measured, and lung pathology was analyzed and scored on the 3rd day after challenge (5 mice per group). Group 1 (Control group): Mice were intranasally administered with physiological saline/excipient (50 µL per nostril). Group 2: Mice received intranasal administration of ACE2-Fc fusion protein (SCB-719) at a dose of 50 µg/50 µL per nostril. Group 3: Mice received intranasal administration of ACE2-Fc fusion protein (SCB-719) at a dose of 500 µg/50 µL per nostril. Group 4: Mice received intraperitoneal administration of 0.2 ml ACE2-Fc fusion protein (SCB-719) at a dose of 1000 µg/mouse.

The test results demonstrated that the intranasal administration group exhibited a lower pulmonary viral load compared to the intraperitoneal administration group. Both ACE2-Fc fusion protein-treated groups showed significantly reduced lung viral loads relative to the untreated group, wherein the live virus load in the 50 µg dose group was below the detection limit. For detailed results, refer to FIGs. 7A-7B. These findings confirmed that intranasal delivery effectively blocked SARS-CoV-2 invasion and outperformed systemic administration (intraperitoneal injection, ip), supporting the practical feasibility of the ACE2-Fc fusion protein.

### Example 6: Evaluation of distribution in mice following intranasal administration

Thirty-two all-female experimental animals were divided into vehicle group B2, test article group B1, and test article group S4 according to body weight. The protein (SCB-719) administered to the animals in groups B2 (n = 2), B1 (n = 3), and S4 (n = 27) was all labeled with AF750 fluorescein, and the protein content was 6 mg/ml.

Specific dosing information is presented in Table 3.

**Table 3. Administration Information Table**

| **Grou p** | **Animal No.** | | | **Route of administr ation** | **Num ber of Doses** | **Dose Volu me (µL)** | **Dosin g time** | **Tim e after dosi ng** | **Testi ng time** | **Num ber of anim als** |
|---|---|---|---|---|---|---|---|---|---|---|
| Vehi cle Grou p B2 | | 1F0101 | | | 2 | 25/do se | 18:30 | 0 | 18:3 0 | 2 |
| | | 1F0102 | | | | | | | | |
| Test Artic le Grou p B1 | | 2F0101 | | | 2 | 25/do se | 19:30 | 0 19:3 0 | | 3 |
| | | 2F0102 | | | | | | | | |
| | | 2F0103 | | | | | | | | |
| | 3F01 01 | 3F01 02 | 3F01 03 | | 2 | 25/do se | 11:30 (previ ous day) | 24 h | 11:3 0 | 3 |
| Test Artic le Grou p S4 | 3F02 01 | 3F02 02 | 3F02 03 | Nasal drops, one per nostril | 2 | 25/do se | 16:40 (previ ous day) | 16 h | 8:40 | 3 |
| | 3F03 01 | 3F03 02 | 3F03 03 | | 2 | 25/do se | 9:10 | 12 h | 21:1 0 | 3 |
| | 3F04 01 | 3F04 02 | 3F04 03 | | 2 | 25/do se | 9:25 | 8h | 17:2 5 | 3 |
| | 3F05 01 | 3F05 02 | 3F05 03 | | 2 | 25/do se | 9:40 | 6h | 15:4 0 | 3 |
| | 3F06 01 | 3F06 02 | 3F06 03 | | 2 | 25/do se | 9:55 | 4h | 13:5 5 | 3 |
| | 3F07 01 | 3F07 02 | 3F07 03 | | 2 | 25/do se | 10:10 | 2 h | 12:1 0 | 3 |
| | 3F08 01 | 3F08 02 | 3F08 03 | | 2 | 25/do se | 10:25 | 1 h | 11:2 5 | 3 |
| | 3F09 01 | 3F09 02 | 3F09 03 | | 2 | 25/do se | 10:40 | 0 h | 10:4 0 | 3 |

| | | | | | | | | | | |
|---|---|---|---|---|---|---|---|---|---|---|
| Note: The first digit of the animal number represented the group (1, 2 and 3 represented vehicle group B2, test article group B1 and test article group S4, respectively), the second letter represented the sex (F was female), the third and fourth digits represented the cage number within the group, and the fifth and sixth digits represented the animal number within the cage. | | | | | | | | | | |

All animals in each group were tested at 5 minutes post-dose, 1 ± 0.1, 2 ± 0.1, 4 ± 0.1, 6 ± 0.1, 8 ± 0.1, 12 ± 0.1, 16 ± 0.1 and 24 ± 0.1 h post-dose, as shown in Table 3.

Testing Methods and Parameters: After anesthesia, the in vivo distribution and decay of the test substance at various time points (pre- and post-administration) were evaluated in each group using an in vivo imaging system. The photon flux was quantified in units of photons per second per square centimeter per steradian (p/s/cm²/sr), Upon completion of each time point assessment, gross dissection was immediately performed to measure ex vivo fluorescence signal intensity in major organs: heart, liver, spleen, lungs, kidneys, brain, nasal cavity, and blood (2 µL). The AF750 fluorescent dye had a maximum excitation wavelength of 753 nm and a maximum emission wavelength of 782 nm. Each mouse was tested for a total of three times.

Following a single intranasal administration, no fluorescent signal was observed for unlabeled ACE2-Fc fusion protein (Test Group B1) or Vehicle B2. In contrast, AF750-labeled ACE2-Fc fusion protein (Test Group S4) was enriched in the nasal region, and clear fluorescence signals were observed within 0-4 hours post-administration. As shown in FIG. 8A, fluorescence signals were detected in the nasal cavity at various time points, while no signals were observed in other organs, confirming the localized enrichment of ACE2-Fc fusion protein in the nasal cavity. Post-administration, clear fluorescence signals were detected from 0 to 6 hours; from 6 to 8 hours, the signal was significantly weaker but still detectable; and a signal was still detectable at 12 hours. FIG. 8A presents the imaging results across different organs, while FIG. 8B illustrates the trend in nasal cavity fluorescence signal intensity over time.

In summary, the ACE2-Fc fusion protein showed sustained enrichment in the nasal cavity of mice for over 12 hours. This result proved that the ACE2-Fc fusion protein could remain at the administration site for a relatively long time, proving that the tissue half-life of the ACE2-Fc fusion protein could support the use of nasal administration for the prevention of SARS-CoV-2.

FIG. 9 exemplarily shows the ACE2-Fc fusion protein (IV/IP) PK and *in vitro* potency profiles. ACE2-Fc fusion protein was administered to 6-8-week-old female BALB/c mice via intravenous or intraperitoneal injection at a dose of 50 mg/kg, with an administration volume of 0.2 ml per mouse. Each group consisted of 3 mice. After a single dose administration, approximately 50 µL of whole blood was collected at specific time points (5 min, 15 min, 0.5 h, 1 h, 2 h, 4 h, 8 h, 24 h, 48 h, 72 h, 120 h). The blood samples were allowed to stand at room temperature for 1 hour, and then serum was separated for blood drug concentration detection. The serum drug concentration was measured using enzyme-linked immunosorbent assay (ELISA). Mouse anti-human Fc protein was immobilized in 96-well microplates to capture the ACE2-Fc fusion protein in the test serum. Subsequently, biotin-labeled ACE2-Fc fusion protein (SCB-2019-biotin) was added to bind with the captured ACE2-Fc. Finally, HRP-conjugated streptavidin was introduced to bind with biotin, and a color reaction occurred through HRP catalysis of the TMB substrate. The reaction was terminated with 1M sulfuric acid, and absorbance was measured at 450 nm using a microplate reader. A standard curve was generated by plotting the theoretical concentration of ACE2-Fc fusion protein (x-axis) against the measured OD values (y-axis). The content of ACE2-Fc fusion protein in the test samples was calculated by fitting the relevant parameters of the standard curve using a four-parameter logistic regression model. The assay's quantitation range was 3-100 ng/ml. The figure illustrated the blood drug concentration over time, with each time point representing the mean concentration (Mean) and standard error (SEM) from three mice.

The results showed that at a dose of 50 mg/kg, the half-life was 38.0 hours with an AUC of 10454 h*µg/ml following intravenous (IV) administration, whereas intraperitoneal (IP) administration yielded a half-life of 24.6 hours and an AUC of 10127 h*µg/ml. The drug exposure (AUC) and elimination half-life were comparable between the two administration routes. This also indicated that the ACE2-Fc fusion protein disclosed herein could be used for preventing SARS-CoV-2 infection and transmission, as well as for therapeutic purposes.

The present invention is not intended to limit the scope to the specific disclosed embodiments, which are provided, for example, to illustrate various aspects of the invention. Through the description and teachings of this disclosure, various modifications to the compositions and methods will become apparent. Such modifications may be implemented without departing from the true scope and spirit of the invention, and are intended to fall within the scope of the invention.

| **SEQ ID NO.** | **Sequence** | **Description** |
|---|---|---|
| **1** | MSSSSWLLLSLVAVTAAQSTIEEQAKTFLDKFNHEAEDLFYQSSLAS WNYNTNITEENVQNMNNAGDKWSAFLKEQSTLAQMYPLQEIQNLTVK LQLQALQQNGSSVLSEDKSKRLNTILNTMSTIYSTGKVCNPDNPQEC LLLEPGLNEIMANSLDYNERLWAWESWRSEVGKQLRPLYEEYVVLKN EMARANHYEDYGDYWRGDYEVNGVDGYDYSRGQLIEDVEHTFEEIKP LYEHLHAYVRAKLMNAYPSYISPIGCLPAHLLGDMWGRFWTNLYSLT VPFGQKPNIDVTDAMVDQAWDAQRIFKEAEKFFVSVGLPNMTQGFWE NSMLTDPGNVQKAVCHPTAWDLGKGDFRILMCTKVTMDDFLTAHHEM GHIQYDMAYAAQPFLLRNGANEGFHEAVGEIMSLSAATPKHLKSIGL LSPDFQEDNETEINFLLKQALTIVGTLPFTYMLEKWRWMVFKGEIPK DQWMKKWWEMKREIVGVVEPVPHDETYCDPASLFHVSNDYSFIRYYT RTLYQFQFQEALCQAAKHEGPLHKCDISNSTEAGQKLFNMLRLGKSE PWTLALENVVGAKNMNVRPLLNYFEPLFTWLKDQNKNSFVGWSTDWS PYADQSIKVRISLKSALGDKAYEWNDNEMYLFRSSVAYAMRQYFLKV KNQMILFGEEDVRVANLKPRISFNFFVTAPKNVSDIIPRTEVEKAIR MSRSRINDAFRLNDNSLEFLGIQPTLGPPNQPPRSEPKSCDKTHTCP PCPAPELLGGPSVFLFPPKPKDTLMISRTPEVTCVVVDVSHEDPEVK FNWYVDGVEVHNAKTKPREEQYNSTYRVVSVLTVLHQDWLNGKEYKC KVSNKALPAPIEKTISKAKGQPREPQVYTLPPSRDELTKNQVSLTCL VKGFYPSDIAVEWESNGQPENNYKTTPPVLDSDGSFFLYSKLTVDKS RWQQGNVFSCSVMHEALHNHYTQKSLSLSPGKDSNLWN | Full Sequence of Fusion Peptide with Signal Peptide |
| **2** | QSTIEEQAKTFLDKFNHEAEDLFYQSSLASWNYNTNITEENVQNMNN AGDKWSAFLKEQSTLAQMYPLQEIQNLTVKLQLQALQQNGSSVLSED KSKRLNTILNTMSTIYSTGKVCNPDNPQECLLLEPGLNEIMANSLDY NERLWAWESWRSEVGKQLRPLYEEYVVLKNEMARANHYEDYGDYWRG DYEVNGVDGYDYSRGQLIEDVEHTFEEIKPLYEHLHAYVRAKLMNAY PSYISPIGCLPAHLLGDMWGRFWTNLYSLTVPFGQKPNIDVTDAMVD QAWDAQRIFKEAEKFFVSVGLPNMTQGFWENSMLTDPGNVQKAVCHP TAWDLGKGDFRILMCTKVTMDDFLTAHHEMGHIQYDMAYAAQPFLLR NGANEGFHEAVGEIMSLSAATPKHLKSIGLLSPDFQEDNETEINFLL KQALTIVGTLPFTYMLEKWRWMVFKGEIPKDQWMKKWWEMKREIVGV VEPVPHDETYCDPASLFHVSNDYSFIRYYTRTLYQFQFQEALCQAAK HEGPLHKCDISNSTEAGQKLFNMLRLGKSEPWTLALENVVGAKNMNV RPLLNYFEPLFTWLKDQNKNSFVGWSTDWSPYADQSIKVRISLKSAL GDKAYEWNDNEMYLFRSSVAYAMRQYFLKVKNQMILFGEEDVRVANL KPRISFNFFVTAPKNVSDIIPRTEVEKAIRMSRSRINDAFRLNDNSL EFLGIQPTLGPPNQPPRSEPKSCDKTHTCPPCPAPELLGGPSVFLFP PKPKDTLMISRTPEVTCVVVDVSHEDPEVKFNWYVDGVEVHNAKTKP REEQYNSTYRVVSVLTVLHQDWLNGKEYKCKVSNKALPAPIEKTISK AKGQPREPQVYTLPPSRDELTKNQVSLTCLVKGFYPSDIAVEWESNG QPENNYKTTPPVLDSDGSFFLYSKLTVDKSRWQQGNVFSCSVMHEAL HNHYTQKSLSLSPGK | Full Sequence of Fusion Peptide without Signal Peptide, and Unmutated |
| **3** | MSSSSWLLLSLVAVTAAQSTIEEQAKTFLDKFNHEAEDLFYQSSLAS WNYNTNITEENVQNMNNAGDKWSAFLKEQSTLAQMYPLQEIQNLTVK LQLQALQQNGSSVLSEDKSKRLNTILNTMSTIYSTGKVCNPDNPQEC LLLEPGLNEIMANSLDYNERLWAWESWRSEVGKQLRPLYEEYVVLKN EMARANHYEDYGDYWRGDYEVNGVDGYDYSRGQLIEDVEHTFEEIKP LYEHLHAYVRAKLMNAYPSYISPIGCLPAHLLGDMWGRFWTNLYSLT VPFGQKPNIDVTDAMVDQAWDAQRIFKEAEKFFVSVGLPNMTQGFWE NSMLTDPGNVQKAVCHPTAWDLGKGDFRILMCTKVTMDDFLTAHHEM GHIQYDMAYAAQPFLLRNGANEGFHEAVGEIMSLSAATPKHLKSIGL LSPDFQEDNETEINFLLKQALTIVGTLPFTYMLEKWRWMVFKGEIPK DQWMKKWWEMKREIVGVVEPVPHDETYCDPASLFHVSNDYSFIRYYT RTLYQFQFQEALCQAAKHEGPLHKCDISNSTEAGQKLFNMLRLGKSE PWTLALENVVGAKNMNVRPLLNYFEPLFTWLKDQNKNSFVGWSTDWS PYADQSIKVRISLKSALGDKAYEWNDNEMYLFRSSVAYAMRQYFLKV KNQMILFGEEDVRVANLKPRISFNFFVTAPKNVSDIIPRTEVEKAIR MSRSRINDAFRLNDNSLEFLGIQPTLGPPNQPPRSEPKSCDKTHTCP PCPAPELLGGPSVFLFPPKPKDTLMISRTPEVTCVVVDVSHEDPEVK FNWYVDGVEVHNAKTKPREEQYNSTYRVVSVLTVLHQDWLNGKEYKC KVSNKALPAPIEKTISKAKGQPREPQVYTLPPSRDELTKNQVSLTCL VKGFYPSDIAVEWESNGQPENNYKTTPPVLDSDGSFFLYSKLTVDKS RWQQGNVFSCSVMHEALHNHYTQKSLSLSPGK | With signal peptide, without mutations (wild-type) |
| **4** | QSTIEEQAKTFLDKFNHEAEDLFYQSSLASWNYNTNITEENVQNMNN AGDKWSAFLKEQSTLAQMYPLQEIQNLTVKLQLQALQQNGSSVLSED KSKRLNTILNTMSTIYSTGKVCNPDNPQECLLLEPGLNEIMANSLDY NERLWAWESWRSEVGKQLRPLYEEYVVLKNEMARANHYEDYGDYWRG DYEVNGVDGYDYSRGQLIEDVEHTFEEIKPLYEHLHAYVRAKLMNAY PSYISPIGCLPAHLLGDMWGRFWTNLYSLTVPFGQKPNIDVTDAMVD QAWDAQRIFKEAEKFFVSVGLPNMTQGFWENSMLTDPGNVQKAVCHP TAWDLGKGDFRILMCTKVTMDDFLTAHHEMGHIQYDMAYAAQPFLLR NGANEGFHEAVGEIMSLSAATPKHLKSIGLLSPDFQEDNETEINFLL KQALTIVGTLPFTYMLEKWRWMVFKGEIPKDQWMKKWWEMKREIVGV VEPVPHDETYCDPASLFHVSNDYSFIRYYTRTLYQFQFQEALCQAAK HEGPLHKCDISNSTEAGQKLFNMLRLGKSEPWTLALENVVGAKNMNV RPLLNYFEPLFTWLKDQNKNSFVGWSTDWSPYADQSIKVRISLKSAL GDKAYEWNDNEMYLFRSSVAYAMRQYFLKVKNQMILFGEEDVRVANL KPRISFNFFVTAPKNVSDIIPRTEVEKAIRMSRSRINDAFRLNDNSL EFLGIQPTLGPPNQPPRSEPKSCDKTHTCPPCPAPELLGGPSVFLFP PKPKDTLMISRTPEVTCVVVDVSHEDPEVKFNWYVDGVEVHNAKTKP REEQYNSTYRVVSVLTVLHQDWLNGKEYKCKVSNKALPAPIEKTISK AKGQPREPQVYTLPPSRDELTKNQVSLTCLVKGFYPSDIAVEWESNG QPENNYKTTPPVLDSDGSFFLYSKLTVDKSRWQQGNVFSCSVMHEAL HNHYTQKSLSLSPGKDSNLWN | No signal peptide with mutated sequence |
| **5** | QSTIEEQAKTFLDKFNHEAEDLFYQSSLASWNYNTNITEENVQNMNN AGDKWSAFLKEQSTLAQMYPLQEIQNLTVKLQLQALQQNGSSVLSED KSKRLNTILNTMSTIYSTGKVCNPDNPQECLLLEPGLNEIMANSLDY NERLWAWESWRSEVGKQLRPLYEEYVVLKNEMARANHYEDYGDYWRG DYEVNGVDGYDYSRGQLIEDVEHTFEEIKPLYEHLHAYVRAKLMNAY PSYISPIGCLPAHLLGDMWGRFWTNLYSLTVPFGQKPNIDVTDAMVD QAWDAQRIFKEAEKFFVSVGLPNMTQGFWENSMLTDPGNVQKAVCHP TAWDLGKGDFRILMCTKVTMDDFLTAHHEMGHIQYDMAYAAQPFLLR NGANEGFHEAVGEIMSLSAATPKHLKSIGLLSPDFQEDNETEINFLL KQALTIVGTLPFTYMLEKWRWMVFKGEIPKDQWMKKWWEMKREIVGV VEPVPHDETYCDPASLFHVSNDYSFIRYYTRTLYQFQFQEALCQAAK HEGPLHKCDISNSTEAGQKLFNMLRLGKSEPWTLALENVVGAKNMNV RPLLNYFEPLFTWLKDQNKNSFVGWSTDWSPYADQSIKVRISLKSAL GDKAYEWNDNEMYLFRSSVAYAMRQYFLKVKNQMILFGEEDVRVANL KPRISFNFFVTAPKNVSDIIPRTEVEKAIRMSRSRINDAFRLNDNSL EFLGIQPTLGPPNQPP | ACE2 extracellular domain |
| **6** | EPKSCDKTHTCPPCPAPELLGGPSVFLFPPKPKDTLMISRTPEVTCV VVDVSHEDPEVKFNWYVDGVEVHNAKTKPREEQYNSTYRVVSVLTVL HQDWLNGKEYKCKVSNKALPAPIEKTISKAKGQPREPQVYTLPPSRD ELTKNQVSLTCLVKGFYPSDIAVEWESNGQPENNYKTTPPVLDSDGS FFLYSKLTVDKSRWQQGNVFSCSVMHEALHNHYTQKSLSLSPGK | Fc Tag |
| **7** | MSSSSWLLLSLVAVTAAQSTIEEQAKTFLDKFNHEAEDLFYQSSLAS WNYNTNITEENVQNMNNAGDKWSAFLKEQSTLAQMYPLQEIQNLTVK LQLQALQQNGSSVLSEDKSKRLNTILNTMSTIYSTGKVCNPDNPQEC LLLEPGLNEIMANSLDYNERLWAWESWRSEVGKQLRPLYEEYVVLKN EMARANHYEDYGDYWRGDYEVNGVDGYDYSRGQLIEDVEHTFEEIKP LYEHLHAYVRAKLMNAYPSYISPIGCLPAHLLGDMWGRFWTNLYSLT VPFGQKPNIDVTDAMVDQAWDAQRIFKEAEKFFVSVGLPNMTQGFWE NSMLTDPGNVQKAVCHPTAWDLGKGDFRILMCTKVTMDDFLTAHHEM GHIQYDMAYAAQPFLLRNGANEGFHEAVGEIMSLSAATPKHLKSIGL LSPDFQEDNETEINFLLKQALTIVGTLPFTYMLEKWRWMVFKGEIPK DQWMKKWWEMKREIVGVVEPVPHDETYCDPASLFHVSNDYSFIRYYT RTLYQFQFQEALCQAAKHEGPLHKCDISNSTEAGQKLFNMLRLGKSE PWTLALENVVGAKNMNVRPLLNYFEPLFTWLKDQNKNSFVGWSTDWS PYADQSIKVRISLKSALGDKAYEWNDNEMYLFRSSVAYAMRQYFLKV KNQMILFGEEDVRVANLKPRISFNFFVTAPKNVSDIIPRTEVEKAIR MSRSRINDAFRLNDNSLEFLGIQPTLGPPNQPP | ACE2 extracellular domain |
| **8** | EPKSCDKTHTCPPCPAPELLGGPSVFLFPPKPKDTLMISRTPEVTCV VVDVSHEDPEVKFNWYVDGVEVHNAKTKPREEQYNSTYRVVSVLTVL HQDWLNGKEYKCKVSNKALPAPIEKTISKAKGQPREPQVYTLPPSRD ELTKNQVSLTCLVKGFYPSDIAVEWESNGQPENNYKTTPPVLDSDGS FFLYSKLTVDKSRWQQGNVFSCSVMHEALHNHYTQKSLSLSPGKDSN LWN | Fc Tag |
| **9** | MSSSSWLLLSLVAVTAA | Signal peptide |
| **10** | DSNLWN | Mutant sequence |
| **11** | MSSSSWLLLSLVAVTAAQSTIEEQAKTFLDKFNHEAEDLFYQSSLAS WNYNTNITEENVQNMNNAGDKWSAFLKEQSTLAQMYPLQEIQNLTVK LQLQALQQNGSSVLSEDKSKRLNTILNTMSTIYSTGKVCNPDNPQEC LLLEPGLNEIMANSLDYNERLWAWESWRSEVGKQLRPLYEEYVVLKN EMARANHYEDYGDYWRGDYEVNGVDGYDYSRGQLIEDVEHTFEEIKP LYEHLHAYVRAKLMNAYPSYISPIGCLPAHLLGDMWGRFWTNLYSLT VPFGQKPNIDVTDAMVDQAWDAQRIFKEAEKFFVSVGLPNMTQGFWE NSMLTDPGNVQKAVCHPTAWDLGKGDFRILMCTKVTMDDFLTAHHEM GHIQYDMAYAAQPFLLRNGANEGFHEAVGEIMSLSAATPKHLKSIGL LSPDFQEDNETEINFLLKQALTIVGTLPFTYMLEKWRWMVFKGEIPK DQWMKKWWEMKREIVGVVEPVPHDETYCDPASLFHVSNDYSFIRYYT RTLYQFQFQEALCQAAKHEGPLHKCDISNSTEAGQKLFNMLRLGKSE PWTLALENVVGAKNMNVRPLLNYFEPLFTWLKDQNKNSFVGWSTDWS PYADQSIKVRISLKSALGDKAYEWNDNEMYLFRSSVAYAMRQYFLKV KNQMILFGEEDVRVANLKPRISFNFFVTAPKNVSDIIPRTEVEKAIR MSRSRINDAFRLNDNSLEFLGIQPTLGPPNQPPRS | ACE2 extracellular domain with a signal peptide |
| **12** | MSSSSWLLLSLVAVTAAQSTIEEQAKTFLDKFNHEAEDLFYQSSLAS WNYNTNITEENVQNMNNAGDKWSAFLKEQSTLAQMYPLQEIQNLTVK LQLQALQQNGSSVLSEDKSKRLNTILNTMSTIYSTGKVCNPDNPQEC LLLEPGLNEIMANSLDYNERLWAWESWRSEVGKQLRPLYEEYVVLKN EMARANHYEDYGDYWRGDYEVNGVDGYDYSRGQLIEDVEHTFEEIKP LYEHLHAYVRAKLMNAYPSYISPIGCLPAHLLGDMWGRFWTNLYSLT VPFGQKPNIDVTDAMVDQAWDAQRIFKEAEKFFVSVGLPNMTQGFWE NSMLTDPGNVQKAVCHPTAWDLGKGDFRILMCTKVTMDDFLTAHHEM GHIQYDMAYAAQPFLLRNGANEGFHEAVGEIMSLSAATPKHLKSIGL LSPDFQEDNETEINFLLKQALTIVGTLPFTYMLEKWRWMVFKGEIPK DQWMKKWWEMKREIVGVVEPVPHDETYCDPASLFHVSNDYSFIRYYT RTLYQFQFQEALCQAAKHEGPLHKCDISNSTEAGQKLFNMLRLGKSE PWTLALENVVGAKNMNVRPLLNYFEPLFTWLKDQNKNSFVGWSTDWS PYADQSIKVRISLKSALGDKAYEWNDNEMYLFRSSVAYAMRQYFLKV KNQMILFGEEDVRVANLKPRISFNFFVTAPKNVSDIIPRTEVEKAIR MSRSRINDAFRLNDNSLEFLGIQPTLGPPNQPPVS | ACE2 extracellular domain with a signal peptide |
| **13** | QSTIEEQAKTFLDKFNHEAEDLFYQSSLASWNYNTNITEENVQNMNN AGDKWSAFLKEQSTLAQMYPLQEIQNLTVKLQLQALQQNGSSVLSED KSKRLNTILNTMSTIYSTGKVCNPDNPQECLLLEPGLNEIMANSLDY NERLWAWESWRSEVGKQLRPLYEEYVVLKNEMARANHYEDYGDYWRG DYEVNGVDGYDYSRGQLIEDVEHTFEEIKPLYEHLHAYVRAKLMNAY PSYISPIGCLPAHLLGDMWGRFWTNLYSLTVPFGQKPNIDVTDAMVD QAWDAQRIFKEAEKFFVSVGLPNMTQGFWENSMLTDPGNVQKAVCHP TAWDLGKGDFRILMCTKVTMDDFLTAHHEMGHIQYDMAYAAQPFLLR NGANEGFHEAVGEIMSLSAATPKHLKSIGLLSPDFQEDNETEINFLL KQALTIVGTLPFTYMLEKWRWMVFKGEIPKDQWMKKWWEMKREIVGV VEPVPHDETYCDPASLFHVSNDYSFIRYYTRTLYQFQFQEALCQAAK HEGPLHKCDISNSTEAGQKLFNMLRLGKSEPWTLALENVVGAKNMNV RPLLNYFEPLFTWLKDQNKNSFVGWSTDWSPYADQSIKVRISLKSAL GDKAYEWNDNEMYLFRSSVAYAMRQYFLKVKNQMILFGEEDVRVANL KPRISFNFFVTAPKNVSDIIPRTEVEKAIRMSRSRINDAFRLNDNSL EFLGIQPTLGPPNQPPRS | ACE2 extracellular domain without a signal peptide |
| **14** | QSTIEEQAKTFLDKFNHEAEDLFYQSSLASWNYNTNITEENVQNMNN AGDKWSAFLKEQSTLAQMYPLQEIQNLTVKLQLQALQQNGSSVLSED KSKRLNTILNTMSTIYSTGKVCNPDNPQECLLLEPGLNEIMANSLDY NERLWAWESWRSEVGKQLRPLYEEYVVLKNEMARANHYEDYGDYWRG DYEVNGVDGYDYSRGQLIEDVEHTFEEIKPLYEHLHAYVRAKLMNAY PSYISPIGCLPAHLLGDMWGRFWTNLYSLTVPFGQKPNIDVTDAMVD QAWDAQRIFKEAEKFFVSVGLPNMTQGFWENSMLTDPGNVQKAVCHP TAWDLGKGDFRILMCTKVTMDDFLTAHHEMGHIQYDMAYAAQPFLLR NGANEGFHEAVGEIMSLSAATPKHLKSIGLLSPDFQEDNETEINFLL KQALTIVGTLPFTYMLEKWRWMVFKGEIPKDQWMKKWWEMKREIVGV VEPVPHDETYCDPASLFHVSNDYSFIRYYTRTLYQFQFQEALCQAAK HEGPLHKCDISNSTEAGQKLFNMLRLGKSEPWTLALENVVGAKNMNV RPLLNYFEPLFTWLKDQNKNSFVGWSTDWSPYADQSIKVRISLKSAL GDKAYEWNDNEMYLFRSSVAYAMRQYFLKVKNQMILFGEEDVRVANL KPRISFNFFVTAPKNVSDIIPRTEVEKAIRMSRSRINDAFRLNDNSL EFLGIQPTLGPPNQPPVS | ACE2 extracellular domain without a signal peptide |
| **15** | MSSSSWLLLSLVAVTAAQSTIEEQAKTFLDKFNHEAEDLFYQSSLAS WNYNTNITEENVQNMNNAGDKWSAFLKEQSTLAQMYPLQEIQNLTVK LQLQALQQNGSSVLSEDKSKRLNTILNTMSTIYSTGKVCNPDNPQEC LLLEPGLNEIMANSLDYNERLWAWESWRSEVGKQLRPLYEEYVVLKN EMARANHYEDYGDYWRGDYEVNGVDGYDYSRGQLIEDVEHTFEEIKP LYEHLHAYVRAKLMNAYPSYISPIGCLPAHLLGDMWGRFWTNLYSLT VPFGQKPNIDVTDAMVDQAWDAQRIFKEAEKFFVSVGLPNMTQGFWE NSMLTDPGNVQKAVCHPTAWDLGKGDFRILMCTKVTMDDFLTAHHEM GHIQYDMAYAAQPFLLRNGANEGFHEAVGEIMSLSAATPKHLKSIGL LSPDFQEDNETEINFLLKQALTIVGTLPFTYMLEKWRWMVFKGEIPK DQWMKKWWEMKREIVGVVEPVPHDETYCDPASLFHVSNDYSFIRYYT RTLYQFQFQEALCQAAKHEGPLHKCDISNSTEAGQKLFNMLRLGKSE PWTLALENVVGAKNMNVRPLLNYFEPLFTWLKDQNKNSFVGWSTDWS PYADQSIKVRISLKSALGDKAYEWNDNEMYLFRSSVAYAMRQYFLKV KNQMILFGEEDVRVANLKPRISFNFFVTAPKNVSDIIPRTEVEKAIR MSRSRINDAFRLNDNSLEFLGIQPTLGPPNQPPVSEPKSCDKTHTCP PCPAPELLGGPSVFLFPPKPKDTLMISRTPEVTCVVVDVSHEDPEVK FNWYVDGVEVHNAKTKPREEQYNSTYRVVSVLTVLHQDWLNGKEYKC KVSNKALPAPIEKTISKAKGQPREPQVYTLPPSRDELTKNQVSLTCL VKGFYPSDIAVEWESNGQPENNYKTTPPVLDSDGSFFLYSKLTVDKS RWQQGNVFSCSVMHEALHNHYTQKSLSLSPGKDSNLWN | Full-length fusion peptide sequence with a signal peptide |
| **16** | QSTIEEQAKTFLDKFNHEAEDLFYQSSLASWNYNTNITEENVQNMNN AGDKWSAFLKEQSTLAQMYPLQEIQNLTVKLQLQALQQNGSSVLSED KSKRLNTILNTMSTIYSTGKVCNPDNPQECLLLEPGLNEIMANSLDY NERLWAWESWRSEVGKQLRPLYEEYVVLKNEMARANHYEDYGDYWRG DYEVNGVDGYDYSRGQLIEDVEHTFEEIKPLYEHLHAYVRAKLMNAY PSYISPIGCLPAHLLGDMWGRFWTNLYSLTVPFGQKPNIDVTDAMVD QAWDAQRIFKEAEKFFVSVGLPNMTQGFWENSMLTDPGNVQKAVCHP TAWDLGKGDFRILMCTKVTMDDFLTAHHEMGHIQYDMAYAAQPFLLR NGANEGFHEAVGEIMSLSAATPKHLKSIGLLSPDFQEDNETEINFLL KQALTIVGTLPFTYMLEKWRWMVFKGEIPKDQWMKKWWEMKREIVGV VEPVPHDETYCDPASLFHVSNDYSFIRYYTRTLYQFQFQEALCQAAK HEGPLHKCDISNSTEAGQKLFNMLRLGKSEPWTLALENVVGAKNMNV RPLLNYFEPLFTWLKDQNKNSFVGWSTDWSPYADQSIKVRISLKSAL GDKAYEWNDNEMYLFRSSVAYAMRQYFLKVKNQMILFGEEDVRVANL KPRISFNFFVTAPKNVSDIIPRTEVEKAIRMSRSRINDAFRLNDNSL EFLGIQPTLGPPNQPPVSEPKSCDKTHTCPPCPAPELLGGPSVFLFP PKPKDTLMISRTPEVTCVVVDVSHEDPEVKFNWYVDGVEVHNAKTKP REEQYNSTYRVVSVLTVLHQDWLNGKEYKCKVSNKALPAPIEKTISK AKGQPREPQVYTLPPSRDELTKNQVSLTCLVKGFYPSDIAVEWESNG QPENNYKTTPPVLDSDGSFFLYSKLTVDKSRWQQGNVFSCSVMHEAL HNHYTQKSLSLSPGK | Fusion peptide without a signal peptide and without mutations |
| **17** | MSSSSWLLLSLVAVTAAQSTIEEQAKTFLDKFNHEAEDLFYQSSLAS WNYNTNITEENVQNMNNAGDKWSAFLKEQSTLAQMYPLQEIQNLTVK LQLQALQQNGSSVLSEDKSKRLNTILNTMSTIYSTGKVCNPDNPQEC LLLEPGLNEIMANSLDYNERLWAWESWRSEVGKQLRPLYEEYVVLKN EMARANHYEDYGDYWRGDYEVNGVDGYDYSRGQLIEDVEHTFEEIKP LYEHLHAYVRAKLMNAYPSYISPIGCLPAHLLGDMWGRFWTNLYSLT VPFGQKPNIDVTDAMVDQAWDAQRIFKEAEKFFVSVGLPNMTQGFWE NSMLTDPGNVQKAVCHPTAWDLGKGDFRILMCTKVTMDDFLTAHHEM GHIQYDMAYAAQPFLLRNGANEGFHEAVGEIMSLSAATPKHLKSIGL LSPDFQEDNETEINFLLKQALTIVGTLPFTYMLEKWRWMVFKGEIPK DQWMKKWWEMKREIVGVVEPVPHDETYCDPASLFHVSNDYSFIRYYT RTLYQFQFQEALCQAAKHEGPLHKCDISNSTEAGQKLFNMLRLGKSE PWTLALENVVGAKNMNVRPLLNYFEPLFTWLKDQNKNSFVGWSTDWS PYADQSIKVRISLKSALGDKAYEWNDNEMYLFRSSVAYAMRQYFLKV KNQMILFGEEDVRVANLKPRISFNFFVTAPKNVSDIIPRTEVEKAIR MSRSRINDAFRLNDNSLEFLGIQPTLGPPNQPPVSEPKSCDKTHTCP PCPAPELLGGPSVFLFPPKPKDTLMISRTPEVTCVVVDVSHEDPEVK FNWYVDGVEVHNAKTKPREEQYNSTYRVVSVLTVLHQDWLNGKEYKC KVSNKALPAPIEKTISKAKGQPREPQVYTLPPSRDELTKNQVSLTCL VKGFYPSDIAVEWESNGQPENNYKTTPPVLDSDGSFFLYSKLTVDKS RWQQGNVFSCSVMHEALHNHYTQKSLSLSPGK | Full sequence with signal peptide and without mutations |
| **18** | QSTIEEQAKTFLDKFNHEAEDLFYQSSLASWNYNTNITEENVQNMNN AGDKWSAFLKEQSTLAQMYPLQEIQNLTVKLQLQALQQNGSSVLSED KSKRLNTILNTMSTIYSTGKVCNPDNPQECLLLEPGLNEIMANSLDY NERLWAWESWRSEVGKQLRPLYEEYVVLKNEMARANHYEDYGDYWRG DYEVNGVDGYDYSRGQLIEDVEHTFEEIKPLYEHLHAYVRAKLMNAY PSYISPIGCLPAHLLGDMWGRFWTNLYSLTVPFGQKPNIDVTDAMVD QAWDAQRIFKEAEKFFVSVGLPNMTQGFWENSMLTDPGNVQKAVCHP TAWDLGKGDFRILMCTKVTMDDFLTAHHEMGHIQYDMAYAAQPFLLR NGANEGFHEAVGEIMSLSAATPKHLKSIGLLSPDFQEDNETEINFLL KQALTIVGTLPFTYMLEKWRWMVFKGEIPKDQWMKKWWEMKREIVGV VEPVPHDETYCDPASLFHVSNDYSFIRYYTRTLYQFQFQEALCQAAK HEGPLHKCDISNSTEAGQKLFNMLRLGKSEPWTLALENVVGAKNMNV RPLLNYFEPLFTWLKDQNKNSFVGWSTDWSPYADQSIKVRISLKSAL GDKAYEWNDNEMYLFRSSVAYAMRQYFLKVKNQMILFGEEDVRVANL KPRISFNFFVTAPKNVSDIIPRTEVEKAIRMSRSRINDAFRLNDNSL EFLGIQPTLGPPNQPPVSEPKSCDKTHTCPPCPAPELLGGPSVFLFP PKPKDTLMISRTPEVTCVVVDVSHEDPEVKFNWYVDGVEVHNAKTKP REEQYNSTYRVVSVLTVLHQDWLNGKEYKCKVSNKALPAPIEKTISK AKGQPREPQVYTLPPSRDELTKNQVSLTCLVKGFYPSDIAVEWESNG QPENNYKTTPPVLDSDGSFFLYSKLTVDKSRWQQGNVFSCSVMHEAL HNHYTQKSLSLSPGKDSNLWN | Full sequence without signal peptide and with mutations |

## Claims

1. A fusion protein comprising a plurality of recombinant polypeptides, wherein the fusion protein comprises a human ACE2 protein or a fragment thereof and a human IgG Fc protein or a functional variant thereof.

2. The fusion protein according to claim 1, wherein the human ACE2 protein or a fragment thereof comprises a human ACE2 extracellular domain or a fragment thereof.

3. The fusion protein according to claim 1 or 2, wherein the human ACE2 protein or a fragment thereof comprises an amino acid sequence selected from the group consisting of SEQ ID NO: 5, SEQ ID NO: 7, SEQ ID NO: 11, SEQ ID NO: 12, SEQ ID NO: 13, and SEQ ID NO: 14, or an amino acid sequence having at least about 75%, 80%, 85%, 90%, 92%, 95%, 96%, 97%, 98%, or 99% sequence identity thereto, or a fragment thereof.

4. The fusion protein according to any one of claims 1 to 3, wherein the human ACE2 protein or a fragment thereof comprises: an amino acid sequence of SEQ ID NO: 5, or an amino acid sequence having at least about 75%, 80%, 85%, 90%, 92%, 95%, 96%, 97%, 98%, or 99% sequence identity to SEQ ID NO: 5, or a fragment thereof.

5. The fusion protein according to any one of claims 1 to 4, wherein the human IgG Fc protein or a functional variant thereof comprises an Fc region sequence selected from the group consisting of human IgG1 Fc, IgG2 Fc, IgG3 Fc, and IgG4 Fc, a functional variant, or a fragment thereof.

6. The fusion protein according to any one of claims 1 to 5, wherein the human IgG Fc protein or a functional variant thereof is selected from the group consisting of a human IgG1 Fc region sequence, a functional variant thereof, and a fragment thereof.

7. The fusion protein according to any one of claims 1 to 6, wherein the human IgG Fc protein or a functional variant thereof comprises: an amino acid sequence of SEQ ID NO: 6 or SEQ ID NO: 8, or an amino acid sequence having at least about 75%, 80%, 85%, 90%, 92%, 95%, 96%, 97%, 98%, or 99% sequence identity to SEQ ID NO: 6 or SEQ ID NO: 8, or a fragment thereof.

8. The fusion protein according to any one of claims 1 to 7, wherein the human IgG Fc protein or a functional variant thereof comprises: an amino acid sequence of SEQ ID NO: 6, or an amino acid sequence having at least about 75%, 80%, 85%, 90%, 92%, 95%, 96%, 97%, 98%, or 99% sequence identity to SEQ ID NO: 6, or a fragment thereof.

9. The fusion protein according to any one of claims 1 to 8, optionally comprising a signal peptide.

10. The fusion protein according to any one of claims 1 to 9, optionally comprising a peptide linker, wherein the human ACE2 protein or a fragment thereof and the human IgG Fc protein or a functional variant thereof are directly linked or linked via the peptide linker.

11. The fusion protein according to claim 10, wherein the peptide linker is selected from the group consisting of: an arginine-serine linker (-RS-), a valine-serine linker (-VS-), and a glycine-serine linker (-GS-).

12. The fusion protein according to any one of claims 1 to 11, optionally comprising a mutated sequence.

13. The fusion protein according to claim 1, comprising: an amino acid sequence selected from the group consisting of SEQ ID NO: 1 through SEQ ID NO: 18, or an amino acid sequence having at least about 75%, 80%, 85%, 90%, 92%, 95%, 96%, 97%, 98%, or 99% sequence identity thereto, or a fragment thereof, or a combination thereof.

14. The fusion protein according to claim 1, comprising: an amino acid sequence selected from the group consisting of SEQ ID NO: 1, SEQ ID NO: 2, SEQ ID NO: 3, SEQ ID NO: 4, SEQ ID NO: 15, SEQ ID NO: 16, SEQ ID NO: 17, and SEQ ID NO: 18, or an amino acid sequence having at least about 75%, 80%, 85%, 90%, 92%, 95%, 96%, 97%, 98%, or 99% sequence identity thereto, or a fragment thereof; for example, comprising an amino acid sequence selected from the group consisting of SEQ ID NO: 1, SEQ ID NO: 2, SEQ ID NO: 3, SEQ ID NO: 4, SEQ ID NO: 15, SEQ ID NO: 16, SEQ ID NO: 17, and SEQ ID NO: 18, or an amino acid sequence having at least about 75%, 80%, 85%, 90%, 92%, 95%, 96%, 97%, 98%, or 99% sequence identity thereto, or a fragment thereof.

15. The fusion protein according to any one of claims 1 to 14, further comprising a detectable label.

16. A pharmaceutical composition comprising: the fusion protein according to any one of claims 1 to 15, and optionally, a pharmaceutically acceptable carrier.

17. The pharmaceutical composition according to claim 16, comprising the fusion protein according to any one of claims 1 to 15 at a concentration of about 0.1 mg/ml to about 100 mg/ml, for example about 0.50 mg/ml to about 20.00 mg/ml, such as about 1.25 mg/ml, about 2.50 mg/ml, or about 5.00 mg/ml.

18. The pharmaceutical composition according to claim 16 or 17, wherein the pharmaceutically acceptable carrier comprises one or more selected from the group consisting of a buffer and a tonicity agent.

19. The pharmaceutical composition according to claim 18, wherein the buffer comprises one or more selected from the group consisting of sodium dihydrogen phosphate monohydrate, disodium hydrogen phosphate dihydrate, or a combination thereof.

20. The pharmaceutical composition according to claim 18 or 19, wherein the tonicity agent comprises one or more selected from the group consisting of sodium chloride, potassium chloride, glycerol, glucose, sorbitol, sucrose, xylitol, and mannitol.

21. The pharmaceutical composition according to any one of claims 18 to 20, wherein the tonicity agent comprises sodium chloride and/or sucrose.

22. The pharmaceutical composition according to any one of claims 18 to 21, wherein the pharmaceutically acceptable carrier optionally comprises a stabilizer selected from the group consisting of: proteins, peptides, or hydrolysates, e.g., albumin, gelatin; sugars, e.g., sucrose, lactose, sorbitol; amino acids, e.g., sodium glutamate; or a combination thereof, for example, wherein the stabilizer comprises sucrose.

23. The pharmaceutical composition according to any one of claims 18 to 22, wherein the pharmaceutically acceptable carrier optionally comprises a bacteriostatic agent selected from the group consisting of benzoic acid, sorbic acid, thimerosal, and phenylethyl alcohol, or a combination thereof, for example, wherein the bacteriostatic agent comprises phenylethyl alcohol and/or thimerosal.

24. The pharmaceutical composition according to any one of claims 18 to 23, formulated for administration via a route selected from intramuscular, intradermal, subcutaneous, intravenous, intraarterial, intraarticular, intraperitoneal, intranasal, sublingual, tonsillar, oropharyngeal, or other parenteral and mucosal routes, preferably formulated for intranasal administration, for example as a nasal spray.

25. The fusion protein according to any one of claims 1 to 15, or the pharmaceutical composition according to any one of claims 16 to 24, for use in: preventing or treating infection by coronavirus SARS-CoV-2 and a variant thereof; and/or preventing transmission of SARS-CoV-2 and a variant thereof from an infected subject.

26. The fusion protein according to any one of claims 1 to 15, or the pharmaceutical composition according to any one of claims 16 to 24, for use according to claim 25, wherein the SARS-CoV-2 and a variant thereof comprise, but are not limited to: SARS-CoV-2 Hu-1, Alpha, Beta, Gamma, Delta, Mu, Omicron, JN.1; and hitherto unknown variants of coronavirus SARS-CoV-2.

27. The fusion protein according to any one of claims 1 to 15, or the pharmaceutical composition according to any one of claims 16 to 24, for use according to claim 25 or 26, wherein the fusion protein or pharmaceutical composition is administered via a route selected from intramuscular, intradermal, subcutaneous, intravenous, intraarterial, intraarticular, intraperitoneal, intranasal, sublingual, tonsillar, oropharyngeal, or other parenteral and mucosal routes, preferably via intranasal administration.

28. The fusion protein according to any one of claims 1 to 15, or the pharmaceutical composition according to any one of claims 16 to 24, for use according to any one of claims 25 to 27, wherein the fusion protein or pharmaceutical composition is administered intranasally as: a single dose, or a plurality of doses separated by one or more intervals measured in hours, days, or weeks.

29. The fusion protein according to any one of claims 1 to 15, or the pharmaceutical composition according to any one of claims 16 to 24, for use according to claim 28, wherein the interval is selected from the group consisting of 4 hours, 6 hours, 8 hours, 12 hours, 24 hours, 48 hours, or 72 hours.

30. The fusion protein according to any one of claims 1 to 15, or the pharmaceutical composition according to any one of claims 16 to 24, for use according to any one of claims 25 to 29, wherein the fusion protein or pharmaceutical composition is administered at a frequency of once or multiple times per day, for example once per day or twice per day.

31. Use of the fusion protein according to any one of claims 1 to 15, or the pharmaceutical composition according to any one of claims 16 to 24, in the manufacture of a medicament for: preventing or treating infection by coronavirus SARS-CoV-2 and a variant thereof; and/or preventing transmission of SARS-CoV-2 and a variant thereof from an infected subject.

32. The use according to claim 31, wherein the SARS-CoV-2 and a variant thereof comprise, but are not limited to: SARS-CoV-2 Hu-1, Alpha, Beta, Gamma, Delta, Mu, Omicron, JN.1, and hitherto unknown variants of coronavirus SARS-CoV-2.

33. A method for preventing or treating infection by coronavirus SARS-CoV-2 and a variant thereof, and/or preventing transmission of SARS-CoV-2 and a variant thereof from an infected subject, comprising administering to a subject a therapeutically effective amount of: the fusion protein according to any one of claims 1 to 15, or the pharmaceutical composition according to any one of claims 16 to 24.

34. The method according to claim 33, wherein the SARS-CoV-2 and a variant thereof comprise, but are not limited to: SARS-CoV-2 Hu-1, Alpha, Beta, Gamma, Delta, Mu, Omicron, JN.1, and hitherto unknown variants of coronavirus SARS-CoV-2.

35. The method according to claim 33 or 34, wherein the fusion protein or pharmaceutical composition is administered via a route selected from intramuscular, intradermal, subcutaneous, intravenous, intraarterial, intraarticular, intraperitoneal, intranasal, sublingual, tonsillar, oropharyngeal, or other parenteral and mucosal routes, preferably via intranasal administration.

36. The method according to any one of claims 33 to 35, wherein the fusion protein according to any one of claims 1 to 15 or the pharmaceutical composition according to any one of claims 16 to 24 is administered intranasally as: a single dose, or a plurality of doses separated by one or more intervals measured in hours, days, or weeks.

37. The method according to claim 36, wherein the interval is selected from 4 hours, 6 hours, 8 hours, 12 hours, 24 hours, 48 hours, or 72 hours.

38. The method according to any one of claims 33 to 37, wherein the fusion protein or pharmaceutical composition is administered at a frequency of once or multiple times per day, for example once per day or twice per day.

39. A kit for preventing or treating infection by coronavirus SARS-CoV-2 and a variant thereof, and/or preventing transmission of SARS-CoV-2 and a variant thereof from an infected subject, comprising: the fusion protein according to any one of claims 1 to 15, or the pharmaceutical composition according to any one of claims 16 to 24;
a container; and
optionally, a package insert or label with instructions for prevention and/or treatment.

40. The kit according to claim 39, wherein the SARS-CoV-2 and a variant thereof comprise, but are not limited to: SARS-CoV-2 Hu-1, Alpha, Beta, Gamma, Delta, Mu, Omicron, JN.1, and hitherto unknown variants of coronavirus SARS-CoV-2.

41. A nucleic acid encoding the fusion protein according to any one of claims 1 to 15, or a fragment of the fusion protein.

42. A vector comprising the nucleic acid according to claim 41.

43. The vector according to claim 33, which is phFC(IM).

44. A host cell comprising: the nucleic acid according to claim 41, or the vector according to claim 42.

45. The host cell according to claim 44, which is a CHO cell, for example GH-CHO.
